(19) Europäisches Patentamt
European Patent Office
Office européen des brevets

(11) EP 3 200 829 B1

(12) **EUROPEAN PATENT SPECIFICATION**

(45) Date of publication and mention of the grant of the patent:
**06.12.2023 Bulletin 2023/49**

(21) Application number: **15782151.3**

(22) Date of filing: **01.10.2015**

(51) International Patent Classification (IPC):
***A61K 47/50*** *(2017.01)*

(52) Cooperative Patent Classification (CPC):
**A61K 47/60; A61K 47/6803; A61K 47/6817; A61K 47/6889; A61P 35/00**

(86) International application number:
**PCT/US2015/053397**

(87) International publication number:
**WO 2016/054315 (07.04.2016 Gazette 2016/14)**

# (54) METHOD OF CONJUGATING A POLYPEPTIDE

VERFAHREN ZUM KONJUGIEREN EINES POLYPEPTIDS

MÉTHODE DE CONJUGAISON D'UN POLYPEPTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2014 US 201462058502 P**

(43) Date of publication of application:
**09.08.2017 Bulletin 2017/32**

(73) Proprietor: **Medimmune, LLC**
**Gaithersburg, Maryland 20878 (US)**

(72) Inventors:
- **CHRISTIE, Ronald James**
  **Gaithersburg, MD 20878 (US)**
- **GAO, Changshou**
  **Gaithersburg, MD 20878 (US)**
- **DIMASI, Nazzareno**
  **Gaithersburg, MD 20878 (US)**

(74) Representative: **AstraZeneca Intellectual Property**
**Eastbrook House**
**Shaftesbury Road**
**Cambridge CB2 8BF (GB)**

(56) References cited:

- **KRATZ F ET AL: "A NOVEL MACROMOLECULAR PRODRUG CONCEPT EXPLOITING ENDOGENOUS SERUM ALBUMIN AS A DRUG CARRIER FOR CANCER CHEMOTHERAPY", JOURNAL OF MEDICINAL CHEMISTRY, AMERICAN CHEMICAL SOCIETY, US, vol. 43, 6 April 2000 (2000-04-06), pages 1253-1256, XP000990088, ISSN: 0022-2623, DOI: 10.1021/JM9905864**
- **Z. T. SHEN ET AL: "Bi-specific MHC Heterodimers for Characterization of Cross-reactive T Cells", JOURNAL OF BIOLOGICAL CHEMISTRY, vol. 285, no. 43, 20 August 2010 (2010-08-20), pages 33144-33153, XP055233580, US ISSN: 0021-9258, DOI: 10.1074/jbc.M110.141051**
- **KANG MYUNG J ET AL: "Design of a Pep-1 peptide-modified liposomal nanocarrier system for intracellular drug delivery: Conformational characterization and cellular uptake evaluation", JOURNAL OF DRUG TARGETING, HARWOOD ACADEMIC PUBLISHERS GMBH, DE, vol. 19, no. 7, 1 January 2011 (2011-01-01), pages 497-505, XP009172102, ISSN: 1061-186X, DOI: 10.3109/1061186X.2010.511226**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 200 829 B1

- FUJIWARA K ET AL: "Novel enzyme immunoassay for thyrotropin-releasing hormone using N-(4-diazophenyl)maleimide as a coupling agent", FEBS LETTERS, ELSEVIER, AMSTERDAM, NL, vol. 202, no. 2, 7 July 1986 (1986-07-07), pages 197-201, XP025753284, ISSN: 0014-5793, DOI: 10.1016/0014-5793(86)80686-X [retrieved on 1986-07-07]
- LIBURDY ET AL: "Antibody induced fluorescence enhancement of an N-(3-pyrene)maleimide conjugate of rabbit anti-human immunoglobulin G: Quantitation of human IgG", JOURNAL OF IMMUNOLOGICAL METHODS, ELSEVIER SCIENCE PUBLISHERS B.V.,AMSTERDAM, NL, vol. 28, no. 3-4, 24 July 1979 (1979-07-24), pages 233-242, XP023667367, ISSN: 0022-1759, DOI: 10.1016/0022-1759(79)90190-X [retrieved on 1979-07-24]
- TSUNEHIRO KITAGAWA ET AL: "Enzyme Coupled Immunoassay of Insulin Using a Novel Coupling Reagent", JOURNAL OF BIOCHEMISTRY, OXFORD UNIVERSITY PRESS, GB , vol. 79, no. 1 1 January 1976 (1976-01-01), pages 233-236, XP008178319, ISSN: 0021-924X Retrieved from the Internet: URL:http://jb.oxfordjournals.org/content/79/1/233.abstract
- ANNUNZIATO M E ET AL: "P-MALEIMIDOPHENYL ISOCYANATE: A NOVEL HETEROBIFUNCTIONAL LINKER FOR HYDROXYL TO THIOL COUPLING", BIOCONJUGATE CHEMISTRY, ACS, WASHINGTON, DC, US, vol. 4, no. 3, 1 May 1993 (1993-05-01), pages 212-218, XP002074803, ISSN: 1043-1802, DOI: 10.1021/BC00021A005
- JOACHIM DREVS ET AL: "In Vivo and In Vitro Efficacy of an Acid-Sensitive Albumin Conjugate of Adriamycin Compared to the Parent Compound in Murine Renal-Cell Carcinoma", DRUG DELIVERY, ACADEMIC PRESS, ORLANDO, FL, US, vol. 6, no. 2, 1 January 1999 (1999-01-01) , pages 89-95, XP008178359, ISSN: 1071-7544, DOI: 10.1080/107175499267002 [retrieved on 2008-09-29]
- OJU JEON ET AL: "Poly(l-lactide-co-glycolide) nanospheres conjugated with a nuclear localization signal for delivery of plasmid DNA", JOURNAL OF DRUG TARGETING, HARWOOD ACADEMIC PUBLISHERS GMBH, DE, vol. 15, no. 3, 1 January 2007 (2007-01-01), pages 190-198, XP008178360, ISSN: 1061-186X, DOI: 10.1080/10611860601143479 [retrieved on 2008-10-08]

## Description

The present disclosure relates to a method of conjugating an antibody as defined in the appended claims.

## BACKGROUND

Conjugation techniques are very important in the context of pharmaceuticals, for example a number of marketed drugs/biologicals are conjugated to a polyethylene glycol (PEG) molecule. The PEG has one of several functions in that it is thought to reduce immunogenicity of some molecules and/or increases the half-life of the molecules. Examples of marketed therapies include: include: peginesatide, pegloticase, certolizumab pegol (Cimzia), Methoxy polyethylene glycol-epoetin beta (Mircera), pegaptanib (Macugen), pegfilgrastim (Neulasta), pegvisomant (Somavert), peginterferon alfa-2a (Pegasys), Doxorubicin HCl liposome (Doxil/Caelyx), peginterferon alfa-2b (PegIntron), pegaspargase (Oncaspar), and pegademase bovine (Adagen).

Antibody drug conjugates (ADCs) are also an important category of therapeutic molecules. They are highly potent and similar to a "guided missile" in that an antibody component is conjugated to a payload, for example a cytotoxin, which is guided to the intended target by the antibody component. Examples of ADCs include brentuximab vedotin and trastuzumab emtansine. The linker formed in the conjugation chemistry is of vital importance in ADCs.

In a publication by Liburdy, a specific coupling reaction between a maleimide and a freely reactive lysine in an antibody was shown (Liburdy et al., J. of Immunological methods, 1979, 28, 233-242). Many of the conjugation technologies employ a maleimide element in a 1, 4 Michael addition reaction giving a succinimide component in the conjugated molecule. However, as discussed in WO2013/173337 this reaction is reversible and seems to exist as a dynamic equilibrium. That is to say purification does not solve the problem of providing a single pure entity.

There are some publications that suggest this occurs in plasma and part of the conjugated molecule may be replaced by serum albumin Alley et al Bioconjugate Chem. 2008, 19, 759-765. This is a problem, particularly in the context of ADCs.

One answer is to hydrolyse the resultant succinimide. However, this requires relatively harsh conditions, for example 50mM borate buffer at pH 9.2 and 45°C for about 12 hours. This can cause antibody aggregation/degradation and also degradation of the payload or warhead.

A solution to this problem is disclosed in WO2013/173337 (Seattle Genetics Inc) which employs a maleimide derivative with an amine substituent at the position beta to the nitrogen in the maleimide ring. The resultant succinimide can be hydrolysed under milder conditions. However, there appears to be significant maleimide hydrolysis prior to the thiol conjugation step. In addition the conjugation step is performed at a basic pH of about 8. The latter conditions can promote aggregation of proteinaceous materials, such as antibodies and antibody fragments, thereby causing loss of this precious material in the conjugation step. Furthermore, the ratio of payload to antibody generally has to be increased to ensure all the antibody is conjugated.

Amine-catalyzed deconjugation mechanisms are discussed in the paper by Md. Rowshon Alam et al (Bioconjugate Chem 2011, 22, 1673-1681), which shows that the Seattle Genetics strategy is not universally applicable (see scheme 2 therein).

An alternative solution is provided in WO2013/121175 which discloses use of bromo derivatives of maleimide in a so-called SN2 reaction. However, under low pH conditions a reactive species namely maleic anhydride is reformed, see for example page 54:

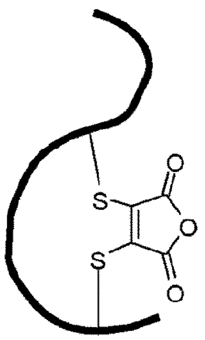

*"The pH was then changed by ultrafiltration... ... ..to pH 4. 5. The antibody conjugate was incubated at 37°C and aliquots were analysed after 2, 6, 24, 48 and 72 hours by LCMS. Doxorubicin was released upon linker disassembly and Trastuzumab-Fab-maleic anhydride formed."*

The present inventors believe that this instability in the conjugates, i.e. the ability to form the maleic ring, is a

disadvantage because there are a number of low pH environments in the body and this limits the utility of the molecules of WO2013/1211175 because the payload can become disconnected.

The present disclosure provides methods employing mild conditions which provide molecules which are stable after preparation and therefore suitable for *in vivo* administration and in particular, which are suitable for use in therapy.

## SUMMARY OF THE DISCLOSURE

Thus there is provided a method for preparing an antibody conjugated to a payload comprising the step of:

a) performing a Michael addition reaction with the maleimide entity in the molecule of formula (I):

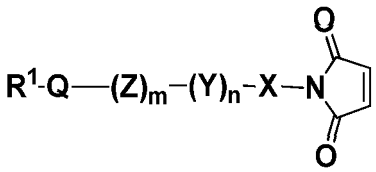

**(I)**

and an antibody molecule comprising at least one thiol group

wherein in formula (I):

- n is 0 or 1;
- m is 0 or 1;
- p is 0 or 1;
- q is 0 or an integer in the range 1 to 12, for example 1 to 6, such as 2 or 3;
- Q is a bond or a residue from a conjugation component;
- X is $C_{0-18}$ alkylene$C_{6-36}$ Aryl, wherein the aryl has 0, 1, 2, 3 or 4 substituent independently selected from the group comprising halogen, hydroxyl, $C_{1-6}$ alkyl, $C_{1-6}$ alkoxy, $-COOR^3$,$-COR^3$, -CN, $-CF_3$, $-NO_2$, $-SO_2$, $-SO_3$, $-NR^4R^5$, $-PO_4$, $-(OCH_2CH_2)_q-OR^3$;
- Y is C(O);
- Z is a saturated or unsaturated branched or unbranched $C_{1-30}$ alkylene chain, wherein one or more carbons (such as 1, 2, 3, 4, 5, 6, 7 or 8) are optionally independently replaced by -O-, N and the chain is optionally bears one or more (such as 1, 2, 3 or 4) oxo substituents;
- $R^1$ is H, a solid surface or a payload molecule;
- $R^3$ is H or $C_{1-6}$ alkyl;
- $R^4$ is H or $C_{1-6}$ alkyl;
- $R^5$ is H or $C_{1-6}$ alkyl; and pharmaceutically acceptable salts thereof,

b) wherein $R^1$ is a payload molecule or a solid surface the method includes a further step of hydrolysing the resultant thio-succinimide entity formed by the reaction of compound of formula (I) and the antibody, or

c) wherein $R^1$ is H the method comprises the further step of performing a conjugation with a payload or solid surface followed by hydrolysing the thio-succinimide entity formed by the reaction of compound of formula (I) and the antibody.

Advantageously the conjugates of the present disclosure can be prepared at acidic pHs such as about pH 5, and thio-succinimide hydrolysis can be performed under mild conditions. In certain embodiments the thio-succinimide in the conjugates can be rapidly hydrolysed under mild conditions. Furthermore once prepared the molecules of the present disclosure are stable in use, for example are stable in serum.

Without wishing to be bound be theory it is hypothesised that the resonance capabilities of the non-saturated system, such the aryl, phenyl, vinyl, alkynyl or combination thereof are important because they facilitate the mild hydrolysis. The schematic diagram in Figure 1 shows how an unsaturaturated system attached to the nitrogen in the succinimide ring can facilitate nucleophilic attack by water at either of the carbonyls in the ring.

In one embodiment the aryl bears 1, 2, 3 or 4 fluoro substituents.

## BRIEF DESCRIPTION OF THE FIGURES

**Figure 1** A schematic representation of hydrolysis of compounds of the disclosure
**Figure 1A** Spectra of Unreacted mAb
**Figure 1B** Spectra of Alkyl maleimide-PEG-biotin conjugate
**Figure 2A** Spectrum of Alkyl maleimide-PEG-biotin (comparator)
**Figure 2B** Spectrum of Phenyl maleimide-PEG-biotin
**Figure 2C** Spectrum of Fluorophenyl maleimide-PEG-biotin
**Figure 3** Conjugation Efficiency of x-Maleimide-PEG-biotins to T289C mAb at 22 oC
**Figure 4** Conjugation Efficiency of x-Maleimide-PEG-biotins to T289C mAb at 22 oC
**Figure 5** Conjugation Kinetics of x-Maleimide-PEG-biotins to T289C mAb, pH 5.5, at 22 oC
**Figure 6** Conjugation Kinetics of x-Maleimide-PEG-biotins to T289C mAb, pH 7.4, at 22∘C
**Figure 7** Conjugation Kinetics of x-Maleimide-PEG-biotins to T289C mAb, pH 8.6, at 22∘C
**Figure 8** Reaction Kinetics of x-Maleimide-PEG-Biotins with T289C mAb Following Pre-Incubation: Indirect Measurement of Maleimide Hydrolysis
**Figure 9** Maleimide Hydrolysis Kinetics pH 5.5, 22 oC
**Figure 10** Maleimide Hydrolysis Kinetics pH 7.4, 22 oC
**Figure 11** Maleimide Hydrolysis Kinetics pH 8.6, 22 oC
**Figure 12** Thiosuccinimide Hydrolysis pH 7.4, 22 °C, for PEG-biotin T289C mAb Conjugates
**Figure 13** Thiosuccinimide Hydrolysis pH 7.4, 37 °C, for PEG-biotin T289C mAb
**Figure 14** Thiosuccinimide Hydrolysis pH 8.6, 22 °C, for PEG-biotin T289C mAb
**Figure 15** x-Thiosuccinimide Hydrolysis for PEG-biotin T289C-mAb Conjugates After 1 Hour Incubation, n=3
**Figure 16** x-Thiosuccinimide-PEG-Biotin Hydrolysis for PEG-Biotin T289C mAb Conjugates in the Presence of Sodium Molybdate
**Figure 17** Sensitivity of T289C mAb Conjugates to Thiol Exchange in Buffer Containing Thiols
**Figure 18** Stability of PEG-Biotin T289C mAb Conjugates in Buffer Containing BME
**Figure 19** Thiosuccinimide Hydrolysis at pH 7.2, 37 °C for PEG-Biotin T289C mAb Conjugates Observed in BME Challenge Assay
**Figure 20** Relationship Between Thiosuccinimide Deconjugation and Hydrolysis Observed in the BME Challenge Assay
**Figure 21** Sensitivity of T289C mAb Conjugates to Thiol Exchange in Buffer after Mild Hydrolysis
**Figure 22** Analysis of mc-PAB-MMAE T289C mAb ADCs
**Figure 23** Stability of MMAE T289C mAb Conjugates in Buffer Containing Thiol (BME)
**Figure 24** Hydrolysis of MMAE-Thiosuccinimides in T289C mAb Conjugates
**Figure 25** Stability of MMAE T289C mAb Conjugates in Buffer Containing Thiol (BME)
**Figure 26** Comparison of PEG-biotin and MMAE Thiosuccinimide Hydrolysis Observed in the BME Challenge
**Figure 27** Comparison of Deconjugation in the Presence of β-Mercaptoethanol: PEG-Biotin vs. MMAE payload
**Figure 28** Stability of MMAE-T289C ADCs in Mouse Serum
**Figure 29** Stability of MMAE-T289C ADCs in Mouse Serum
**Figure 30** Activity of ADCs towards MDA-MB-361 cancer cells after incubation in mouse serum
**Figure 31** Alternative Format for Stabilization of Thiol-linked ADCs
**Figure 32** Mass Spectrometry Analysis of N-phenyl Maleimide-PEG-BCN-mAb Conjugate
**Figure 33** Mass Spectrometry Analysis of N-Fluorophenyl Maleimide-PEG-BCN-mAb Conjugate
**Figure 34** Mass Spectrometry Analysis of N-Alkyl Maleimide-PEG-BCN-mAb Conjugate
**Figure 35** Conjugation Efficiency of x-Maleimide-PEG-BCNs to T289C mAb
**Figure 36** Thiosuccinimide hydrolysis kinetics for x-maleimide-PEG-BCN conjugates
**Figure 37** Analysis of mAb-BCN-Ac4GlcNAz Conjugates
**Figure 38** Reactivity of mAb-BCN Conjugate After Storage
**Figure 39** Analysis of mAb-PBD Conjugates
**Figure 40** In Vitro Activity of mAb-BCN-PBD ADCs Towards MDA-MB-361 Breast Cancer Cells

## DETAILED DESCRIPTION OF THE DISCLOSURE

In one embodiment n is 0. In one embodiment n is 1. In one embodiment m is 0. In one embodiment m is 1.

In one embodiment X is $C_{0-18}$ alkylene$C_{6-10}$ Aryl
wherein the aryl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$.

In one embodiment X is $C_{0-15}$ alkylene$C_{6-10}$ Aryl
wherein the aryl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, -(CH2-O-CH2)q-O-$R^3$.

In one embodiment X is $C_{0-15}$ alkylene$C_{6-10}$ Aryl wherein the aryl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$.

In one embodiment X is $C_{0-15}$ alkylene$C_{6-10}$ Aryl, for example $C_{0-6}$ alkylene$C_{6-10}$ Aryl, such as $C_{6-10}$ Aryl, in particular phenyl.

Z is a saturated or unsaturated branched or unbranched $C_{1-25}$ alkylene chain, wherein one or more carbons (such as 1, 2, 3, 4, 5, 6, 7 or 8) are optionally independently replaced by -O-, N and the chain is optionally bears one or more (such as 1, 2, 3 or 4) oxo substituents.

Generally aryl, such as phenyl will be linked by a covalent bond directly to the nitrogen of the maleimide ring.

In one embodiment the aryl, such as phenyl bear the "$R^1$-Q-$(Z)_m$" containing substituent in the ortho, meta or para position, for example meta or para such as the para position.

In one embodiment the aryl such as phenyl may be substituted with one, two, three or four substituents which are independently selected from electron donating, electron withdrawing and neutral substituents. In one embodiment aryl, such as phenyl is substituted by one, two, three or four electron withdrawing groups. In one embodiment the aryl, such as phenyl is substituted by one, two, three or four electron donating groups. In one embodiment the Aryl, such as phenyl is substituted by one, two, three or four groups with neutral electronic properties.

In one embodiment aryl, such as phenyl is substituted with one, two, three or four substituents, for example independently selected from halogen, $C_{1-3}$ alkyl, trifluoromethyl, such a chloro or fluoro, in particular one, two, three or four fluoro atoms. Molecules containing one or more fluoro atoms may be useful in imaging techniques, such as positron emission tomography.

In one embodiment aryl, such as phenyl bears one substituent in the ortho, meta or para position, for example in the meta or para position of the ring connected to the maleimide.

In one embodiment aryl, such as phenyl bears two substitutents, for example in the meta, para position; meta, ortho position; ortho para position; meta, meta position; or ortho, ortho position of the ring connected to the maleimide.

In one embodiment the aryl, such as phenyl bears three substituents, for example in the ortho, meta and para position; ortho, meta, meta position; ortho, ortho, para position; or meta, meta, para position of the ring connected to the maleimide.

In one embodiment aryl, such as phenyl has no substituents.

Conjugation to a solid surface is an important aspect of the present disclosure because it allows polypetides to be attached to beads or plates, for example of synthetic material such as plastic or resin, to facilitate purification or screening, such as high-through-put screening.

In one embodiment $R^1$ is a payload molecule, examples of which are given below in the definition section. In one embodiment $R^1$ is H. In one embodiment $R^1$ is a solid surface.

In one embodiment the method according to the present disclosure, wherein n is 1, employs a compound of formula **(II):**

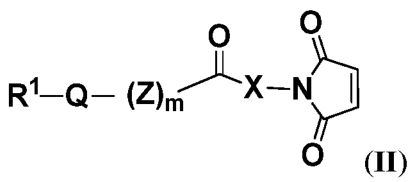

**(II)**

and pharmaceutically acceptable salts thereof, wherein $R^1$, Q, Z, X and m are defined above for compounds of formula **(I).**

In one embodiment the method according to the present disclosure, wherein n is 0, employs a compound of formula **(III):**

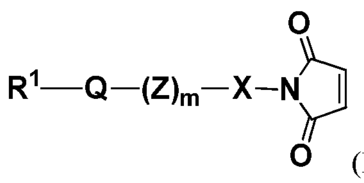

**(III)**

and pharmaceutically acceptable salts thereof, wherein $R^1$, Q, Z, X and m are defined above for compounds of formula **(I).**

In one embodiment the maleimide molecule is in a compound of formula **(IIa):**

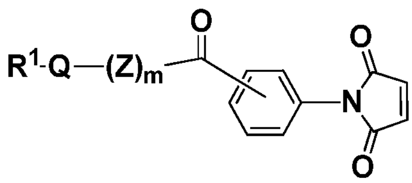

**(IIa)**

and pharmaceutically acceptable salts thereof, wherein $R^1$, Q, Z and m are defined above for compounds of formula (I), and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

In one embodiment the group $R^1Q(Z)_mC(O)$- is in the para position as shown in the compound of formula **(IIaa):**

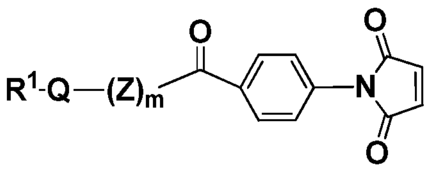

**(IIaa)**

and pharmaceutically acceptable salts thereof, wherein $R^1$, Q, Z and m are defined above for compounds of formula (I) and the phenyl has 0, 1, 2, 3 or 4 substituent independently selected from the group halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

In one embodiment the group $R^1Q(Z)_mC(O)$- is in the meta position as shown in the compound of formula **(IIaa'):**

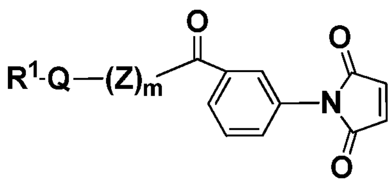

**(IIaa')**

and pharmaceutically acceptable salts thereof, wherein $R^1$, Q, Z and m are defined above for compounds of formula (I) and the phenyl has 0, 1, 2, 3 or 4 substituent independently selected from the group halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

In one embodiment the maleimide molecule is in a compound of formula **(IIaaa):**

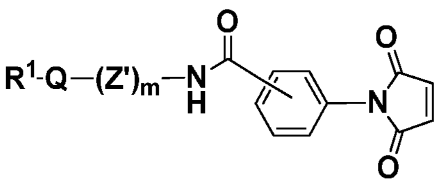

**(IIaaa)**

and pharmaceutically acceptable salts thereof, wherein $R^1$, Q, and m are defined above for compounds of formula (I), the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, - CN, $-CF_3$, $-NO_2$, $-SO_2$, $-SO_3$, $-NR^4R^5$, $-PO_4$ $-(OCH_2CH_2)_q-OR^3$, and Z' is a saturated or unsaturated branched or unbranched $C_{1-24}$ alkylene chain, wherein one or more carbons are optionally independently replaced by -O-, N and the chain is optionally bears one or more oxo substituents. In one embodiment the group $R^1Q(Z')_mNC(O)$- is in the para position. In one embodiment the group $R^1Q(Z')_mNC(O)$- is in the meta position.

In one embodiment the maleimide entity is in a molecule of formula **(IIb):**

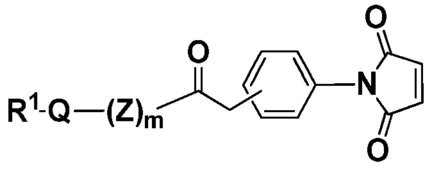
**(IIb)**

and pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

In one embodiment the group $R^1Q(Z)_mC(O)$- is in the para position as shown in the compound of formula **(IIbb):**

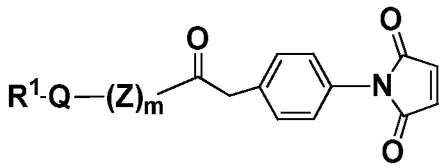
**(IIbb)**

and pharmaceutically acceptable salts thereof, wherein Q, Z, X, $R^1$ and m are defined above for compounds of formula (I).

In one embodiment the group $R^1Q(Z)_mC(O)$- is in the meta position as shown in the compound of formula **(IIbb'):**

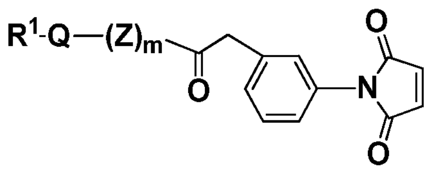
**(IIbb')**

and pharmaceutically acceptable salts thereof, wherein Q, Z, X, $R^1$ and m are defined above for compounds of formula (I).

In one embodiment the maleimide entity is in a molecule of formula **(IIbbb):**

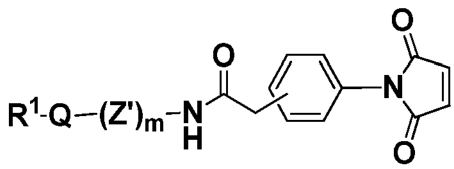
**(IIbbb)**

and pharmaceutically acceptable salts thereof, wherein $R^1$, Q and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$,

Z' is a saturated or unsaturated branched or unbranched $C_{1-24}$ alkylene chain, wherein one or more carbons are optionally independently replaced by -O-, N and the chain is optionally bears one or more oxo substituents. In on embodiment the group $R^1Q(Z')_mNC(O)$- is in the para position of the phenyl ring. In on embodiment the group $R^1Q(Z')_mNC(O)$- is in the meta position of the phenyl ring.

In one embodiment the maleimide molecule is in a compound of formula **(IIIa):**

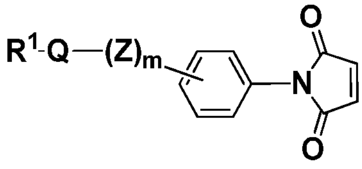
**(IIIa)**

and pharmaceutically acceptable salts thereof, wherein $R^1$, Q, Z and m are defined above for compounds of formula (I), and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$.

In one embodiment the group $R^1Q(Z)_m$- is in the para position as shown in the compound of formula **(IIIaa):**

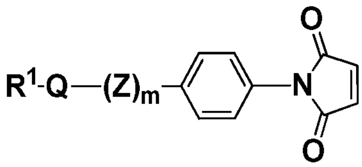

(IIIaa)

or pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$.

In one embodiment the group $R^1Q(Z)_m$- is in the meta position as shown in the compound of formula **(IIIaa'):**

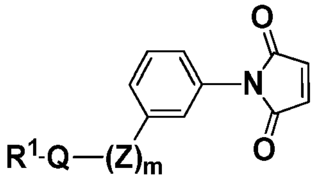

(IIIaa')

and pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$.

In one embodiment the maleimide molecule is in a compound of formula **(IIIaaa):**

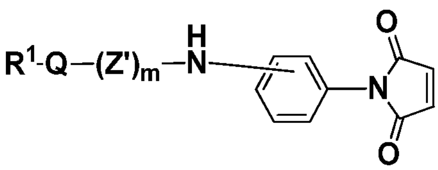

(IIIaaa)

and pharmaceutically acceptable salts thereof wherein $R^1$, Q, and m are defined above for compounds of formula (I), the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$, and $-(OCH_2CH_2)_q-OR^3$, and Z' is a saturated or unsaturated branched or unbranched $C_{1-24}$ alkylene chain, wherein one or more carbons are optionally independently replaced by -O-, N and the chain is optionally bears one or more oxo substituents. In one embodiment the $R^1Q(Z')_mNH$- is in the para position of the phenyl ring. In one embodiment the $R^1Q(Z')_mNH$- is in the meta position of the phenyl ring.

In one embodiment the fragment -QZ (i.e. where m is 1) or -QZ'NH (where m is 1), as appropriate within the given structure, represents: $-QOC(O)(CH_2CH_2O)_{1-8}(CH_2)_{0-1}NH$, for example $-QOC(O)(CH_2CH_2O)_{1-6}(CH_2)_{0-1}NH$ such as $-QOC(O)(CH_2CH_2O)_{2,3,4,5}(CH_2)_{0-1}NH$, more specifically $-QOC(O)(CH_2CH_2O)_4NH$.

In one embodiment Q is a bond. In one embodiment Q is a conjugation component. Conjugation component is an entity that can be employed to conjugate one molecule or fragment to another molecule or fragment.

In one embodiment Q comprises or is a function group for example selected from the group comprising, tetrazine, functionalized tetrazine, NHS ester (N-hydroxy succimide ester), tetrafluorophenyl ester, and combinations thereof.

In one embodiment the Q is a polymerizable function groupd for example selected from the group comprising an alkyne, tetrazine, derivatized tetrazine, lipid, nanoparticle, dendrimer, metal chelator and combinations thereof.

In one embodiment the conjugation components Q is a click chemistry component. Examples of click chemistry components include residues the Click-mates™ collection, such as:

- **Click-mates™ alkyne** which include 5-propargyloxy-dU CEP, 5-octadiynyl-du CEP, alkynyl-modifier-C6-dT CEP, 5-(propargyloxy)-2'-deoxyuridine, 5-(1,7-octadiyn-1-yl)-2'-deoxyuridine, 5-octadiynyl-TMS-dU CEP, 5-octadiynyl-TMS-dC CEP, 5-octadiynyl-dC CEP, 5-octadiynyl-TIPS-dU CEP; and
- **Click-easy® alkynes (for copper-free Click modifications)** which include BCN CEP I, BCN CEP II, BCN-N-hydroxysuccinimide ester I, BCN-N-hydroxysuccinimide ester II, MFCO-N-hydroxysuccinimide ester and MFCO CEP;

Residues as employed herein refer to the structure that is left after the relevant reaction to join the molecule to one or more other molecular fragments (components) has taken place.

In one embodiment the conjugation component is BCN.

In one embodiment the conjugation component Q is biotin. In one embodiment the Q is the fragment:

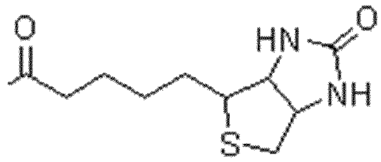

In one embodiment Z is independently selected from $-C_{1-12}$ alkylene, $-OC(O)(CH_2CH_2O)_{1-8}(CH_2)_{0-1}NH$, e.g. $-OC(O)(CH_2CH_2O)_{1-6}(CH_2)_{0-1}NH$, such as - $OC(O)(CH_2CH_2O)_{2,3,4,5}(CH_2)_{0-1}NH$, more specifically $-OC(O)(CH_2CH_2O)_4NH$.

In one embodiment Z' is are independently selected from $-C_{1-12}$ alkylene, $-OC(O)(CH_2CH_2O)_{1-8}(CH_2)_{0-1}$, for example $-OC(O)(CH_2CH_2O)_{1-6}(CH_2)_{0-1}$, such as $-OC(O)(CH_2CH_2O)_{2,3,4,5}(CH_2)_{0-1}$, more specifically $-OC(O)(CH_2CH_2O)_4$.

In one embodiment Y-X in molecules of formula (I) is C(O)CH2phenyl(maleimide or succinimide) and m is 0 and Q is a bond and the payload is linked via amide bond with the oxo group in the fragment Y-X.

In one embodiment the payload is linked via a labile bond, for example hydrazone (low pH release), disulfide, imine or similar.

reference given herein for compounds of formula (I) apply equally to compounds of other formulas described herein, as appropriate.

Pharmaceutically acceptable salts can be used, for example mineral acid salts, such as hydrochlorides, hydrobromides, phosphates and sulphates, or salts of organic acids, such as acetates, propionates, malonates and benzoates.

In one embodiment the maleimide entity is conjugated via a native cysteine in the antibody. In one embodiment the maleimide in conjugate via a cysteine engineered into the antibody (also referred to herein as an engineered cysteine). In one embodiment the cysteine is a solvent exposed cysteine.

In one embodiment one or more conjugation steps, for example conjugation of the maleimide to the antibody is performed at a pH in the range 5 to 9, for example pH 5.5 to 8.6, such as 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5 or 8.6 or a combination of one or more of said pH values.

In one embodiment one or more conjugation steps, for example conjugation of the maleimide to the antibody is performed at a temperature in the range about 4 to about 37°C, for example 8 to 37°C, 8 to 30°C, 8 to 25°C or 21 to 31°C, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 or 37°C or a combination of one or more said temperatures.

In one embodiment one or more conjugation steps are performed in a suitable buffer, for example a buffer selected from the group comprising phosphate buffer, citrate buffer, borate buffer, HEPES (4-(2-hydroxy ethyl)-1-piperazineethanesulfonic acid), MES (2-(N-morpholino)ethanesulfonic acid)), PIPES (piperazine-N,N'-bis(2-ethanesulfonic acid)), MOPS (3-(N-morpholino)propanesulfonic acid)), such as phosphate.

In one embodiment the buffer does not comprise a primary amine, for example TRIS and the like.

In one embodiment the efficiency of one or more conjugations reactions, for example the maleimide conjugation to the antibody is 50% or greater, for example 60, 65, 70, 75, 80, 85, 90, 91, 92, 93, 94, 95, 96, 97, 98, 99 or 100%.

Thus in one embodiment there is a low percentage of unconjugated antibody after the reaction, for example 5% or less, such as 4, 3, 2, 1% or less.

In one embodiment the ratio of a compound of formula (I) and other formulas described herein to antibody in the conjugation step is 1 to 1 respectively to 5 to 1, such as 2 to 1, 3 to 1 or 4 to 1.

In one embodiment the thiol-conjugate is further reacted with a payload, nanoparticle, or solid surface by Cu(I) catalized azide-alkyne cycloaddition (CuAAC) using copper salts, reducing agents such as sodium ascorbate, and/or Cu(I) stabilizing ligands such as; 2-[4-{(bis [(1 -tert-butyl- 1H-1,2,3 -triazol-4-yl)methyl] amino)-methyl}-1H-1,2,3-triazol-1-yl]ethyl hydrogen sulfate ( BTTES); tris[(1-benzyl-1H-1,2,3-triazol-4-yl)methyl]amine (TBTA); tris[(1-hydroxypropyl-1H-1,2,3-triazol-4-yl)methyl]amine (THTPA), and the like to form a triazole linkage.

In one embodiment, the thiol-conjugate is further reacted with a payload, nanoparticle, or solid surface by strain-promoted azide-alkyne cycloaddition (SPAAC) using cyclic alkynes such as biarylazacyclooctynone (BARAC); dibezocyclooctyne (DBCO), bicyclo[6.1.0]nonyne (BCN) and the like to form a triazole linkage.

In one embodiment, the thiol-conjugate is further reacted with a payload, nanoparticle, or solid surface by the tetrazine-transcyclooctene reaction to form a dihydropyrazine linkage.

In one embodiment a hydrolysis step employed in the method, for example the hydrolysis of the a resultant succinimide is performed at a pH in the range 7 to 12, for example pH7.4 to 9, such as 7.5, 7.6, 7.7, 7.8, 7.9, 8.0, 8.1, 8.2, 8.3, 8.4, 8.5, 8.6, 8.7, 8.8, 8.9 or 9.0 or a combination of one or more the same.

In one embodiment a buffer is employed in the hydrolysis step, for example a buffer selected from the group comprising phosphate buffer, citrate buffer, borate buffer, HEPES (4-(2-hydroxyethyl)-1-piperazineethanesulfonic acid), MES (2-(N-morpholino)ethanesulfonic acid)), PIPES (piperazine-N,N'-bis(2-ethanesulfonic acid)), MOPS (3-(N-morpholino)propanesulfonic acid)), such as phosphate.

In one embodiment one or more hydrolysis steps, for example hydrolysis of a succinimide is performed at a temperature in the range about 4 to about 37°C, for example 8 to 37°C, 8 to 30°C, 8 to 25°C or 21 to 31°C, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 or 37°C or a combination of one or more said temperatures.

In one embodiment one or more reactions in the present method, for example the conjugation reaction, are performed in the presence of a catalyst. Examples of catalysts include transition metal catalysts, such as copper, tin, inorganic oxides, such as molybdate.

In one embodiment the one or more reactions in the present method, for example the conjugation reaction do not employ a catalyst.

It should also be noted that under a wide range of conditions the hydrolysis occurs highly efficiently post-conjugation so conditions can readily be chosen such that the conjugation and hydrolysis take place sequentially in the same reaction. Indeed, this is desirable as it minimizes the number of steps. Altematively/additionally conditions for downstream steps (e.g., filtration, storage, buffer exchange, purification over a column) can be selected to ensure the conjugate is hydrolyzed. In one embodiment the hydrolysis does not employ a combination of low pH and low temperature.

Hydrolysed molecules, prepared from the method of the disclosure include the molecules of formula (IV):

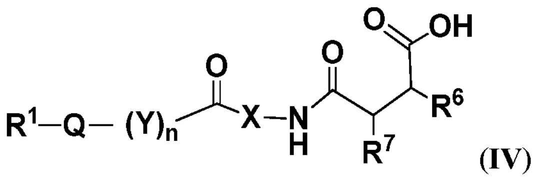

**(IV)**

and pharmaceutically acceptable salts thereof wherein:

Q, $R^1$, Z and m are defined above for compounds of formula (I) and
$R^6$ is H or a antibody residue, $R^7$ is H or a antibody residue, wherein at least one of $R^6$ or $R^7$ is a antibody residue and the other is H.

The hydrolysis can occur at one of two positions as shown in scheme 1:

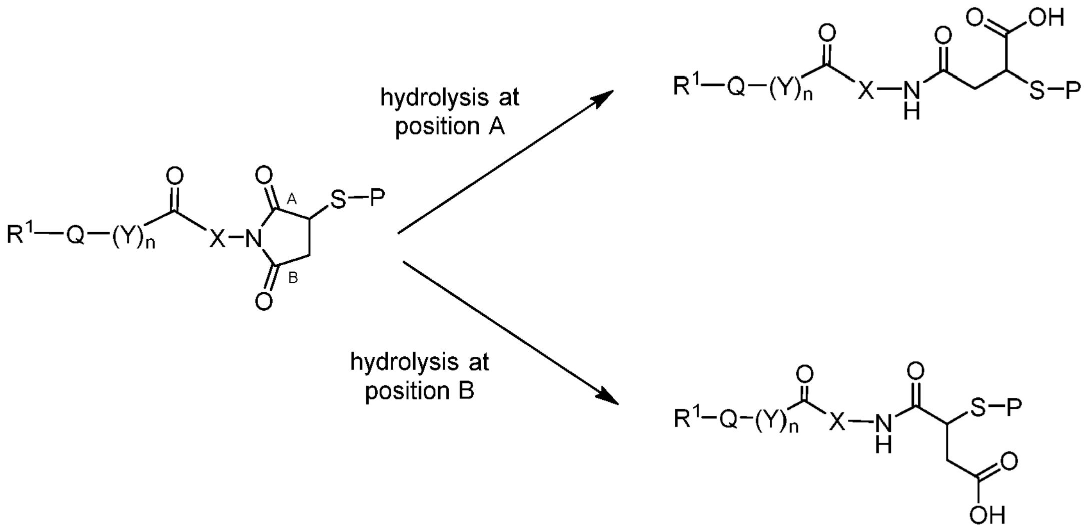


wherein P represents a antibody.

In one embodiment the molecules prepared by the method of the disclosure are stable, or example physically, chemically and thermally stable. Evidence of physically instability is, for example aggregation, which can be measured by routine techniques, such as size exclusion chromatography. Evidence of chemical instability is, for example degradation or disintegration of the molecule, such as disconnection of the payload. Evidence of thermal instability is, for example denaturing.

In one embodiment in hydrolysed molecules prepared by the method of the present disclosure there is 20% loss or less of the payload after incubation with beta-mercaptoethanol, for example 10% loss or less, such as 5% loss or less. In one embodiment the loss occurs over a period 1 to 36 hours, such as 2 to 24 hours.

In one embodiment in hydrolysed molecules prepared by the method of the present disclosure there is 20% loss or less of the payload after incubation with serum (for example murine serum or bovine serum), for example 10% loss or less, such as 5% loss or less. In one embodiment the loss occurs over a period of 1 to 36 hours, such as 24 hours.

In one embodiment the reduction in potency, for example as measured by IC50, upon incubation of serum, is 20% or less, for example 10% or less, such as 5% or less, more specifically 4%, 3%, 2% or 1%.

**DEFINITIONS**

A Michael addition (also referred to herein as a 1,4-Michael addition) is a nucleophilic addition of a carbanion or other anion to an alpha-beta unsaturated carbonyl compound.

Alkyl as used herein refers to straight chain or branched chain alkyl, such as, without limitation, methyl, ethyl, propyl, iso-propyl, butyl, and tert-butyl. In one embodiment alkyl refers to straight chain alkyl. Alkyl is a terminal group that is to say at the end of chain. $C_{1-6}$ alkyl includes $C_1$, $C_2$, $C_3$, $C_4$, $C_5$ and $C_6$.

Alkoxy as used herein refers to straight or branched chain alkoxy, for example methoxy, ethoxy, propoxy, butoxy. Alkoxy as employed herein also extends to embodiments in which the oxygen atom is located within the alkyl chain, for example $-CH_2CH_2OCH_3$ or $-CH_2OCH_3$. In one embodiment the alkoxy is linked through oxygen to the remainder of the molecule. In one embodiment the disclosure relates to straight chain alkoxy.

Aryl as employed herein refers to a a $C_6$ to $C_{36}$ carbocyclic system, for example a $C_6$ to $C_{10}$ carbocyclic system comprising at least one aromatic ring, for example napthylene, phenyl, indene, indane, 1, 2, 3,4-tetrahydronapthylene, azulene, for example napthylene or phenyl, such as phenyl.

Heteroaryl is a $_{5-36}$ membered aromatic carbocylic ring or bicyclic ring system comprising one or more, (for example 1, 2, 3 or 4) heteroatoms independently selected from O, N and S. Examples of heteroaryls include: pyrrole, oxazole, thiazole, isothiazole, imidazole, pyrazole, isoxazole, pyridine, pyridazine, pyrimidine, pyrazine, benzothiophene, benzofuran, or 1, 2, 3 and 1, 2, 4 triazole. In a bicyclic ring system the definition of heteroaryl will be satisfied if at least one ring contains a heteroatom and at least one ring is aromatic. The heteroaryl may be linked to the remainder of the molecule through a carbocyclic ring or a ring comprising a heteroatom.

Oxo as used herein refers to C=O and will usually be represented as C(O).

In relation to a saturated or unsaturated, branched or unbranched $C_{1-25}$ alkylene chain, wherein at least one carbon (for example 1, 2 or 3 carbons, suitably 1 or 2, in particular 1) is replaced by a heteroatom selected from O, N and said chain is optionally, substituted by one or more groups oxo, groups, it will be clear to persons skilled in the art that the heteroatom may replace a primary, secondary or tertiary carbon, that is $CH_3$, $-CH_2-$ or a -CH- or a branched

carbon group, as technically appropriate. This definition also applies where this language is employed in the context of chains of lengths other than 1 to 25 carbons.

Alkylene is a linking group i.e. joined at each end to other parts/components of the molecule. $C_{1-25}$ alkylene includes $C_1$, $C_2$, $C_3$, $C_4$, $C_5$, $C_6$, $C_7$, $C_8$ or $C_9$, $C_{10}$, $C_{11}$, $C_{12}$, $C_{13}$, $C_{14}$, $C_{15}$, $C_{16}$, $C_{17}$, $C_{18}$, $C_{19}$, $C_{20}$, $C_{21}$, $C_{22}$, $C_{23}$, $C_{24}$ and $C_{25}$.

$C_0$ is where the "feature" is absent.

Halo or halogen as employed herein refers to iodo, bromo, chloro or fluoro, such as fluoro.

Conjugated as employed herein refers to a compound/molecule formed by joining two compounds or molecules or fragments together.

Hydrolysis as employed herein refers to reactions with cleave rings or molecules by the addition of a water molecule.

Electron withdrawing group as employed herein refers to a substituent that draws electrons (or at attracts electrons) from the entity to which is it attached.

Electron donating group as employed herein refers a substitutent that gives "donates" eletrons to the entity to which it is attached.

A neutral group in the context of the present disclosure does not relate to charge as such but refers to electron donating or withdrawing properties. These groups have no impact on the electronic properties on the entity to which they are attached.

**Antibodies for use in the present disclosure**

The terms "antibody" or "immunoglobulin," as used interchangeably herein, include whole antibodies and any antigen binding fragment or single chains thereof.

A typical antibody comprises at least two heavy (H) chains and two light (L) chains interconnected by disulfide bonds. Each heavy chain is comprised of a heavy chain variable region (abbreviated herein as VH, VH region, or VH domain) and a heavy chain constant region. The heavy chain constant region is comprised of three or four constant domains, CH1, CH2, CH3, and CH4. The Fc region includes the polypeptides comprising the constant region of an antibody excluding the first constant region immunoglobulin domain, and fragments thereof. Thus, for IgG the "Fc region" refers to CH2 and CH3 and optionally all or a portion of the flexible hinge region N-terminal to these domains. The term "Fc region" can refer to this region in isolation, or this region in the context of an antibody, antibody fragment, or Fc fusion protein.

Each light chain is comprised of a light chain variable region (abbreviated herein as VL, VL region, or VL domain) and a light chain constant region. The light chain constant region is comprised of one domain, CL.

The VH and VL regions can be further subdivided into regions of hyper variability, termed Complementarity Determining Regions (CDR), interspersed with regions that are more conserved, termed framework regions (FW). Each VH and VL is composed of three CDRs and four FWs, arranged from amino-terminus to carboxy-terminus in the following order: FW1, CDR1, FW2, CDR2, FW3, CDR3, FW4. Framework regions can be designated according to their respective VH and VL regions. Thus, e.g., VH-FW1 would refer to the first framework region of VH. The variable regions of the heavy and light chains contain a binding domain that interacts with an antigen. The constant regions of the antibodies can mediate the binding of the immunoglobulin to host tissues or factors, including various cells of the immune system (e.g., effector cells) and the first component (C1q) of the classical complement system.

The term "antibody" means an immunoglobulin molecule or antigen binding fragment thereof that recognizes and specifically binds to a target, such as a protein, polypeptide, peptide, carbohydrate, polynucleotide, lipid, or combinations of the foregoing through at least one antigen recognition site (also referred to as a binding site) within the variable region of the immunoglobulin molecule. As used herein, the term "antibody" encompasses intact polyclonal antibodies, intact monoclonal antibodies, antibody fragments (such as Fab, Fab', F(ab')2, and Fv fragments), single chain antibody fragments (scFv and disulfide stabilized scFv (dsFv)), multispecific antibodies such as bispecific antibodies generated from at least two different antibodies or multispecific antibodies formed from antibody fragments (see, e.g, PCT Publications WO96/27011, WO2007/024715WO2009018386, WO2009/080251, WO2013006544, WO2013/070565, and WO2013/096291), chimeric antibodies, humanized antibodies, human antibodies, fusion proteins comprising an antigen-binding fragment of an antibody, and any other modified immunoglobulin molecule comprising an antigen-binding fragment so long as the antibodies exhibit the desired biological activity.

An antibody can be of any the five major classes of immunoglobulins: IgA, IgD, IgE, IgG, and IgM, or subclasses (isotypes) (e.g. IgG1, IgG2, IgG3, IgG4, IgA1 and IgA2), or allotype (e.g., Gm, e.g., G1m(f, z, a or x), G2m(n), G3m(g, b, or c), Am, Em, and Km(1, 2 or 3)). The different classes of immunoglobulins have different and well known subunit structures and three-dimensional configurations. Antibodies may be derived from any mammal, including, but not limited to, humans, monkeys, pigs, horses, rabbits, dogs, cats, mice, etc., or other animals such as birds (e.g. chickens).

The terms "antigen-binding fragment" refers to a fragment comprising antigenic determining variable regions

of an intact antibody. It is known in the art that the antigen binding function of an antibody can be performed by fragments of a full-length antibody. Examples of antibody fragments include, but are not limited to Fab, Fab', F(ab')2, Fv fragments, scFvs, linear antibodies, single chain antibodies, and multispecific antibodies formed from antibody fragments.

Thus in one embodiment the antibody used in the present invention may comprise a complete antibody molecule having full length heavy and light chains or a fragment thereof and may be, but are not limited to Fab, modified Fab, Fab', modified Fab', $F(ab')_2$, Fv, single domain antibodies (e.g. VH or VL or VHH), scFv, bi, tri or tetra-valent antibodies, Bis-scFv, diabodies, triabodies, tetrabodies, combinations of the same and epitope-binding fragments of any of the above.

Other antibodies specifically contemplated are "oligoclonal" antibodies which are a predetermined mixture of distinct monoclonal antibodies. See, *e.g.,* PCT publication WO 95/20401; U.S. Pat. Nos. 5,789,208 and 6,335,163. Preferably oligoclonal antibodies consist of a predetermined mixture of antibodies against one or more epitopes are generated in a single cell. More preferably oligoclonal antibodies comprise a plurality of heavy chains capable of pairing with a common light chain to generate antibodies with multiple specificities (e.g., PCT publication WO 04/009618). Oligoclonal antibodies are particularly useful when it is desired to target multiple epitopes on a single target molecule. Those skilled in the art will know or can determine what type of antibody or mixture of antibodies is applicable for an intended purpose and desired need.

Other moieties specifically contemplated for use in the present disclosure are small, engineered protein domains such as scaffold (see for example, U.S. Patent Publication Nos. 2003/0082630 and 2003/0157561). Scaffolds are based upon known naturally-occurring, non-antibody domain families, specifically protein extracellular domains, which typically of small size (~100 to ~300 AA) and containing a highly structured core associated with variable domains of high conformational tolerance allowing insertions, deletions or other substitutions. These variable domains can create a putative binding interface for any targeted protein. In general, the design of a generic protein scaffold consists of two major steps: (i) selection of a suitable core protein with desired features and (ii) generation of complex combinatorial libraries by mutagenizing a portion or all of the domains accepting high structural variability, display of these libraries in an appropriate format (i.e., phage, ribosome, bacterial, or yeast) and screening of the library for mutagenized scaffold having the desired binding characteristics (e.g. target specificity and/or affinity). The structure of the parental scaffolds can be highly diverse and include highly structured protein domains including but not limited to, FnIII domains (e.g., AdNectins, see, e.g., Protein Eng. Des. Sel. 18, 435-444 (2005), US2008/00139791, and WO 2005/056764, TN3, see e.g., WO2009/058379 and WO2011/130324); Z domains of protein A (Affibody, see, e.g., Protein Eng. Des. Sel. 17,455-462 (2004) and EP1641818A1); domain A from LDL receptor (Avimers, see, e.g., Nature Biotechnology 23(12), 1556 - 1561 (2005) and Expert Opinion on Investigational Drugs 16(6), 909-917 (June 2007)); Ankyrin repeat domains (DARPins, J. Mol. Biol. 332,489-503 (2003), PNAS (2003) and Biol. 369, (2007) and WO02/20565); C-type lectin domains (Tetranectins, see, e.g., WO02/48189). If desired two or more such engineered scaffold domains can be linked together, to form a multivalent binding protein. The individual domains can target a single type of protein or several, depending upon the use/disease indication.

Virtually any molecule (or a portion thereof, e.g., subunits, domains, motifs or a epitope) may be targeted by and/or incorporated into a moiety including, but not limited to, integral membrane proteins including ion channels, ion pumps, G-protein coupled receptors, structural proteins; adhesion proteins such as integrins; transporters; proteins involved in signal tranduction and lipid-anchored proteins including G proteins, enzymes such as kinases including membrane-anchored kinases, membrane-bound enzymes, proteases, lipases, phosphatases, fatty acid synthetases, digestive enzymes such as pepsin, trypsin, and chymotrypsin, lysozyme, polymerases; receptors such as hormone receptors, lymphokine receptors, monokine receptors, growth factor receptors, cytokine receptors; cytokines; and more.

In some aspects a antibody employed in the present disclosure targets and/or incorporates all or a portion (e.g., subunits, domains, motifs or a epitope) of a growth factor, a cytokine, a cytokine-related protein, a growth factor, a receptor ligand or a receptor selected from among, for example, BMP1, BMP2, BMP3B (GDF10), BMP4, BMP6, BMP8, CSF1(M-CSF), CSF2 (GM-CSF), CSF3 (G-CSF), EPO, FGF1 ($\alpha$FGF), FGF2 ($\beta$FGF), FGF3 (int-2), FGF4 (HST), FGF5, FGF6 (HST-2), FGF7 (KGF), FGF9, FGF10, FGF11, FGF12, FGF12B, FGF14, FGF16, FGF17, FGF19, FGF20, FGF21, FGF23, FGFR, FGFR1, FGFR2, FGFR3, FGFR4, FGFRL1, FGFR6, IGF1, IGF2, IGF1R, IGF2R, IFNA1, IFNA2, IFNA4, IFNA5, IFNA6, IFNA7, IFNAR1, IFNAR2, IFNB1, IFNG, IFNW1, FIL1, FIL1 (EPSILON), FIL1 (ZETA), IL1A, IL1B, IL2, IL3, IL4, IL5, IL6, IL7, IL8, IL9, IL10, IL11, IL12A, IL12B, IL13, IL14, IL15, IL16, IL17, IL17B, IL18, IL19, IL20, IL22, IL23, IL24, IL25, IL26, IL27, IL28A, IL28B, IL29, IL30, IL2RA, IL1R1, IL1R2, IL1RL1, IL1RL2, IL2RA, IL2RB, IL2RG, IL3RA, IL4R, IL5RA, IL6R, IL7R, IL8RA, IL8RB, IL9R, IL10RA, IL10RB, IL11RA, IL12RB1, IL12RB2, IL13RA1, IL13RA2, IL15RA, IL17R, IL17RA, IL17RB, IL17RC, IL17RD, IL18R1, IL20RA, IL20RB, IL21R, IL22R, IL22RA1, IL23R, IL27RA, IL28RA, PDGFA, PDGFB, PDGFRA, PDGFRB, TGFA, TGFB1, TGFB2, TGFB3, TGFBR1, TGFBR2, TGFBR3, ACVRL1, GFRA1, LTA (TNF-beta), LTB, TNF (TNF-alpha), TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TNFSF10 (TRAIL), TNFSF11 (TRANCE), TNFSF12 (APO3L), TNFSF13 (April), TNFSF13B, TNFSF14 (HVEM-L), TNFSF15 (VEGI), TNFSF18, TNFRSF1A, TNFRSF1B, TNFRSF10A (Trail-receptor), TNFRSF10B (Trail-receptor 2), TNFRSF10C (Trail-receptor 3), TNFRSF10D (Trail-receptor 4), FIGF (VEGFD), VEGF, VEGFB, VEGFC, KDR, FLT1, FLT4, NRP1, IL1HY1, IL1RAP, IL1RAPL1, IL1RAPL2,

IL1RN, IL6ST, IL18BP, IL18RAP, IL22RA2, AIF1, HGF, LEP (leptin), PTN, ALK and THPO.

In some aspects a antibody employed in the present disclosure targets and/or incorporates all or a portion (e.g., subunits, domains, motifs or a epitope) of a chemokine, a chemokine receptor, or a chemokine-related protein selected from among, for example, CCL1(I-309), CCL2 (MCP-1/MCAF), CCL3 (MIP-1a), CCL4 (MIP-1b), CCL5 (RANTES), CCL7 (MCP-3), CCL8 (mcp-2), CCL11 (eotaxin), CCL13 (MCP-4), CCL15 (MIP-1d), CCL16 (HCC-4), CCL17 (TARC), CCL18 (PARC), CCL19 (MIP-3b), CCL20 (MIP-3a), CCL21 (SLC/exodus-2), CCL22 (MDC/STC-1), CCL23 (MPIF-1), CCL24 (MPIF-2/eotaxin-2), CCL25 (TECK), CCL26 (eotaxin-3), CCL27 (CTACK/ILC), CCL28, CXCL1(GRO1), CXCL2 (GRO2), CXCL3 (GRO3), CXCL5 (ENA-78), CXCL6 (GCP-2), CXCL9 (MIG), CXCL10 (IP 10), CXCL11 (I-TAC), CXCL12 (SDF1), CXCL13, CXCL14, CXCL16, PF4 (CXCL4), PPBP (CXCL7), CX3CL1 (SCYD1), SCYE1, XCL1 (lymphotactin), XCL2 (SCM-1b), BLR1 (MDR15), CCBP2 (D6/JAB61), CCR1 (CKR1/HM145), CCR2 (mcp-1RB/RA), CCR3 (CKR3/CMKBR3), CCR4, CCR5 (CMKBR5/ChemR13), CCR6 (CMKBR6/CKR-L3/STRL22/DRY6), CCR7 (CKR7/EBI1), CCR8 (CMKBR8/TER1/ CKR-L1), CCR9 (GPR-9-6), CCRL1 (VSHK1), CCRL2 (L-CCR), XCR1 (GPR5/CCXCR1), CMKLR1, CMKOR1 (RDC1), CX3CR1 (V28), CXCR4, GPR2 (CCR10), GPR31, GPR81 (FKSG80), CXCR3 (GPR9/CKR-L2), CXCR6 (TYMSTR/STRL33/Bonzo), HM74, IL8RA (IL8Ra), IL8RB (IL8Rb), LTB4R (GPR16), TCP10, CKLFSF2, CKLFSF3, CKLFSF4, CKLFSF5, CKLFSF6, CKLFSF7, CKLFSF8, BDNF, C5R1, CSF3, GRCC10 (C10), EPO, FY (DARC), GDF5, HIF1A, IL8, PRL, RGS3, RGS13, SDF2, SLIT2, TLR2, TLR4, TREM1, TREM2, and VHL.

In some aspects a antibody employed in the present disclosure targets and/or incorporates all or a portion (e.g., subunits, domains, motifs or a epitope) of a protein selected from among, for example renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; clotting factors such as factor VII, factor VIIIC, factor IX, tissue factor (TF), and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and - beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; protein A or D; rheumatoid factors; a neurotrophic factor such as bone-derived neurotrophic factor (BDNF), neurotrophin-3,-4,-5, or -6 (NT-3, NT-4, NT-5, or NT-6), or a nerve growth factor; epidermal growth factor (EGF); insulin-like growth factor binding proteins; CD proteins such as CD2, CD3, CD4, CD 8, CD11a, CD14, CD18, CD19, CD20, CD22, CD23, CD25, CD33, CD34, CD40, CD40L, CD52, CD63, CD64, CD80 and CD147; erythropoietin; osteoinductive factors; immunotoxins; superoxide dismutase; T-cell receptors; surface membrane proteins; decay accelerating factor; viral antigen such as, for example, a portion of the AIDS envelope, e.g.,gp120; transport proteins; homing receptors; addressins; regulatory proteins; cell adhesion molecules such as LFA-1, Mac 1, p150.95, VLA-4, ICAM-1, ICAM-3 and VCAM, a4/p7 integrin, and (Xv/p3 integrin including either a or subunits thereof, integrin alpha subunits such as CD49a, CD49b, CD49c, CD49d, CD49e, CD49f, alpha7, alpha8, alpha9, alphaD, CD11a, CD11b, CD51, CD11c, CD41, alphaIIb, alphaIELb; integrin beta subunits such as, CD29, CD 18, CD61, CD104, beta5, beta6, beta7 and beta8; Integrin subunit combinations including but not limited to, $\alpha V\beta 3$, $\alpha V\beta 5$ and $\alpha 4\beta 7$; a member of an apoptosis pathway; IgE; blood group antigens; flk2/flt3 receptor; obesity (OB) receptor; mpl receptor; CTLA-4; protein C; an Eph receptor such as EphA2, EphA4, EphB2, etc.; a Human Leukocyte Antigen (HLA) such as HLA-DR; complement proteins such as complement receptor CR1, CIRq and other complement factors such as C3, and C5; a glycoprotein receptor such as GpIb$\alpha$, GPIIb/IIIa and CD200.

Also contemplated are moieties that specifically bind and/or comprises cancer antigens including, but not limited to, ALK receptor (pleiotrophin receptor), pleiotrophin, KS 1/4 pan-carcinoma antigen; ovarian carcinoma antigen (CA125); prostatic acid phosphate; prostate specific antigen (PSA); melanoma-associated antigen p97; melanoma antigen gp75; high molecular weight melanoma antigen (HMW-MAA); prostate specific membrane antigen; carcinoembryonic antigen (CEA); polymorphic epithelial mucin antigen; human milk fat globule antigen; colorectal tumor-associated antigens such as: CEA, TAG-72, CO17-1A, GICA 19-9, CTA-1 and LEA; Burkitt's lymphoma antigen-38.13; CD19; human B-lymphoma antigen-CD20; CD33; melanoma specific antigens such as ganglioside GD2, ganglioside GD3, ganglioside GM2 and ganglioside GM3; tumor-specific transplantation type cell-surface antigen (TSTA); virally-induced tumor antigens including T-antigen, DNA tumor viruses and Envelope antigens of RNA tumor viruses; oncofetal antigen-alpha-fetoprotein such as CEA of colon, 5T4 oncofetal trophoblast glycoprotein and bladder tumor oncofetal antigen; differentiation antigen such as human lung carcinoma antigens L6 and L20; antigens of fibrosarcoma; human leukemia T cell antigen-Gp37; neoglycoprotein; sphingolipids; breast cancer antigens such as EGFR (Epidermal growth factor receptor); NY-BR-16, NY-BR-16, HER2 antigen (p185HER2), and HER3; polymorphic epithelial mucin (PEM); malignant human lymphocyte antigen-APO-1; differentiation antigen such as I antigen found in fetal erythrocytes; primary endoderm I antigen found in adult erythrocytes; preimplantation embryos; I(Ma) found in gastric adenocarcinomas; M18, M39 found in breast epithelium; SSEA-1 found in myeloid cells; VEP8; VEP9; Myl; VIM-D5; D156-22 found in colorectal cancer; TRA-1-85

(blood group H); SCP-1 found in testis and ovarian cancer; C14 found in colonic adenocarcinoma; F3 found in lung adenocarcinoma; AH6 found in gastric cancer; Y hapten; Ley found in embryonal carcinoma cells; TL5 (blood group A); EGF receptor found in A431 cells; E1 series (blood group B) found in pancreatic cancer; FC10.2 found in embryonal carcinoma cells; gastric adenocarcinoma antigen; CO-514 (blood group Lea) found in Adenocarcinoma; NS-10 found in adenocarcinomas; CO-43 (blood group Leb); G49 found in EGF receptor of A431 cells; MH2 (blood group ALeb/Ley) found in colonic adenocarcinoma; 19.9 found in colon cancer; gastric cancer mucins; T5A7 found in myeloid cells; R24 found in melanoma; 4.2, GD3, D1.1, OFA-1, GM2, OFA-2, GD2, and M1:22:25:8 found in embryonal carcinoma cells and SSEA-3 and SSEA-4 found in 4 to 8-cell stage embryos; Cutaneous Tcell Lymphoma antigen; MART-1 antigen; Sialy Tn (STn) antigen; Colon cancer antigen NY-CO-45; Lung cancer antigen NY-LU-12 variant A; Adenocarcinoma antigen ART1; Paraneoplastic associated brain-testis-cancer antigen (onconeuronal antigen MA2; paraneoplastic neuronal antigen); Neuro-oncological ventral antigen 2 (NOVA2); Hepatocellular carcinoma antigen gene 520; TUMOR-ASSOCIATED ANTIGEN CO-029; Tumor-associated antigens MAGE-C1 (cancer/testis antigen CT7), MAGE-B1 (MAGE-XP antigen), MAGE-B2 (DAM6), MAGE-2, MAGE-4a, MAGE-4b and MAGE-X2; Cancer-Testis Antigen (NY-EOS-1) and fragments of any of the above-listed polypeptides.

In one embodiment the polypeptide employed is recombinant. A "recombinant" polypeptide or protein refers to a polypeptide or protein produced via recombinant DNA technology. Recombinantly produced polypeptides and proteins expressed in engineered host cells are considered isolated for the purpose of this disclosure, as are native or recombinant polypeptides which have been separated, fractionated, or partially or substantially purified by any suitable technique. The polypeptides disclosed herein can be recombinantly produced using methods known in the art. Alternatively, the proteins and peptides disclosed herein can be chemically synthesized.

**Payload Molecules**

Payload as employed herein refers to a molecule or component, which is intended for "delivery" to a target region location by conjugation to the antibody. Generally the payload will generally be an effector molecule, for example selected from the group consisting of a toxin, for example a cytotoxin, such as a chemotherapeutic agent, a drug, a pro-drug, an enzyme, an immunomodulator, an anti-angiogenic agent, a pro- apoptotic agent, a cytokine, a hormone, an antibody or fragment thereof, synthetic or naturally occurring polymers, nucleic acids and fragments thereof e.g. DNA, RNA and fragments thereof (e.g., an antisense molecule or a gene), radionuclides, particularly radioiodide, radioisotopes, chelated metals, nanoparticles and reporter groups such as fluorescent compounds or compounds which may be detected by NMR or ESR spectroscopy.

In one embodiment the payload is selected from the group comprising a toxin, drug, radionuclide, immunomodulator, cytokine, lymphokine, chemokine, growth factor, tumor necrosis factor, hormone, hormone antagonist, enzyme, oligonucleotide, DNA, RNA, siRNA, RNAi, microRNA, peptide nucleic acid, photoactive therapeutic agent, anti-angiogenic agent, pro-apoptotic agent, non-natural amino acid, peptide, lipid, , a polymer, carbohydrate, scaffolding molecule, fluorescent tag, visualization peptide, biotin, serum half-life extender, capture tag, chelating agent, solid support, or a combination thereof.

In one embodiment the payload is a drug molecule (also referred to herein as a drug). Examples of drug molecules for use in the present disclosure include nitrogen mustard, ethylenimine derivative, alkyl sulfonates, nitrosourea, gemcitabine, triazene, folic acid analog, anthracycline, taxane, COX-2 inhibitor, pyrimidine analog, purine analog, antibiotic, enzyme inhibitor, epipodophyllotoxin, platinum coordination complex, vinca alkaloid, substituted urea, methyl hydrazine derivative, adrenocortical suppressant, hormone antagonist, endostatin, taxol, camptothecin, doxorubicin, doxorubicin analog, antimetabolite, alkylating agent, antimitotic, anti-angiogenic agent, tyrosine kinase inhibitor, mTOR inhibitor, heat shock protein (HSP90) inhibitor, proteosome inhibitor, HDAC inhibitor, pro-apoptotic agent, methotrexate, CPT-11, or a combination thereof, and wherein conjugation is.

In particular aspects, the drug is amifostine, cisplatin, dacarbazine, dactinomycin, mechlorethamine, streptozocin, cyclophosphamide, carmustine, lomustine, doxorubicin lipo, gemcitabine, daunorubicin, daunorubicin lipo, procarbazine, mitomycin, cytarabine, etoposide, methotrexate, 5-fluorouracil, vinblastine, vincristine, bleomycin, paclitaxel, docetaxel, aldesleukin, asparaginase, busulfan, carboplatin, cladribine, 10-hydroxy-7-ethyl-camptothecin (SN38), gefitinib, dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil aromatase inhibitors, and combinations thereof.

In one embodiment the drug is selected from the group comprising alkylphosphocholines, topoisomerase I inhibitors, taxoids and suramin.

In one embodiment toxin comprise cytotoxins or cytotoxic agents including any agent that is detrimental to (*e.g.* kills) cells. Examples include aplidin, anastrozole, azacytidine,, bortezomib, bryostatin-1, busulfan, combrestatins, carmustine, dolastatins, epothilones, staurosporin, maytansinoids, spongistatins, rhizoxin, halichondrins, roridins, hemiasterlins, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

In one embodiment the drug (also a cytotoxin in this instance) comprises an antimetabolites (*e.g.* methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), carboplatin, anthracyclines (*e.g.* daunorubicin (formerly daunomycin) and doxorubicin or doxorubicin glucuronide), antibiotics (*e.g.* dactinomycin (formerly actinomycin), bleomycin, mithramycin, anthramycin (AMC), calicheamicins or duocarmycins), and anti-mitotic agents (*e.g.* vincristine and vinblastine).

In some aspects, the drug is an auristatin (U.S. Pat. Nos. 5,635,483; 5,780,588), for example, MMAE (monomethyl auristatin E) or MMAF (monomethyl auristatin F). In other aspects, the drug is a dolastatin or dolastatin peptidic analog or derivative. Dolastatins and auristatins have been shown to interfere with microtubule dynamics, GTP hydrolysis, and nuclear and cellular division (Woyke et al., Antimicrob. Agents and Chemother. 45:3580-3584 (2001)) and have anticancer activity (U.S. Pat. No. 5,663,149). The dolastatin or auristatin drug moiety can be attached to the conjugate compound through the N (amino) terminus or the C (carboxyl) terminus of the peptidic drug moiety. See, e.g., Intl. Publ. No. WO2002/088172. In other aspects, the drug is a maytansinoid. In some aspects, the maytansinoid is N 2'-deacetyl-N 2'-(3-mercapto-1-oxopropyl)-maytansine (DM1), N 2'-deacetyl-N2'-(4-mercapto-1-oxopentyl)-maytansine (DM3) or N 2'-deacetyl-N 2'(4-methyl-4-mercapto-1-oxopentyl)-maytansine (DM4). Maytansinoids are mitotic inhibitors which act by inhibiting tubulin polymerization. Maytansine was first isolated from the east African shrub *Maytenus serrata* (U.S. Pat. No. 3,896,111). Subsequently, it was discovered that certain microbes also produce maytansinoids, such as maytansinol and C-3 maytansinol esters (U.S. Pat. No. 4,151,042). Synthetic maytansinol and derivatives and analogues thereof are disclosed, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines. Conjugates containing maytansinoids, methods of making same, and their therapeutic use are disclosed, for example, in U.S. Pat. Nos. 5,208,020, 5,416,064 and European Patent EP0425235B1; Liu et al., Proc. Natl. Acad. Sci. USA 93:8618-8623 (1996) (described immunoconjugates comprising a maytansinoid designated DM1); and Chari et al., Cancer Research 52:127-131 (1992).

Maytansinoids are well known in the art and can be synthesized by known techniques or isolated from natural sources. Suitable maytansinoids are disclosed, for example, in U.S. Pat. No. 5,208,020. Exemplary maytansinoid drug moieties include those having a modified aromatic ring, such as: C-19-dechloro (U.S. Pat. No. 4,256,746) prepared by lithium aluminum hydride reduction of ansamytocin P2); C-20-hydroxy (or C-20-demethyl)+/-C-19-dechloro (U.S. Pat. Nos. 4,361,650 and 4,307,016) (prepared by demethylation using Streptomyces or Actinomyces or dechlorination using LAH); and C-20-demethoxy, C-20-acyloxy (-OCOR), +/-dechloro (U.S. Pat. No. 4,294,757) (prepared by acylation using acyl chlorides). and those having modifications at other positions. Exemplary maytansinoid drug moieties also include those having modifications such as: C-9-SH, prepared by the reaction of maytansinol with H2S or P2S5 (U.S. Pat. No. 4,424,219); C-14-alkoxymethyl(demethoxy/CH2OR) (U.S. Pat. No. 4,331,598); C-14-hydroxymethyl or acyloxymethyl (CH2OH or CH2OAc), prepared from *Nocardia* (U.S. Pat. No. 4,450,254); C-15-hydroxy/acyloxy, prepared by the conversion of maytansinol by *Streptomyces* (U.S. Pat. No. 4,364,866); C-15-methoxy, isolated from *Trewia nudiflora* (U.S. Pat. Nos. 4,313,946 and 4,315,929); C-18-N-demethyl, prepared by the demethylation of maytansinol by *Streptomyces* (U.S. Pat. Nos. 4,362,663 and 4,322,348); and 4,5-deoxy, prepared by the titanium trichloride/LAH reduction of maytansinol (U.S. Pat. No. 4,371,533). Many positions on maytansine compounds are known to be useful as the linkage position, depending upon the type of link. For example, for forming an ester linkage, the C-3 position having a hydroxyl group, the C-14 position modified with hydroxymethyl, the C-15 position modified with a hydroxyl group and the C-20 position having a hydroxyl group are all suitable.

In some aspects, the drug is calicheamicin. The calicheamicin family of antibiotics is capable of producing double-stranded DNA breaks at sub-picomolar concentrations. For the preparation of conjugates of the calicheamicin family see, e.g., U.S. Pat. Nos. 5,712,374, 5,714,586, 5,739,116, 5,767,285, 5,770,701, 5,770,710, 5,773,001, 5,877,296. Structural analogues of calicheamicin that can be used include, but are not limited to, $\gamma$1I, $\alpha$2I, $\alpha$3I, N-acetyl-$\gamma$1I, PSAG and 011 (Hinman et al., Cancer Research 53:3336-3342 (1993), Lode et al., Cancer Research 58:2925-2928 (1998) and the aforementioned U.S. patents to American Cyanamid). In some aspects, the drug is tubulysin. Tubulysins are members of a class of natural products isolated from myxobacterial species (Sasse et al., J. Antibiot. 53:879-885 (2000)). As cytoskeleton interacting agents, tubulysins are mitotic poisons that inhibit tubulin polymerization and lead to cell cycle

arrest and apoptosis (Steinmetz et al., Chem. Int. Ed. 43:4888-4892 (2004); Khalil et al., ChemBioChem. 7:678-683 (2006); Kaur et al., Biochem. J. 396: 235-242 (2006)). Tubulysins are extremely potent cytotoxic molecules, exceeding the cell growth inhibition of any clinically relevant traditional chemotherapeutic, e.g., epothilones, paclitaxel, and vinblastine. Furthermore, they are potent against multidrug resistant cell lines (Domling et al., Mol. Diversity 9:141-147 (2005)). These compounds show high cytotoxicity tested against a panel of cancer cell lines with $IC_{50}$ values in the low picomolar range; thus, they are of interest as anticancer therapeutics. See, *e.g.,* Intl. Publ. No. WO/2012019123. Tubulysin conjugates are disclosed, *e.g.,* in U.S. Pat. No. 7,776,814.

In some aspects, the drug is a pyrrolobenzodiazepine (PBD). PBDs are relatively small molecules and some have the ability to recognize and covalently bind to specific sequences in the minor groove of DNA and thus exhibit antibiotic/antitumor activity. A number of PBDs and derivatives thereof are known in the art, for example, PBD dimers (e.g., SJG-136 or SG2000), C2-unsaturated PBD dimers, pyrrolobenzodiazepine dimers bearing C2 aryl substitutions (e.g., SG2285), PBD dimer pro-drug that is activated by hydrolysis (e.g., SG2285), and polypyrrole-PBD (e.g., SG2274). PBDs are further described in Intl. Publ. Nos. WO2000/012507, WO2007/039752, WO2005/110423, WO2005/085251, and WO2005/040170, and U.S. Pat. No. 7,612,062.

In some aspects, the toxin comprises, for example, abrin, brucine, cicutoxin, diphteria toxin, botulinum toxin, shiga toxin, endotoxin, tetanus toxin, pertussis toxin, anthrax toxin, cholera toxin, falcarinol, alpha toxin, geldanamycin, gelonin, lotaustralin, ricin, strychnine, tetrodotoxin, saponin, ribonuclease (RNase), DNase I, *Staphylococcal* enterotoxin-A, pokeweed antiviral protein, *Pseudomonas* exotoxin, *Pseudomonas* endotoxin, or a combination thereof. In other aspects, the toxin comprises, for example, modeccin A chain, alpha-sarcin, *Aleurites fordii* proteins, dianthin proteins, *Phytolaca americana* proteins (PAPI, PAPII, and PAP-S), *Momordica charantia* inhibitor, curcin, crotin, *Saponaria officinalis* inhibitor, mitogellin, restrictocin, phenomycin, neomycin, tricothecenes, or a combination thereof. See, for example, Intl. Publ. No. WO1993/021232. In some aspects, the chelating agent is DTPA, EC, DMSA, EDTA, Cy-EDTA, EDTMP, DTPA, CyDTPA, Cy2DTPA, BOPTA, DTPA-MA, DTPA-BA, DTPMP, DOTA, TRITA, TETA, DOTMA, DOTA-MA, HP-DO3A, pNB-DOTA, DOTP, DOTMP, DOTEP, DOTPP, DOTBzP, DOTPME, HEDP, DTTP, an N3S triamidethiol, DADS, MAMA, DADT, an N2S4 diaminetetrathiol, an N2P2 dithiol-bisphosphine, a 6-hydrazinonicotinic acid, a propylene amine oxime, a tetraamine, a cyclam, or a combination thereof.

In one embodiment the drug is an auristatin, a tubulysin or a pyrrolobenzodiazepine (PBD).

In one embodiment the auristatin is MMAE (monomethyl auristatin E) or MMAF (monomethyl auristatin F).

In one embodiment the drug is a maytansinoid, for example N 2'-deacetyl-N 2'-(3-mercapto-1-oxopropyl)-maytansine (DM1), N 2'-deacetyl-N2'-(4-mercapto-1-oxopentyl)-maytansine (DM3) or N 2'-deacetyl-N 2'(4-methyl-4-mercapto-1-oxopentyl)-maytansine (DM4).

Examples of radionuclides include $^{3}H$, $^{11}C$, $^{13}N$, $^{15}O$, $^{18}F$, $^{32}P$, $^{33}P$, $^{35}S$, $^{47}Sc$, $^{51}Cr$, $^{54}Mn$, $^{57}Co$, $^{58}Co$, $^{59}Fe$, $^{62}Cu$, $^{65}Zn$, $^{67}Cu$, $^{67}Ga$, $^{68}Ge$, $^{75}Br$, $^{75}Se$, $^{76}Br$, $^{77}Br$, $^{77}As$, $^{80m}Br$, $^{85}Sr$, $^{89}Sr$, $^{90}Y$, $^{95}Ru$, $^{97}Ru$, $^{99}Mo$ and $^{99m}Tc$, $^{103}Pd$, $^{103m}Rh$, $^{103}Ru$, $^{105}Rh$, $^{105}Ru$, $^{107}Hg$, $^{109}Pd$, $^{109}Pt$, $^{111}Ag$, $^{111}In$, $^{112}In$, $^{113m}In$, $^{113}Sn$, $^{115}In$, $^{117}Sn$, $^{119}Sb$, $^{121m}Te$, $^{121}I$, $^{122m}Te$, $^{125m}Te$, $^{125}I$, $^{126}I$, $^{131}I$, $^{133}I$, $^{133}Xe$, $^{140}La$, $^{142}Pr$, $^{143}Pr$, $^{149}Pm$, $^{152}Dy$, $^{153}Sm$, $^{153}Gd$, $^{159}Gd$, $^{161}Ho$, $^{161}Tb$, $^{165}Tm$, $^{166}Dy$, $^{166}Ho$, $^{167}Tm$, $^{168}Tm$, $^{169}Er$, $^{169}Yb$, $^{175}Yb$, $^{177}Lu$, $^{186}Re$, $^{188}Re$, $^{188}W$, $^{189m}Os$, $^{189}Re$, $^{192}Ir$, $^{194}Ir$, $^{197}Pt$, $^{198}Au$, $^{199}Au$, $^{201}T1$, $^{203}Hg$, $^{211}At$, $^{211}Bi$, $^{211}Pb$, $^{212}Pb$, $^{212}Bi$, $^{213}Bi$, $^{215}Po$, $^{217}At$, $^{219}Rn$, $^{221}Fr$, $^{223}Ra$, $^{224}Ac$, $^{225}Ac$, $^{225}Fm$, $^{252}Cf$ and a combination thereof.

In one embodiment the radionuclide is selected from the group comprising or consisting of chromium ($^{51}Cr$), cobalt ($^{57}Co$), fluorine ($^{18}F$), gadolinium ($^{153}Gd$, $^{159}Gd$), germanium ($^{68}Ge$), holmium ($^{166}Ho$), indium ($^{115}In$, $^{113}In$, $^{112}In$, $^{111}In$), iodine ($^{131}I$, $^{125}I$, $^{123}I$, $^{121}I$), lanthanum ($^{140}La$), lutetium ($^{177}Lu$), manganese ($^{54}Mn$), molybdenum ($^{99}Mo$), palladium ($^{103}Pd$), phosphorous ($^{32}P$), praseodymium ($^{142}Pr$), promethium ($^{149}Pm$), rhenium ($^{186}Re$, $^{188}Re$), rhodium ($^{105}Rh$), ruthenium ($^{97}Ru$), samarium ($^{153}Sm$), scandium ($^{47}Sc$), selenium ($^{75}Se$), strontium ($^{85}Sr$), sulfur ($^{35}S$), technetium ($^{99}Tc$), thallium ($^{201}Tl$), tin ($^{113}Sn$, $^{117}Sn$), tritium ($^{3}H$), xenon ($^{133}Xe$), ytterbium ($^{169}Yb$, $^{175}Yb$), yttrium ($^{90}Y$), zinc ($^{65}Zn$), or a combination thereof.

In one embodiment the radionuclide is attached to the conjugate compound of the present disclosure by a chelating agent.

In one embodiment $R^1$ is a serum half-life extender, for example comprising albumin, albumin binding polypeptide, PAS, the $\beta$ subunit of the C-terminal peptide (CTP) of human chorionic gonadotropin, polyethylene glycol (PEG), hydroxyethyl starch (HES), XTEN, albumin-binding small molecules, or a combination thereof.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific naturally occurring polymers include lactose, hyaluronic acid, heparan sulphate, chondroitin sulphate, alginate, cellulose amylose, dextran, glycogen or derivatives thereof.

In some embodiments, the polymer is polyethylene glycol (PEG), branched PEG, polysialic acid (PSA), hydroxyalkyl starch (HAS), hydroxylethyl starch (HES), carbohydrate, polysaccharides, pullulane, chitosan, hyaluronic acid, chondroitin sulfate, dermatan sulfate, starch, dextran, carboxymethyl-dextran, polyalkylene oxide (PAO), polyalkylene glycol (PAG), polypropylene glycol (PPG) polyoxazoline, poly acryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC). In some embodiments, the polymer is polyethylene glycol. In one embodiment of the invention, the polyethylene glycol has a molecular weight range of 300 to 10,000,000, 500 to 100,000, 1000 to 50,000, 1500 to 30,000, 2,000 to 20,000 Da, 3,000 to 5,000 Da, and 4,000 to 5,000 Da. In other embodiments, the polyethylene glycol has a molecular weight of about 1,000 Da, about 1,500 Da, about 2,000 Da, about 3,000 Da, about 4,000 Da, about 5,000 Da, about 10,000 Da, or about 20,000 Da.

In one embodiment $R^1$ or the antibody (in particular $R^1$) comprises a visualization label. Visualization labels include, without limitation, a chromophore, a fluorophore, a fluorescent protein, a phosphorescent dye, a tandem dye, a particle, a hapten, an enzyme, a radioisotope, or a combination thereof.

In one embodiment the visualization label is a visualization peptide. In some aspects, the visualization peptide enables visualization or localization of the conjugate compound in vitro, in vivo, ex vivo, or any combination thereof. In some aspects, the visualization peptide is, for example, a biotin acceptor peptide, a lipoic acid acceptor peptide, a fluorescent protein, a cysteine-containing peptide for ligation of a biarsenical dye or for conjugating metastable technetium, a peptide for conjugating europium clathrates for fluorescence resonance energy transfer (FRET)-based proximity assays, or any combination thereof. In some aspects, the fluorescent protein is, for example, green fluorescent protein (GFP), red fluorescent protein (RFP), yellow fluorescent protein (YFP), enhanced green fluorescent protein (EGFP), enhanced yellow fluorescent protein (EYFP), or any combination thereof. In some aspects, the fluorescent protein is a phycobiliprotein or a derivative thereof. Fluorescent proteins, especially phycobiliprotein, are useful for creating tandem dye labeled labeling reagents. These tandem dyes comprise a fluorescent protein and a fluorophore for the purposes of obtaining a larger stokes shift where the emission spectra is farther shifted from the wavelength of the fluorescent protein's absorption spectra. This can be effective for detecting a low quantity of a target in a sample where the emitted fluorescent light is maximally optimized, in other words little to none of the emitted light is reabsorbed by the fluorescent protein. For this to work, the fluorescent protein and fluorophore function as an energy transfer pair where the fluorescent protein emits at the wavelength that the fluorophore absorbs at and the fluorophore then emits at a wavelength farther from the fluorescent proteins than could have been obtained with only the fluorescent protein. A functional combination can be phycobiliproteins and sulforhodamine fluorophores, or sulfonated cyanine fluorophores as known in the art. The fluorophore sometimes functions as the energy donor and the fluorescent protein is the energy acceptor.

In other aspects, the biarsenical dye is 4',5'-bis(1,3,2-dithioarsolan-2-yl)fluorescein (FlAsH). In some aspects, the biotin acceptor peptide facilitates conjugation of avidin- and streptavidin-based reagents. In some aspects, the lipoic acid acceptor peptide facilitates conjugation of thiol-reactive probes to bound lipoic acid or direct ligation of fluorescent lipoic acid analogs.

In one embodiment $R^1$ or the antibody (in particular $R^1$) comprises a fluorescent tag. In some aspects, the fluorescent tag comprises, for example, a fluorescein-type dye, a rhodamine-type dye, dansyl-type dye, a lissamine-type dye, a cyanine-type dye, a phycoerythrin-type dye, a Texas Red-type dye, or any combination thereof. Fluorophores suitable for conjugation to the cysteine-engineered antibodies or antigen-binding fragments thereof disclosed herein include, without limitation; a pyrene (including any of the corresponding derivative compounds), an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine (including any corresponding compounds), a carbocyanine (including any corresponding compounds), a carbostyryl, a porphyrin, a salicylate, an anthranilate, an azulene, a perylene, a pyridine, a quinoline, a borapolyazaindacene (including any corresponding compounds), a xanthene (including any corresponding compounds), an oxazine (including any corresponding compounds) or a benzoxazine, a carbazine (including any corresponding compounds), a phenalenone, a coumarin (including an corresponding compounds disclosed), a benzofuran (including an corresponding compounds) and benzphenalenone (including any corresponding compounds) and derivatives thereof. As used herein, oxazines include resorufins (including any corresponding compounds), aminooxazinones, diaminooxazines, and their benzo-substituted analogs, or any combination thereof.

In certain aspects, the fluorophores include, for example, xanthene (rhodol, rhodamine, fluorescein and derivatives thereof) coumarin, cyanine, pyrene, oxazine, borapolyazaindacene, or any combination thereof. In some embodiments, such fluorophores are, for example, sulfonated xanthenes, fluorinated xanthenes, sulfonated coumarins, fluorinated coumarins, sulfonated cyanines, or any combination thereof. Also included are dyes sold under the tradenames, and generally known as, ALEXA FLUOR®, DYLIGHT®, CY DYES®, BODIPY®, OREGON GREEN®, PACIFIC BLUE®, IRDYES®, FAM®, FITC®, and ROX®.

The choice of the fluorophore attached via a linker "Z" as disclosed herein will determine the absorption and fluorescence emission properties of the final compound. Physical properties of a fluorophore label that can be used include, but are not limited to, spectral characteristics (absorption, emission and stokes shift), fluorescence intensity,

lifetime, polarization and photo-bleaching rate, or combination thereof. All of these physical properties can be used to distinguish one fluorophore from another, and thereby allow for multiplexed analysis. In certain aspects, the fluorophore has an absorption maximum at wavelengths greater than 480 nm. In some aspects, the fluorophore absorbs at or near 488 nm to 514 nm (particularly suitable for excitation by the output of the argon-ion laser excitation source) or near 546 nm (particularly suitable for excitation by a mercury arc lamp). In some aspects, a fluorophore can emit in the NIR (near infrared region) for tissue or whole organism applications. Other desirable properties of the fluorescent label can include cell permeability and low toxicity, for example if labeling of the antibody is to be performed in a cell or an organism (e.g., a living animal).

In one embodiment $R^1$ or the antibody (in particular $R^1$) comprises a capture tag. In some aspects, the capture tag is biotin or a His6 tag. Biotin is useful because it can function in an enzyme system to further amplify a detectable signal, and it can also function as a tag to be used in affinity chromatography for isolation purposes. For detection purposes, an enzyme conjugate that has affinity for biotin can be used, such as avidin-HRP.

Subsequently a peroxidase substrate can be added to produce a detectable signal. In addition to biotin, other haptens can be used, including hormones, naturally occurring and synthetic drugs, pollutants, allergens, effector molecules, growth factors, chemokines, cytokines, lymphokines, amino acids, peptides, chemical intermediates, nucleotides and the like.

In one embodiment $R^1$ comprises an enzyme. Enzymes are effective labels because amplification of the detectable signal can be obtained resulting in increased assay sensitivity. The enzyme itself often does not produce a detectable response but functions to break down a substrate when it is contacted by an appropriate substrate such that the converted substrate produces a fluorescent, colorimetric or luminescent signal. Enzymes amplify the detectable signal because one enzyme on a labeling reagent can result in multiple substrates being converted to a detectable signal. The enzyme substrate is selected to yield the measurable product, e.g., colorimetric, fluorescent or chemiluminescence. Such substrates are extensively used in the art and are known in the art.

In some embodiments, colorimetric or fluorogenic substrate and enzyme combination uses oxidoreductases such as horseradish peroxidase and a substrate such as 3,3'-diaminobenzidine (DAB) and 3-amino-9-ethylcarbazole (AEC), which yield a distinguishing color (brown and red, respectively). Other colorimetric oxidoreductase substrates that yield detectable products include, but are not limited to: 2,2-azino-bis(3-ethylbenzothiazoline-6-sulfonic acid) (ABTS), o- phenylenediamine (OPD), 3,3',5,5'-tetramethylbenzidine (TMB), o-dianisidine, 5-aminosalicylic acid, 4-chloro-1 -naphthol. Fluorogenic substrates include, but are not limited to, homovanillic acid or 4-hydroxy-3-methoxyphenylacetic acid, reduced phenoxazines and reduced benzothiazines, including Amplex® Red reagent and its variants and reduced dihydroxanthenes, including dihydrofluoresceins and dihydrorhodamines including dihydrorhodamine 123.

The present disclosure extends to employing peroxidase substrates that are tyramides represent a unique class of peroxidase substrates in that they can be intrinsically detectable before action of the enzyme but are "fixed in place" by the action of a peroxidase in the process described as tyramide signal amplification (TSA). These substrates are extensively utilized to label targets in samples that are cells, tissues or arrays for their subsequent detection by microscopy, flow cytometry, optical scanning and fluorometry.

The present disclosure extends to a colorimetric (and in some cases fluorogenic) substrate and enzyme combination sometimes uses a phosphatase enzyme such as an acid phosphatase, an alkaline phosphatase or a recombinant version of such a phosphatase in combination with a colorimetric substrate such as 5-bromo-6- chloro-3-indolyl phosphate (BCIP), 6-chloro-3-indolyl phosphate, 5-bromo-6-chloro-3-indolyl phosphate, p-nitrophenyl phosphate, or o-nitrophenyl phosphate or with a fluorogenic substrate such as 4-methylumbelliferyl phosphate, 6,8-difluoro-7-hydroxy-4-methylcoumarinyl phosphate (DiFMUP, U.S. Pat. No. 5,830,912) fluorescein diphosphate, 3-O-methylfluorescein phosphate, resorufin phosphate, 9H-(1,3-dichloro-9,9-dimethylacridin-2-one-7-yl) phosphate (DDAO phosphate), or ELF 97, ELF 39 or related phosphates.

The disclosure also extends to $R^1$ comprising a glycosidase, in particular beta-galactosidase, beta-glucuronidase and beta-glucosidase, are additional suitable enzymes. Appropriate colorimetric substrates include, but are not limited to, 5- bromo-4-chloro-3-indolyl beta-D-galactopyranoside (X-gal) and similar indolyl galactosides, glucosides, and glucuronides, o-nitrophenyl beta-D-galactopyranoside (ONPG) and p-nitrophenyl beta-D-galactopyranoside. In some embodiments, fluorogenic substrates include resorufin beta-D-galactopyranoside, fluorescein digalactoside (FDG), fluorescein diglucuronide and their structural variants, 4-methylumbelliferyl beta-D-galactopyranoside, carboxyumbelliferyl beta-D- galactopyranoside and fluorinated coumarin beta-D-galactopyranosides. Additional enzymes include, but are not limited to, hydrolases such as cholinesterases and peptidases, oxidases such as glucose oxidase and cytochrome oxidases, and reductases for which suitable substrates are known.

Enzymes and their appropriate substrates that produce chemiluminescence are useful for incorporation into molecules of the present disclosure. These include, but are not limited to, natural and recombinant forms of luciferases and aequorins. Chemiluminescence-producing substrates for phosphatases, glycosidases and oxidases such as those containing stable dioxetanes, luminol, isoluminol and acridinium esters are additionally productive. wherein such heterologous moiety is a nucleic acid. The nucleic acid can be selected from the group consisting of DNA, RNA, short interfering

RNA (siRNA), microRNA, hairpin or nucleic acid mimetics such as peptide nucleic acids. In certain aspects, the conjugated nucleic acid is at least 10, at least 20, at least 30, at least 40, at least 50 , at least 60 at least 100, at least 200, at least 500, at least 1000, at least 5000, or more base pairs. The conjugated nucleic acid can be single stranded. In various aspects, the conjugated nucleic acid can be double stranded. In some aspects, the conjugated nucleic acid encodes an open reading frame. In some aspects, the open reading frame encoded by the conjugated nucleic acid corresponds to an apoptosis inducing protein, a viral protein, an enzyme, or a tumor suppressor protein. Techniques for delivery of such nucleic acids to cells are known in the art.

**Other Definitions**

Before describing the provided embodiments in detail, it is to be understood that this disclosure is not limited to specific compositions or process steps, and as such can vary. As used in this specification and the appended claims, the singular forms "a", "an" and "the" include plural referents unless the context clearly dictates otherwise. The terms "a" (or "an"), as well as the terms "one or more," and "at least one" can be used interchangeably herein.

Furthermore, "and/or" where used herein is to be taken as specific disclosure of each of the two specified features or components with or without the other. Thus, the term and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects, which can be had by reference to the specification as a whole. Accordingly, the terms defined immediately below are more fully defined by reference to the specification in its entirety.

Amino acids are referred to herein by either their commonly known three letter symbols or by the one-letter symbols recommended by the IUPAC-IUB Biochemical Nomenclature Commission. Nucleotides, likewise, are referred to by their commonly accepted single-letter codes.

The term "subject" refers to any animal (*e.g.,* a mammal), including, but not limited to humans, non-human primates, rodents, and the like, which is to be the recipient of a particular treatment. Typically, the terms "subject" and "patient" can be used interchangeably in reference to a human subject.

The term "pharmaceutical composition" refers to a preparation which is in such form as to permit the biological activity of the active ingredient (*e.g.,* a conjugate compound disclosed herein) to be effective, and which contains no additional components which are unacceptably toxic to a subject to which the composition would be administered. Such composition may comprise one or more pharmaceutically acceptable excipients. Such composition can be sterile.

An "effective amount" of a conjugate compound as disclosed herein is an amount sufficient to carry out a specifically stated purpose. An "effective amount" can be determined empirically and in a routine manner, in relation to the stated purpose.

The term "therapeutically effective amount" refers to an amount of conjugate compound disclosed herein or other drug effective to "treat" a disease or disorder in a subject or mammal.

The word "label" when used herein refers to a detectable compound or composition which is conjugated directly or indirectly to an engineered antibody or fragment thereof disclosed herein (*e.g.,* a cysteine engineered antibody or fragment thereof) so as to generate a "labeled" conjugate compound. The label can be detectable by itself (*e.g.,* radioisotope labels or fluorescent labels) or, in the case of an enzymatic label, can catalyze chemical alteration of a substrate compound or composition that is detectable.

Terms such as "treating" or "treatment" or "to treat" refer to both (1) therapeutic measures that cure, slow down, lessen symptoms of, and/or halt progression of a diagnosed pathologic condition or disorder and (2) prophylactic or preventative measures that prevent and/or slow the development of a targeted pathologic condition or disorder. Thus, those in need of treatment include those already with the disorder; those prone to have the disorder; and those in whom the disorder is to be prevented. In certain aspects, a subject is successfully "treated" for a disease or condition, for example, cancer, according to the methods of the present disclosure if the patient shows, *e.g.,* total, partial, or transient remission of the disease or condition, for example, a certain type of cancer.

The terms "polynucleotide" and "nucleic acid" are used interchangeably herein and refer to polymers of nucleotides of any length, including DNA and RNA. The nucleotides can be deoxyribonucleotides, ribonucleotides, modified

nucleotides or bases, and/or their analogs, or any substrate that can be incorporated into a polymer by DNA or RNA polymerase. A polynucleotide can comprise modified nucleotides, such as methylated nucleotides and their analogs.

As used herein, the term "vector" refers to a construct, which is capable of delivering, and in some aspects, expressing, one or more gene(s) or sequence(s) of interest in a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells.

As used herein, the term "comprising" in context of the present specification should be interpreted as "including".

"Employed in the present disclosure" as used herein refers to employed in the method disclosed herein, employed in the molecules including intermediates disclosed herein or both, as appropriate to the context of the term used.

It is understood that wherever aspects are described herein with the language "comprising," otherwise analogous aspects described in terms of "consisting of" and/or "consisting essentially of" are also provided.

Any positive embodiment or combination thereof described herein may be the basis of a negative exclusion i.e. a disclaimer.

**Compositions**

The present disclosure extends to processes of preparing compositions comprising a molecule described herein (such as hydrolysed molecules of the disclosure), for example preparation of a pharmaceutical or diagnostic composition comprising adding and mixing a molecule of the present disclosure, such as hydrolysed molecule of the disclosure of the present invention together with one or more of a pharmaceutically acceptable excipient, diluent or carrier, in particular a pharmaceutical composition (or diagnostic composition) comprising a molecule of the present disclosure and pharmaceutical excipient, diluent or carrier.

The composition will usually be supplied as part of a sterile, pharmaceutical composition that will normally include a pharmaceutically acceptable carrier. A pharmaceutical composition may additionally comprise a pharmaceutically-acceptable adjuvant in the context of vaccine formulation.

The antibody of the disclosure may be the sole active ingredient in the pharmaceutical or diagnostic composition or may be accompanied by other active ingredients.

The pharmaceutical compositions suitably comprise a therapeutically effective amount of a molecule according to the disclosure. The term "therapeutically effective amount" as used herein refers to an amount of a therapeutic agent needed to treat, ameliorate or prevent a targeted disease or condition, or to exhibit a detectable therapeutic or preventative effect. The therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgment of the clinician. Generally, a therapeutically effective amount will be from 0.01 mg/kg to 50 mg/kg, for example 0.1 mg/kg to 20 mg/kg. Pharmaceutical compositions may be conveniently presented in unit dose forms containing a predetermined amount of an active agent of the invention per dose. The actual dose at which an molecule of the present disclosure is administered depends on the nature of the condition to be treated, for example the extent of the disease/inflammation present and on whether the molecule is being used prophylactically or to treat an existing condition.

Compositions may be administered individually to a patient or may be administered in combination (*e.g.* simultaneously, sequentially or separately) with other agents, drugs or hormones.

The pharmaceutically acceptable carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the molecule of the disclosure may be in

dry form, for reconstitution before use with an appropriate sterile liquid.

Suitably in formulations according to the present disclosure, the pH of the final formulation is not similar to the value of the isoelectric point of the antibody, for example if the pH of the formulation is 7 then a pI of from 8-9 or above may be appropriate. Whilst not wishing to be bound by theory it is thought that this may ultimately provide a final formulation with improved stability, for example the antibody remains in solution.

The pharmaceutical compositions of this invention may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

It will be appreciated that the composition comprises a antibody and as such, it may be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Remington's Pharmaceutical Sciences (Mack Publishing Company, N.J. 1991).

In one embodiment the formulation is provided as a formulation for topical administrations including inhalation.

Suitable inhalable preparations include inhalable powders, metering aerosols containing propellant gases or inhalable solutions free from propellant gases. Inhalable powders according to the disclosure containing the active substance may consist solely of the abovementioned active substances or of a mixture of the abovementioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 μm, in particular from 1 to 5 μm. The particle size of the active ingredient (such as the antibody) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient.

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

The molecules of the present disclosure can be delivered dispersed in a solvent, e.g., in the form of a solution or a suspension. It can be suspended in an appropriate physiological solution, e.g., saline or other pharmacologically acceptable solvent or a buffered solution.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent/solution used for the for the formulation, aseptic suspension of the molecule in the sterile buffered solvent solution, and dispensing of the formulation into sterile receptacles by methods familiar to those of ordinary skill in the art.

## EXAMPLES

The examples below employ the monoclonal antibody referred to herein as T289C.

| | |
|---|---|
| $AC_4GlcNAz$ | Tetraacylated N-azidoacetylglucosamine |
| ADC | antibody drug conjugate. |
| BCN | bicyclo[6.1.0]nonyne |
| BME | β-mercapto ethanol |
| DTT | dithiothreitol |
| MMAE | *tris*(2-carboxyethyl)phosphine |
| PAB | para-aminobenzyl alcohol |

### Example 1 Synthesis of Maleimidopropionyl-PEG$_7$-Biotin

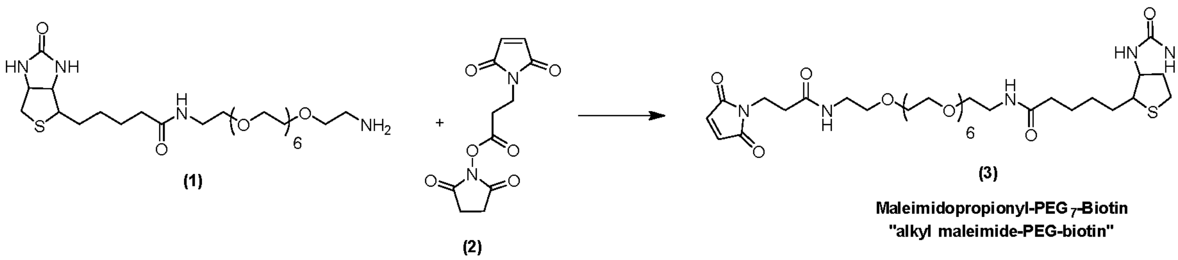

(1) (2) (3)

Maleimidopropionyl-PEG$_7$-Biotin
"alkyl maleimide-PEG-biotin"

To a stirred solution of Biotin-PEG7 amine (1) (100 mg, 0.168 mmol) in anhydrous dichloromethane (7 mL) at room temperature under N2 was added maleimidopropionic NHS ester (2) (49.2 mg, 0.186 mmol, 1.1 eq). After stirring for 1 h, TLC analysis indicated the completion of reaction, whereupon 1 N HCl (2 mL) was added. The organic solution was separated, washed with brine and dried over Na2SO4. The crude product obtained after evaporation under vacuum was purified by flash column chromatography on silica gel, eluting with step gradients of MeOH in dichloromethane (v/v = 1:30, 1:20 and 1:10), to afford the target compound, maleimidoproprionyl-PEG7-biotin (3) (65 mg, 0.087 mmol, 52% yield) as a wax solid. 1H NMR (CDCl3) d 7.01 (br s, 1H), 6.96 (br s, 1H), 6.70 (s, 2H), 5.86 (s, 1H), 5.06 (s, 1H), 4.51 (m, 1H), 4.33 (m, 1H), 3.84 (t, J= 7.2 Hz, 2H), 3.62-3.66 (m, 24H), 3.53 (m, 4H), 3.40 (m, 4H), 3.15 (m, 1H), 2.88-2.96 (m, 1H), 2.73 (d, J= 12.6 Hz, 1H), 2.52 (t, J= 7.2 Hz, 2H), 2.24 (t, J= 7.0 Hz, 2H), 1.66 (m, 4H), 1.45 (m, 2H).

### Example 2 Synthesis of Maleimidophenylacetyl-PEG$_7$-Biotin

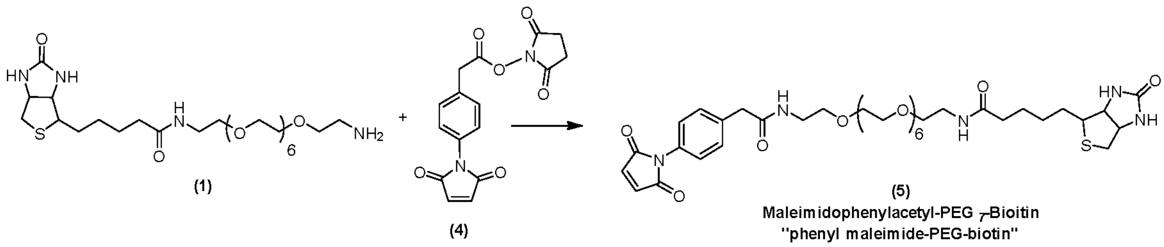

(1) (4) (5)

Maleimidophenylacetyl-PEG $_7$-Bioitin
"phenyl maleimide-PEG-biotin"

A mixture of Biotin-PEG$_7$-NH$_2$ (1) (0.25 g, 0.42 mmol) and maleimidophenylacetic NHS ester *(4)* (0.2 g, 0.609 mmol) in acetonitrile (10 mL) was stirred overnight at ambient temperature under $N_2$. The solvent was removed under vacuum and the residue obtained was purified by flash column chromatography on silica gel, eluting with step gradients of dichloromethane to MeOH in dichloromethane at a ratio of v/v 1:30, 1:20, 1:10:1 and 1:5) to afford the target product maleimidophenylacetyl-PEG$_7$-biotin *(5)* as an oil (70 mg, .087 mmol, 21% yield) along with 190 mg mixture of the desired

product and the hydrolyzed form at a ratio of 3:1 as shown by $^1$H NMR as well as 15 mg of 1:2 mixture. $^1$H NMR ($CDCl_3$) d 7.41, 7.30 (AB Type, $J_{AB}$=8.3 Hz, 4H) [7.64, 7.24 (AB Type, $J_{AB}$=9.2 Hz, 4H) minor component], 6.85 (s, 2H) [6.23 (d, *J*=*7.5* Hz, 1H), 6.47 (d, *J*=7.5 Hz, 1H), minor component], 6.63 (br s, 1H), 6.55 (br s, 1H), 5.83 (s, 1H), 5.18 (s, 1H), 4.49 (m, 1H), 4.31 (m, 1H), 3.63-3.40 (m, 34H), 3.15 (m, 1H), 2.90 (d-AB Type, $J_{AB}$=12.9, *J*=4.7 Hz, 1H), 2.72 (AB Type, $J_{AB}$=12.9Hz, 1H), 2.22 (t, *J*= 7.1 Hz, 2H), 1.68 (m, 4H), 1.46 (m, 2H). MS (ESI) m/z 830 (M+Na), 825 (M+$NH_4$, base peak), 808 (M+1).

**Example 3 Synthesis of 2-Fluoro-5-maleimidophenyl-PEG$_7$-biotin**

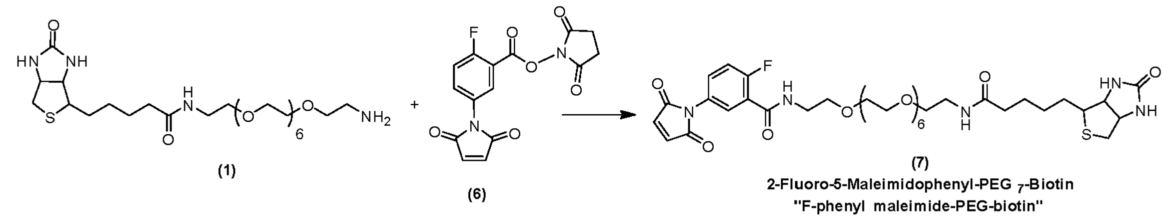

(1)

(6)

(7)
2-Fluoro-5-Maleimidophenyl-PEG $_7$-Biotin
"F-phenyl maleimide-PEG-biotin"

A mixture of Biotin-PEG7-NH2 (1) (0.1 g, 0.168 mmol, 1 eq.) and 2-fluoro-5-maleimidophenolic NHS ester (6) (0.084 g, 0.252 mmol, 1.5 eq) in dichloromethane (1 mL) was stirred overnight at ambient temperature under N2. The solvent was removed under vacuum and the residue obtained purified by size exclusion chromatography (SEC) on an LH20 column, eluting with MeOH/dichloromethane (v/v 1:1), to afford the target product 2-fluoro-5-maleimidophenyl-PEG7-biotin (7) as an oil (28 mg, 0.034 mmol, 20.5% yield) which contained approximately 10% hydrolyzed form as shown by HPLC. An additional 90 mg mixture of the desired product and the hydrolyzed form at a ratio of 3:2 was also obtained. 1H NMR (CDCl3) d 8.05 (dd, J=6.7, 2.9 Hz, 1H), 7.47 (dd, J=4.5, 2.7 Hz, 1H), 7.23 (m, 1H) [8.20 (m, 1H), 7.46 (m, 1H), 7.19 (m, 1H), minor component], 6.88 (s, 2H) [6.45, 6.24 (AB Type, JAB=12.9 Hz, 2H), minor component], 6.63 (br s, 1H), 6.05 (s, 1H), 4.49 (m, 1H), 4.31 (m, 1H), 3.66-3.54 (m, 32H), 3.43 (m, 2H), 3.14 (m, 1H), 2.90 (d-AB Type, JAB=12.7, J=5.0 Hz, 1H), 2.73 (AB Type, JAB=12.7 Hz, 1H), 2.22 (m, 2H), 1.73 (m, 4H), 1.44 (m, 2H). MS (ESI) m/z 835 (M+Na), 829 (M+NH4), 813 (M+1, base peak).

**Example 4 Generic Procedure for Conjugating x-Maleimide-PEG-Biotins to mAbs**

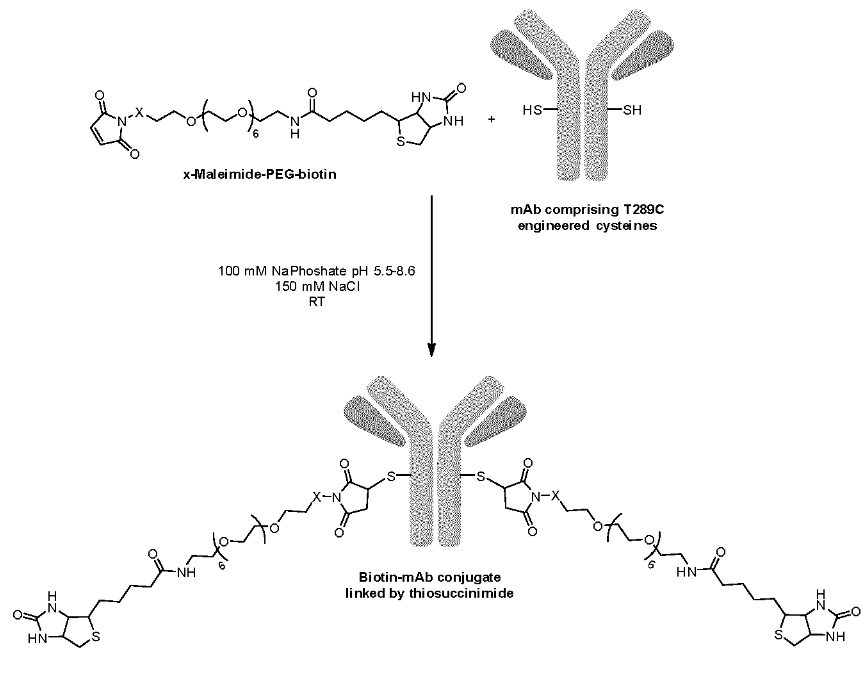


x-Maleimide-PEG-biotin

mAb comprising T289C engineered cysteines

Biotin-mAb conjugate linked by thiosuccinimide

wherein x represents X as defined above for compounds of formula (I) and optionally further comprises Y as defined for

compounds of formula (I).

x-Maleimide-PEG-biotins were conjugated to mAb (comprising a T289C mutation of the Fc) in several steps. First, mAbs were mildly reduced to generate free sulfhydryls by combining 5 mL of 1.6 mg/mL mAb solution in 10 mM PBS, pH 7.2 (8 mg mAb, 53.3 nM, 1 eq) was combined with 43 μL of 50 mM TCEP solution in water (2.15 μmol, 40 eq) followed by gentle mixing at 37 °C for 1 hr. Reduced mAb was transferred to a slide-a-lizer dialysis cassette (10K MWCO) and dialyzed against PBS, 1mM EDTA, pH 7.2-7.8, 4°C for 24 hr with several buffer changes. Reduced mAb was oxidized to reform internal disulfides by addition of dehydroascorbic acid (21 μL of 50 mM stock in DMSO, 1.1 μmol, 20 eq) followed by gentle mixing for 4 hr at room temperature. Oxidized mAb solution was adjusted to the desired pH by addition of 1M sodium phosphate (monobasic for pH 5.5, dibasic for pH 8.6, or a stock adjusted to pH 7.4) to a final phosphate concentration of 100 mM and mAb concentration of 1.3 mg/mL. Next, 1.15 mL of mAb solution (1.5 mg, 10 nmol, 1 eq) was aliquoted into a vial followed by addition of x-maleimide-PEG-biotin stock solution (2 μL of a 10 mM stock colution in DMAc, 20 nmol, 2 eq). The reaction mixture was briefly vortexed and further incubated for the desired amount of time followed by addition of N-acetly cysteine (10 μL of a 100 mM solution in water, 1 μmol, 100 eq) and further incubation for 15 minutes to quench unreacted maleimide. All conjugation reactions were performed at room temperature (22 °C) under ambient atmosphere. Note that 2 eq x-maleimide-PEG-biotin relative to mAb = 1 eq x-maleimide-PEG-biotin relative to free cysteine contained in the mAb. This general procedure was modified as needed to achieve desired reaction stoichiometry (i.e. different maleimide:mAb feeds).

ADC samples were analyzed using an Agilent 6520B Q-TOF mass spectrometer equipped with a RP-HPLC column (Agilent Poroshell 300SB-C3; 5um, 2.1 mm × 75 mm; Part# 660750-909). High-performance liquid chromatography (HPLC) parameters were as follows: flow rate, 0.4 ml/min; mobile phase A was 0.1% (v/v) formic acid in HPLC-grade $H_2O$, and mobile phase B was 0.1% (v/v) formic acid in acetonitrile. The column was equilibrated in 90%A/10%B, which was also used to desalt the ADC samples, followed by elution in 40%A/60%B. Mass spec data were collected for 100-3000 *m*/*z*, positive polarity, a gas temperature of 350°C, a nebulizer pressure of 48 1b/in$^2$, and a capillary voltage of 5,000 V. Data were analyzed using vendor-supplied (Agilent v.B.04.00) MassHunter Qualitative Analysis software and peak intensities from deconvoluted spectra were used to derive the relative proportion of reactants and products; and reaction kinetics.

**Example 5 Calulation of Conjugation Efficiency and Percentage Hydrolysis**

**Calculation of Conjugation Efficiency**

Conjugation efficiency and drug: antibody ratio (DAR) were calculated from the peak intensities of deconvoluted mass spectra using the equations below:

$$Conjugation\ efficiency = \frac{a+b+c}{a+b+c+d+e} \text{ x } 100$$

**Equation 1**

$$DAR = \frac{a+b+c}{a+b+c+d+e} \text{ x } 2$$

**Equation 2**

*a* = G0+1 peak intensity
*b* = G0+1+Na/$H_2O$ peak intensity
*c* = G0+1+Na/$H_2O$+Na peak intensity
*d* = G0 peak intensity
*e* = G0+Na/$H_2O$ peak intensity
DAR = drug: antibody ratio. For this study, any molecule conjugated to a mAb is considered as "drug"

**Method for Calculation of Percent Hydrolysis for T289C mAb-Conjugated**

**Thiosuccinimides**

Percent hydrolysis values were calculated from the peak intensities of deconvoluted mass spectra using the equation below:

$$\% \, hydrolysis = \frac{b+c-[a*(l+m)]}{a+[b-(a*l)]+[c-(a*m)]} \text{ x } 100$$

**Equation 3**

*a*= peak intensity of heavy chain conjugate= (HC+1 )
*b*= peak intensity of heavy chain conjugate +$H_2O$/Na= (HC+1+$H_2O$/Na)
Note: Heavy chain conjugate + 23 amu and +18 amu were both included in this value. At intermediate hydrolysis values these peaks could not be resolved and a single peak appeared in between values expected for (HC+1+$H_2O$) and (HC+1+Na).
*c*= peak intensity of heavy chain conjugate + $H_2O$+ Na = (HC+1+$H_2O$+Na).
*l*= b/a at T=0
*m*= c/a at T=0

**Example 6** **Half-life Calculations**

Half-lives were calculated from the derived rate constants using Equation 4 for psuedo-first order reactions (hydrolysis and deconjugation) and Equation 5 for second-order reactions (conjugation):

$$T_{1/2} = \frac{0.693}{k_{obs}}$$

**Equation 4**

$$T_{1/2} = \frac{1}{k[SH_0]}$$

**Equation** 5
$[SH]_0$ = Initial thiol concentration

**Example 7** **Analysis of x-Maleimide-PEG-biotin Conjugation at 1 eq Maleimide:Thiol**

x-Maleimide-PEG-biotin mAb conjugates were prepared for LC/MS analysis by diluting to 0.2 mg/mL with PBS pH 7.2 followed by combining 50 μL of mAb solution with 5 μL of TCEP (0.5 M in water). This mixture was incubated for 5 min at room temperature to reduce disulfide bonds prior to injection (15 μL) into the LC/MS. Representative data is shown in Figure 1A and 1B.

**Table 1**

| | **Unreacted mAb** | | **Alkyl maleimide-PEG-biotin conjugate** | |
|---|---|---|---|---|
| | **Peak** | **Intensity** | **Peak** | **Intensity** |
| **Light Chain** | 23616.0 | 2241350 | 23616.7 | 1859865 |
| **Heavy Chain (G0)** | 50756.7 | 2520546 | 50758.6 | 94051 |
| **Heavy Chain (G0+Na)** | 50777.3 | 703916 | N/D | ---- |
| **Heavy Chain (G1)** | 50918.7 | 954244 | N/D | ----- |
| **Heavy Chain (G1+Na)** | 50939.3 | 288623 | N/D | ----- |
| **Heavy Chain (G0+1)** | ---- | ----- | 51503.9 | 860388 |
| **Heavy Chain (G0+1+Na/$H_2O$)** | ----- | ----- | 51524.4 | 303709 |

**Example 8** **Analysis of x-Maleimide-PEG-biotin Conjugation at 0.5 eq Maleimide:Thiol**

x-Maleimide-PEG-biotin mAb conjugates were prepared as described above in Example 7, except that 1 eq. maleimide:mAb was used. Note that 1 equivalent x-maleimide-PEG-biotin equals 0.5 equivalent x-maleimide-PEG-biotin:thiol.

**1) Alkyl maleimide-PEG-biotin (comparator) see Figure 2A**

**Table 2**

| | Unreacted mAb | |
|---|---|---|
| | Peak | Intensity |
| **Heavy Chain (G0)** | 50757.9 | 811198 |
| **Heavy Chain (G0+Na)** | 50777.7 | 234247 |
| **Heavy Chain (G0+1)** | 51503.4 | 781791 |
| **Heavy Chain (G0+1+Na/$H_2O$)** | 51523.9 | 254456 |
| **Heavy Chain (G0+1+Na/$H_2O$+Na)** | 51545.6 | 115361 |
| **Calculated conjugation and DAR** | 52%, 1.05 | |

**2) Phenyl maleimide-PEG-biotin see Figure 2B**

**Table 3**

| | Unreacted mAb | |
|---|---|---|
| | Peak | Intensity |
| **Heavy Chain (G0)** | 50756.9 | 790494 |
| **Heavy Chain (G0+Na)** | 50776.7 | 237800 |
| **Heavy Chain (G0+1)** | 51564.8 | 559793 |
| **Heavy Chain (G0+1+Na/$H_2O$)** | 51583.7 | 266980 |
| **Heavy Chain (G0+1+Na/$H_2O$+Na)** | 51605.8 | 103090 |
| **Calculated conjugation and DAR** | 48%, 0.95 | |

**3) Fluorophenyl maleimide-PEG-biotin see Figure 2C**

| | Unreacted mAb | |
|---|---|---|
| | Peak | Intensity |
| **Heavy Chain (G0)** | **50757.7** | **913288** |
| **Heavy Chain (G0+Na)** | 50777.7 | 265152 |
| **Heavy Chain (G0+1)** | 51569.3 | 434825 |
| **Heavy Chain (G0+1+Na/$H_2O$)** | 51587.7 | 420700 |
| **Heavy Chain (G0+1+Na/$H_2O$+Na)** | 51609.5 | 145829 |
| **Calculated conjugation and DAR** | 51%, 1.02 | |

This data shows that the analytical method is reliable, i.e., 50% reaction feed gave us ~50% conjugation.

**Example 9 Conjugation Efficiency of x-Maleimide-PEG-biotins to T289C mAb at 22°C**

x-Maleimide-PEG-biotin mAb conjugates were prepared as described above, with 2 eq. x-maleimide-PEG-biotin:mAb used. Note that 2 eq of x-maleimide-PEG-biotin:mAb equals 1 eq x-maleimide-PEG-biotin:thiol. Reaction products were analyzed by reduced glycosylated mass spectrometry and conjugation efficiency was calculated using equation 1. The results are shown in Figure 3 and Figure 4.

**Example 10 Conjugation Kinetics of x-Maleimide-PEG-Biotins with T289C mAb, pH 5.5, 22°C**

Conjugation data were analyzed in units of molar concentration to determine kinetic constants. Second order rate constants were determined from the slopes of curves generated from plotting 1/[SH] versus time and linear regression analysis. The results are shown in Figure 5.

**Example 11 Conjugation Kinetics of x-Maleimide-PEG-Biotins with T289C mAb, pH 7.4, 22°C**

Conjugation data were analyzed in units of molar concentration to determine kinetic constants. Second order rate constants were determined from the slopes of curves generated from plotting 1/[SH] versus time and linear regression analysis. The results are shown in Figure 6.

**Example 12 Conjugation Kinetics of x-Maleimide-PEG-Biotins with T289C mAb, pH 8.6, 22°C**

Conjugation data were analyzed in units of molar concentration to determine kinetic constants. Second order rate constants were determined from the slopes of curves generated from plotting 1/[SH] versus time and linear regression analysis. The results are shown in Figure 7.

**Summary of x-Maleimide-PEG-biotin Kinetics for Conjugation to T289C mAb**

**Table 4**

| | pH 5.5 | | pH 7.4 | | pH 8.6 | |
|---|---|---|---|---|---|---|
| | **Second order rate constant ($k_2$, $M^{-1}s^{-1}$)** | **Conjugation $T_{1/2}$ (min)** | **Second order rate constant ($k_2$, $M^{-1}s^{-1}$)** | **Conjugation $T_{1/2}$ (min)** | **Second order rate constant ($k_2$, $M^{-1}s^{-1}$)** | **Conjugation $T_{1/2}$ (min)** |
| **Alkyl maleimide-PEG-biotin** | 13 | 74.2 | $0.5\times10^3$ | 1.73 | $-2.8\times10^4$ | 0.33 |
| **Phenyl maleimide-PEG-biotin** | 23.8 | 40.3 | $1.3\times10^3$ | 0.72 | $\sim3.4\times10^4$ | 0.28 |
| **F-phenyl maleimide-PEG-biotin** | 55.4 | 17.4 | $4.5\times10^3$ | 0.21 | $\sim2.9\times10^4$ | 0.25 |

Literature Comparisons:

**1) Reaction of N-ethyl maleimide with cysteine at pH 7.0, $k_2 = 1.62\times10^3 M^{-1}s^{-1}$**

Li, J.; Xu, Q.; Cortes, D.; et al. Reaction of cysteines substituted in the amphipathic N-terminal tail of a bacterial potassium channel with hydrophilic and hydrophobic maleimides. PNAS 2002, 99(18), 11605-11610.

**2) Reaction of N-ethyl maleimide with β-mercaptoethanol at pH 7.0, $k_2 = 0.71\times10^3 M^{-1}s^{-1}$**

Mosser, G. N-Substituted maleimide inactivation of the response of taste cell stimulation. J. Neurobiol. 1976, 7(5), 457-468.

**Example 13 Reaction Kinetics of x-Maleimide-PEG-Biotins with T289C mAb Following PreIncubation: Indirect Measurement of Maleimide Hydrolysis**

x-Maleimide-PEG-biotins were pre-incubated in buffer solutions of different pH to assess their stability and thiol conjugation efficiency over time. This assay indirectly monitors maleimide hydrolysis because hydrolyzed maleimides are not reactive towards thiols. Antibody bearing the T289C FC mutation was reduced and oxidized as described above and adjusted to 1.5 mg/mL in PBS containing 50 mM sodium phosphate pH 7.4. Maleimide solutions were prepared in buffer (100 mM sodium phosphate pH 5.5, 7.4, 8.6) by combining 20 μL x-maleimide-PEG-biotin solution (10 mM solution in DMAc) with 180 μL buffer at the desired pH (final x-maleimide-PEG-biotin concentration = 1 mM) and incubated at 22 °C for determined time intervals prior to reaction with mAb. For conjugation reactions 267 μL mAb (1.5 mg/mL , 2.7 nmol, 1 eq) was combined with 5.3 μL buffer-incubated x-maleimide-PEG-biotin solution (5.4 nmol, 2 eq). Addition of maleimide solution did not affect the pH of the mAb solution. The conjugation reaction proceeded at 22 °C for 1.5 hr at pH 7.4. After reaction samples were reduced with DTT at room temperature for 5 min and analyzed by mass spectrometry. Percent conjugation was calculated using equation 1. The results are shown in Figure 8.

This data shows that maleimide reactivity is lost over time due to hydrolysis. The rate of maleimide hydrolysis is increased in the case of N-aryl maleimides. Overall, maleimide hydrolysis is slower than thiol-maleimide conjugation, which explains why efficient conjugation for N-aryl maleimides was observed at pH 8.6. Half lives can be compared using data that follows.

**Example 14 Maleimide Hydrolysis Kinetics pH 5.5, 22 °C**

Maleimide hydrolysis data were also analyzed in units of molar concentration to determine kinetic constants. Pseudo 1st order rate constants were determined from the slopes of curves generated from plotting ln[maleimide] versus time and linear regression analysis. The results are shown in Figure 9.

**Example 15 Maleimide Hydrolysis Kinetics pH 7.4, 22 oC**

Maleimide hydrolysis data were also analyzed in units of molar concentration to determine kinetic constants. Pseudo 1st order rate constants were determined from the slopes of curves generated from plotting ln[maleimide] versus time and linear regression analysis. The results are shown in Figure 10.

**Example 16 Maleimide Hydrolysis Kinetics pH 8.6, 22 oC**

Maleimide hydrolysis data were also analyzed in units of molar concentration to determine kinetic constants. Pseudo 1st order rate constants were determined from the slopes of curves generated from plotting ln[maleimide] versus time and linear regression analysis. The results are shown in Figure 11.

**Summary of x-Maleimide Hydrolysis Kinetics**

**Table 5** Kinetic constants for maleimide hydrolysis at 22 oC

| | **pH 5.5[a]** | | **pH 7.4** | | **pH 8.6** | |
|---|---|---|---|---|---|---|
| | **Psuedo first order rate constant ($k_{obs}$, $s^{-1}$)** | **Hydrolysis $T_{1/2}$ (hr)** | **Psuedo first order rate constant ($k_{obs}$, $s^{-1}$)** | **Hydrolysis $T_{1/2}$ (hr)** | **Psuedo first order rate constant ($k_{obs}$, $s^{-1}$)** | **Hydrolysis $T_{1/2}$ (hr)** |
| **Alkyl maleimide-PEG-biotin** | ND | ND | $2.3\times10^{-5}$ | 7.7 | $2\times10^{-4}$ | 1 |

(continued)

| | pH 5.5[a] | | pH 7.4 | | pH 8.6 | |
|---|---|---|---|---|---|---|
| | **Psuedo first order rate constant ($k_{obs}$, $s^{-1}$)** | **Hydrolysis $T_{1/2}$ (hr)** | **Psuedo first order rate constant ($k_{obs}$, $s^{-1}$)** | **Hydrolysis $T_{1/2}$ (hr)** | **Psuedo first order rate constant ($k_{obs}$, $s^{-1}$)** | **Hydrolysis $T_{1/2}$ (hr)** |
| **Phenyl maleimide-PEG-biotin** | ND | ND | $8.5\times10^{-5}$ | 2.3 | $7.6\times10^{-4}$ | 0.3 |
| **F-phenyl maleimide-PEG-biotin** | ND | ND | $1.7\times10^{-4}$ | 1.1 | $1.9\times10^{-3}$ | 0.1 |
| *a) No significant hydrolysis detected after 60 min incubation* | | | | | | |

Literature Comparisons:

1) Hydrolysis of N-phenyl maleimide at pH 7.6, 25 °C $k_{obs} = 7\times10^{-5}\ s^{-1}$ Oleksandr, K.; Leriche, G.; et al. Selective Irreversible chemical tagging of cysteine with 3-arylpropiolonitriles. Bioconjug. Chem. 2014, 25, 202-206.

2) Hydrolysis of maleimide (unsubstituted) at pH 8.38, 30 °C, $k_{obs} = 1.51\times10^{-4} s^{-1}$ Khan, M.N. Kinetics and mechanism of the alkaline hydrolysis of maleimide . J. Pharm. Sci. 1984, 73(12), 1767-1171.

This data shows that maleimide hydrolysis is much slower than thiol-maleimide conjugation, **thus, N-aryl maleimides are useful reagents for conjugation.** Even in the most extreme case of F-phenyl maleimides at pH 8.6, the maleimide hydrolysis half-life is 6 minutes while thiol conjugation is complete in 15 seconds.

**Example 17 Thiosuccinimide Hydrolysis Kinetics for x-Maleimide-PEG-Biotin T289C mAb Conjugates**

x-Maleimide-PEG-biotin mAb conjugates were incubated in buffer solutions and monitored by LC/MS over time to observe thiosuccinimide hydrolysis. First, mAb containing the T289C Fc mutation (0.75 mg, 5 nmol, 1 eq) was reacted with x-maleimide-PEG-biotin (50 nmol, 5 mL of a 10 mM stock in DMAC, 10 eq) in 577 mL PBS, pH 7.2. The conjugation reaction was allowed to proceed for 5 minutes and then quenched with N-acetyl cysteine (500 nmol, 4 mL of 100 mM stock in water, 100 eq) and further incubated for 5 minutes. The reaction mixture was then combined with PBS containing 0.5 mM EDTA and 1M sodium phosphate at the desired pH to achieve final concentrations of 0.65 mg/mL mAb, 100 mM phosphate, 0.5 M EDTA and 150 mM NaCl. After sample preparation, an aliquot was removed and diluted 1:3 with 75 mM phosphate buffer pH 5.5 to obtain an initial time point sample. Samples were then further incubated at the desired conditions with aliquots removed at determined time points. All samples were immediately diluted 1:3 with 75 mM phosphate buffer pH 5.5 to stop the hydrolysis reaction. Samples were then sterile filtered, reduced with TCEP, and analyzed by LC/MS. Thiosuccinimide hydrolysis was confirmed by addition of 18 amu to the mAb conjugate peak in the mass spectrum. Semi-quantitative analysis of hydrolysis was performed using peak intensities in deconvoluted mass spectra and equation 3, which includes background subtraction using the T=0 measurement. Data was then converted into loss of thiosuccinimide (M) over time and plotted as ln[thiosuccinimide] vs seconds. The slope of the best fit line yielded the psuedo first-order rate constant for thiosuccinimide hydrolysis. Additional experiments were performed to determine thiosuccinimide hydrolysis at 1 hour, n=3.

**Example 18 Thiosuccinimide Hydrolysis pH 7.4, 22 °C, for PEG-biotin T289C mAb Conjugates**

Thiosuccinimide hydrolysis data were also analyzed in units of molar concentration to determine kinetic constants. Pseudo $1^{st}$ order rate constants were determined from the slopes of curves generated from plotting ln[maleimide] versus time and linear regression analysis. The results are shown in Figure 12.

**Example 19 Thiosuccinimide Hydrolysis pH 7.4, 37 °C, for PEG-biotin T289C mAb Conjugates**

Thiosuccinimide hydrolysis data were also analyzed in units of molar concentration to determine kinetic constants. Pseudo 1st order rate constants were determined from the slopes of curves generated from plotting ln[maleimide] versus time and linear regression analysis. The results are shown in Figure 13.

**Example 20 Thiosuccinimide Hydrolysis pH 8.6, 22 oC, for PEG-biotin T289C mAb Conjugates**

Thiosuccinimide hydrolysis data were also analyzed in units of molar concentration to determine kinetic constants. Pseudo 1st order rate constants were determined from the slopes of curves generated from plotting ln[maleimide] versus time and linear regression analysis. The results are shown in Figure 14.

**Summary of Thiosuccinimide Hydrolysis Kinetics for T289C mAb-PEG-biotin**

**Conjugates**

**Table 6:** Kinetic constants for thiosuccinimide hydrolysis of PEG-biotin conjugates at mAb position T289C

| | **pH 7.4,22 °C** | | **pH 7.4, 37 °C** | | **pH 8.6, 22 °C** | |
|---|---|---|---|---|---|---|
| | **Psuedo first order rate constant ($k_{obs}$, $s^{-1}$)** | **$T_{1/2}$ (hr)** | **Psuedo first order rate constant ($k_{obs}$, $s^{-1}$)** | **$T_{1/z}$ (hr)** | **Psuedo first order rate constant ($k_{obs}$, $s^{-1}$)** | **$T_{1/2}$ (hr)** |
| **Alkyl thio-succinimide** | $2.0\times10^{-6}$ | 96.3 | $1.1\times10^{-5}$ | 17.5 | $1.9\times10^{-5}$ | 10.1 |
| **Phenyl thio-succinimide** | $3.0\times10^{-5}$ | 6.4 | $1.3\times10^{-4}$ | 1.5 | $2.0\times10^{-4}$ | 1.0 |
| **F-phenyl thio-succinimide** | $4.6\times10^{-5}$ | 4.3 | $2.8\times10^{-4}$ | 0.7 | $4.9\times10^{-4}$ | 0.4 |

This data shows that hydrolysis of N-aryl thiosuccinimides occurs on a timescale that can easily be applied to manufacturing, without drastic changes to standard conjugation procedures. For example, performing payload conjugation at pH 7.4, 22 oC for 24 hr would allow for both conjugation and thiosuccinimde hydrolysis in one step.

**Example 21 x-Thiosuccinimide Hydrolysis for PEG-biotin T289C-mAb Conjugates After 1 Hour Incubation, n=3**

The results are shown in Figure 15 plotted as the as the average ± standard deviation, n=3. This data confirms the trend observed with single sample kinetics experiments, with an expanded sample set. Ease of thiosuccinimide hydrolysis depends on the chemistry attached to the ring-head nitrogen as follows: N-alky<<N-phenyl<N-fluorophenyl. The relative error for these experiments was typically less than 5%.

**Example 22 x-Thiosuccinimide Hydrolysis for PEG-Biotin T289C mAb Conjugates in the Presence of Sodium Molybdate**

x-Maleimide-PEG-biotin mAb conjugates were incubated in buffer solutions containing sodium molybdate to investigate if this compound is capable of increasing thiosuccinimide hydrolysis. Samples were monitored by LC/MS over time to observe thiosuccinimide hydrolysis as described above. First, mAb containing the T289C Fc mutation (0.5 mg, 3.3 nmol, 1 eq) was reacted with x-maleimide-PEG-biotin (50 nmol, 5 mL of a 10 mM stock in DMAC, 15 eq) in 0.365 mL PBS, pH 7.2. The conjugation reaction was allowed to proceed for 5 minutes and then quenched with N-acetyl cysteine (500 nmol, 4 mL of 100 mM stock in water, 151 eq) and further incubated for 5 minutes. The reaction mixture was then combined with 1M PBS containing 1M sodium molybdate at the desired pH to achieve a final concentration of 100 mM phosphate and 100 mM molybdate. After addition of molybdate, the mixture was incubated at 22 °C for 1 hr. After 1 hr, samples were diluted 1:5 with 75 mM phosphate buffer pH 6.5 to stop hydrolysis. Samples were then sterile filtered, reduced with TCEP, and analyzed by LC/MS. Thiosuccinimide hydrolysis was confirmed by addition of 18 amu to the mAb conjugate peak in the mass spectrum. Semi-quantitative analysis of hydrolysis was performed using peak

intensities in deconvoluted mass spectra and equation 3. The results are shown in Figure 16.

Notable enhancements in hydrolysis was observed for 2 samples:

A) F-phenyl maleimide-PEG-biotin pH 5.5

B) F-phenyl maleimide-PEG-biotin pH 7.4

**Table 7**

| | **Relative Hydrolysis[a], (+ sodium molybdate/ - sodium molybdate)** | | |
|---|---|---|---|
| | **pH 5.5** | **pH 7.4** | **pH 8.6** |
| **N-alkyl thiosuccinimide** | --- | --- | --- |
| **N-phenyl thiosuccinimide** | --- | 1.6 | 1.2 |
| **N-fluorophenyl thiosuccinimide** | 52 | 2.2 | 1 |
| *a) samples showing less than 10% hydrolysis were not analyzed* | | | |

This data shows that only N-fluorophenyl thiosuccinimides are responsive to molbdate-catalyzed hydrolysis, others are not. Also, catalyst effects are diminished at high pH as expected.

**Example 23 Sensitivity of T289C mAb Conjugates to Thiol Exchange in Buffer Containing Thiols**

Biotin-mAb conjugates were incubated in aqueous buffer containing β-mercaptoethanol (BME) to challenge the thiosuccinimide linkage against deconjugation. x-Maleimide-PEG-biotins were conjugated to mAb containing the T289C mutation as described above with minor modification. Conjugation reactions were performed at 1 equivalent x-PEG-maleimide:thiol for 15 min at 22 °C followed immediately by dilution to 0.2 mg/mL (1.33 μM mAb) with 1X PBS pH 7.2 containing 0.5 mM EDTA. The N-acetyl cysteine quenching step was omitted. For BME-containing samples, BME was added to a final concentration of 1% v/v (143 mM). Samples were further incubated at 37 °C under ambient atmosphere without stirring. Aliquots were removed at various time points, sterile filtered, reduced with DTT and then analyzed by LC/MS. Percent conjugated mAb and thiosuccinimide hydrolysis were determined from peak heights of mass spectra using Equation 1 and Equation 2, respectively. Deconjugated mAb and thiosuccinimide hydrolysis data were plotted as ln[concentration] vs. time (s) to obtain the psuedo-first order rate constants from the slope of the bestfit line. The results are shown in Figure 17.

**Example 24 Stability of PEG-Biotin T289C mAb Conjugates in Buffer Containing BME**

**Table 8:** Kinetic parameters for deconjugation of PEG-biotin

| | **pH 7.2, 37 °C** | | **pH 7.2, 37 °C, +BME** | |
|---|---|---|---|---|
| | **Psuedo first order rate constant ($k_{obs}$, $s^{-1}$)** | **$T_{1/2}$ (hr)** | **Psuedo first order rate constant ($k_{obs}$, $s^{-1}$)** | **$T_{1/2}$ (hr)** |
| **N-Alkyl thiosuccinimide** | $1.4\times10^{-7}$ | 1375 | $1.6\times10^{-6}$ | 120 |

**Table 9:** Summary of initial hydrolysis and deconjugation data

| | **Initial hydrolysi s (%)** | **Max deconjugation -BME (%)** | **Max deconjugation +BME (%)** |
|---|---|---|---|
| **N-alkyl thiosuccinimide** | <1% | 8% | 35 |
| **N-phenyl thiosuccinimide** | <1% | None observed | 18 |
| **N-fluorophenyl thiosuccinimide** | <1% | None observed | 10 |

The result are shown in Figure 18. This data shows that 1) Variable stability was observed between the different thiosuccinimide types in the presence of free thiol. Faster hydrolyzing species showed less deconjugation, 2) N-alkyl thiosuccinimide deconjugated in simple buffer, lacking free thiol.

Also, this data shows that rapid destabilization can be achieved immediately after conjugation without any special treatment in the case of N-fluorophenyl thiosuccinimide.

**Example 25 Thiosuccinimide Hydrolysis at pH 7.2, 37 oC for PEG-Biotin T289C mAb Conjugates Observed in BME Challenge Assay**

The results are shown in Figure 19.

**Table 10:** Kinetics of Thiosuccinimide Hydrolysis for PEG-Biotin T289C mAb Conjugates at pH 7.2, 37 oC

| | pH 7.2, 37 °C | | pH 7.2, 37 °C, +BME | |
|---|---|---|---|---|
| | Psuedo first order rate constant ($k_{obs}$, $s^{-1}$) | $T_{1/2}$ (hr) | Psuedo first order rate constant ($k_{obs}$, $s^{-1}$) | $T_{1/2}$ (hr) |
| N-Alkyl thiosuccinimide-PEG-biotin | $7.1\times10^{-6}$ | 27 | $6.1\times10^{-6}$ | 31 |
| N-phenyl thiosuccinimide-PEG-biotin | $>3.1\times10^{-5}$ | <6 | $>3.1\times10^{-5}$ | <6 |
| N-fluorophenyl thiosuccinimide-PEG-biotin | $>3.1\times10^{-5}$ | <6 | $>3.3\times10^{-5}$ | <6 |

This data shows that N-phenyl and N-fluorophenyl thiosuccinimides completely hydrolyze in the first 24 hrs, which is consistent with other hydrolysis data. N-alkyl thiosuccinimides hydrolyze much slower.

**Example 26 Relationship Between Thiosuccinimide Deconjugation and Hydrolysis Observed in the BME Challenge Assay**

The results as shown in Figure 20.

**Table 11:** Kinetic parameters for thiosuccinimide hydrolysis and deconjugation determined from the BME assay

| | Hydrolysis pH 7.2, 37 °C, +BME | | Deconjugation pH 7.2,37 °C, +BME | |
|---|---|---|---|---|
| | Psuedo first order rate constant ($k_{obs}$, $s^{-1}$) | $T_{1/2}$ (hr) | Psuedo first order rate constant ($k_{obs}$, $s^{-1}$) | $T_{1/2}$ (hr) |
| N-Alkyl thiosuccinimide | $6.5\times10^{-6}$ | 31 | $1.6\times10^{-6}$ | 120 |

This data is plotted as deconjugation on the left axis and hydrolysis on the right axis. Hydrolysis and deconjugation are clearly related. The faster rate of hydrolysis limits the maximum amount of deconjugation.

**Example 27 Stability of mAb-Biotin Conjugates in the Presence of Free Thiol After Mild Hydrolysis**

Biotin-mAb conjugates were incubated in aqueous buffer containing β-mercaptoethanol (BME) to challenge the thiosuccinimide linkage against deconjugation. In this experiment, thiosuccinimide conjugates were subjected to a brief incubation at mildly basic conditions to faciliate thiosuccinimide hydrolysis before the stability challenge. x-Maleimide-PEG-biotins were conjugated to mAb containing the T289C mutation as described above with minor modification. Conjugation reactions were performed at 1 equivalent x-PEG-maleimide:thiol for 15 min at 22 °C followed quenching with N-acetyl cysteine (100 eq rel. to mAb) and further reaction for 5 minutes. Reaction mixtures were adjusted to pH 8.6 by addition of 10% v/v sodium phosphate (1M, dibasic) and then incubated at 37 °C for 1 hr. Reactions were then dialyzed against PBS (pH 7.2) containing 0.5 mM EDTA for 24 hr at 4 °C. After dialysis, mAb concentrations were confirmed by A280 measurement (NanoDrop) and samples were diluted to 0.2 mg/mL (1.33 μM mAb) with 1X PBS pH 7.2 containing 0.5 mM EDTA. For BME-containing samples, BME was added to a final concentration of 1% v/v (143 mM). Samples

were further incubated at 37 °C under ambient atmosphere without stirring. Aliquots were removed at various time points, sterile filtered, reduced with DTT and then analyzed by LC/MS. Percent conjugated mAb and thiosuccinimide hydrolysis was determined from peak heights of mass spectra using Equation 1 and Equation 2, respectively. Deconjugated mAb and thiosuccinimide hydrolysis data were plotted as ln[concentration] vs. time (s) to obtain the psuedo-first order rate constants from the slope of the bestfit line.

**Example 28 Sensitivity of T289C mAb Conjugates to Thiol Exchange in Buffer after Mild Hydrolysis**

**Table 12:** Kinetic parameters for deconjugation of alkyl thiosuccinimide conjugate

| | **pH 7.2, 37 °C** | **pH 7.2, 37 °C, +BME** | |
|---|---|---|---|
| **Species Observed** | **Psuedo first order rate constant $^{-1}$ ($k_{obs}$, $s^{-1}$)** | **Psuedo first order rate constant $^{-1}$ ($k_{obs}$, $s^{-1}$)** | **$T_{1/2}$ (hr)** |
| **N-Alkyl thiosuccinimide** | ND | $1.4x10^{-6}$ | 138 |

**Table 13:** Summary of initial hydrolysis and deconjugation data for PEG-biotin T289C mAb conjugates

| | **Initial hydrolysis (%)** | **Max deconjugation -BME (%)** | **Max deconjugation +BME (%)** |
|---|---|---|---|
| **N-alkyl thiosuccinimide** | 18 | 4 | 27 |
| **N-phenyl thiosuccinimide** | 91 | None observed | None observed |
| **N-fluorophenyl thiosuccinimide** | 96 | None observed | None observed |

This data shows that 1) mild hydrolysis completely stabilized N-aryl thiosuccinimides but not N-alkyl thiosuccinimide. Also, the pseudo first-order rate constant for thiol-mediated deconjugation of N-alkyl thiosuccinides from this experiment closely matches the value from the experiment without pre-hydrolysis (data was reproducible).

**Example 29 Synthesis of N-alkyl maleimido-Val-Cit-PAB-MMAE**

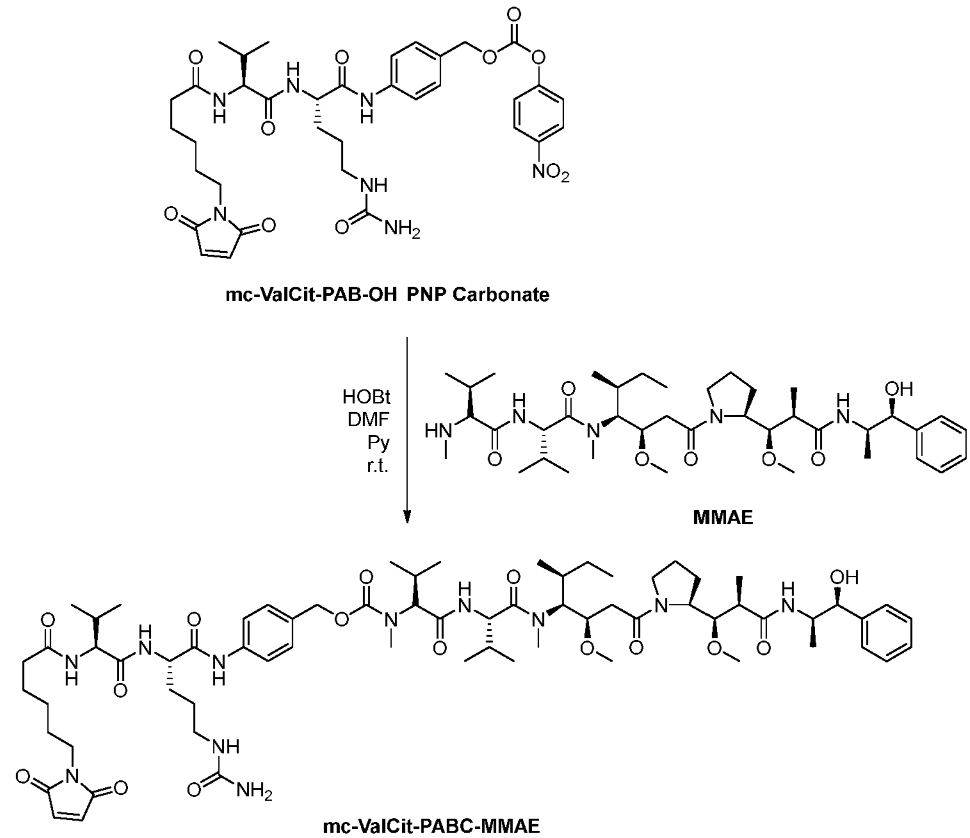


mc-ValCit-PAB-OH PNP Carbonate

MMAE

mc-ValCit-PABC-MMAE
"N-alkyl maleimide Val-Cit-PAB-MMAE"

Maleimidocaproyl-valine-citrulline-*p*-aminobenzylcarbonyl-mono- methyl-Auristatin-E (mc-ValCit-PABC-MMAE) was prepared according to the literature method with modifications.[1] Thus, a mixture of mc-Val-Cit-*p*-aminobenzyl alcohol *p*-nitrophenylcarbonate **(8)** freshly prepared according to literature method[2] (265 mg, 0.36 mmol, 1.5 eq.), MMAE **(9)** (169 mg, 0.24 mmol, 1 eq.) and N-hydroxybenzotriazole (HOBt) (6.48 mg, 0.048 mmol, 0.2 eq.) were stirred in DMF (5 mL) at r.t. for 2 min., followed by addition of pyridine (38 mg, 0.48 mmol, 2 eq.). After stirring for 24 h, volatile organics were removed under vacuum. The residue obtained was triturated with ethyl acetate and methanol (1L) to afford mc-Val-Cit-PAB-MMAE **(10)** as a white powder (220 mg, 0.17 mmol, 71% yield) that was usually >95% pure by RP-HPLC analysis. If necessary, further purification can be carried out by C18 reversed-phase preparative HPLC or size exclusive column chromatography. Electrospray (ES)-MS m/z 1339 (M+Na, base peak), 1317 (M + 1).

**References:**

1) Doronina SO, Toki BE, Torgov MY, Mendelsohn BA, Cerveny CG, Chace DF, DeBlanc RL, Gearing RP, Bovee TD, Siegall CB, Francisco JA, Wahl AF, Meyer DL, Senter PD. Development of potent monoclonal antibody auristatin conjugates for cancer therapy. Nat Biotechnol. 2003;21:778-784. 2) Dubowchik GM, Firestone RA, Padilla L, Willner D, Hofstead SJ, Mosure K, Knipe JO, Lasch, SJ, Trail PA. Cathepsin B-labile dipeptide linkers for lysosomal release of doxorubicin from internalizing immunoconjugates: model studies of enzymatic drug release and antigen-specific in vitro anticancer activity. Bioconjug Chem. 2002;13:855-869.

**Example 30 Synthesis of N-phenyl acetyl maleimido-Val-Cit-PAB-MMAE**

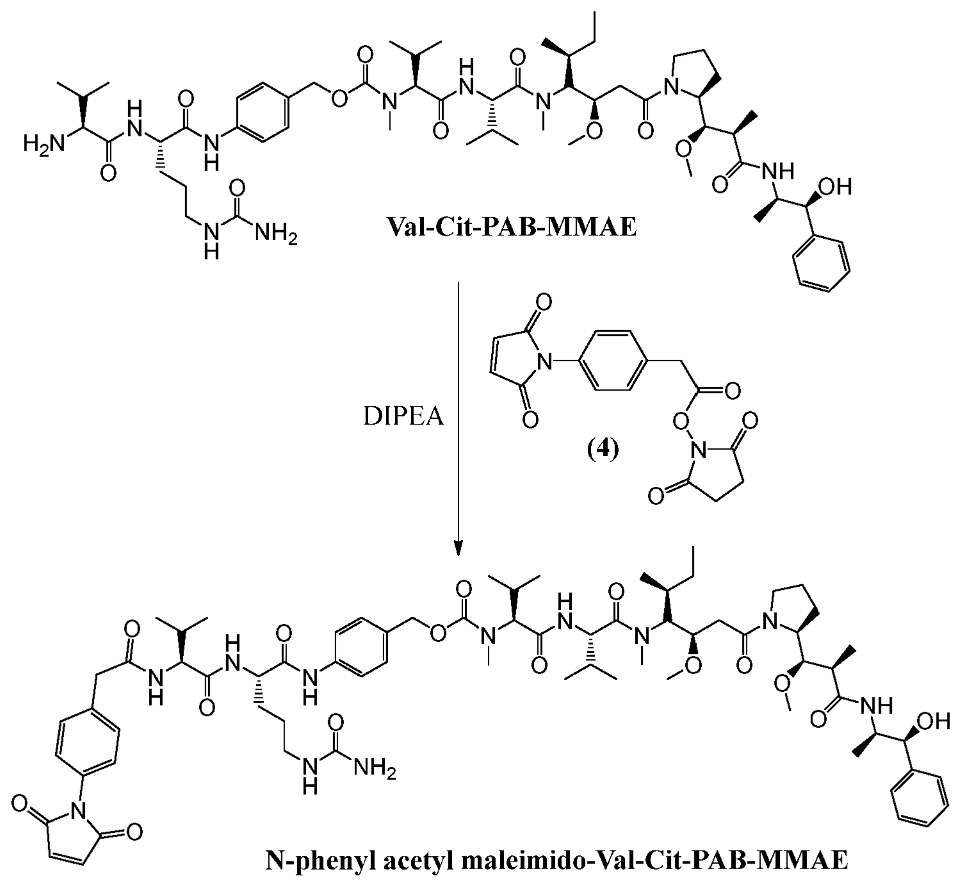


**N-phenyl acetyl maleimido-Val-Cit-PAB-MMAE**

To the stirred solution of Val-Cit-PAB-MMAE (100 mg, 0.089 mmol) and maleimidophenylacetic NHS ester **(4)** (43.8 mg, 0.133 mmol) in DMF (1 mL) at ambient temperature under $N_2$ was added DIPEA (57.5 mg, 0.445 mmol). After stirring overnight, MS analysis indicated the product formation with small amount of starting material present. The mixture was initiated separated by size exclusion chromatography (SEC) on an LH-20 column, eluting with MeOH/dichloromethane (v/v 1:1). Fractions were monitored by MS and those with desired product combined. After evaporation to dryness under vacuum, 25 mg (0.0187 mmol, 21% yield) oily material was obtained. MS exhibited a cluster of ions at m/z 1391 (M+Cl+Na), 1369 (M+Cl, base peak), 1360 (M+Na) and 1337 (M+1) while HPLC showed two peaks at a ratio of approximately 2: 1. Further purification by semi-preparative HPLC afforded 2 main fractions of 5 mg and 6 mg, respectively, after evaporation. However, these two samples showed essentially the same HPLC and MS.

**Example 31 Preparation of N-Phenyl Maleimide Val-Cit-PAB-MMAE T289C mAb Conjugates**

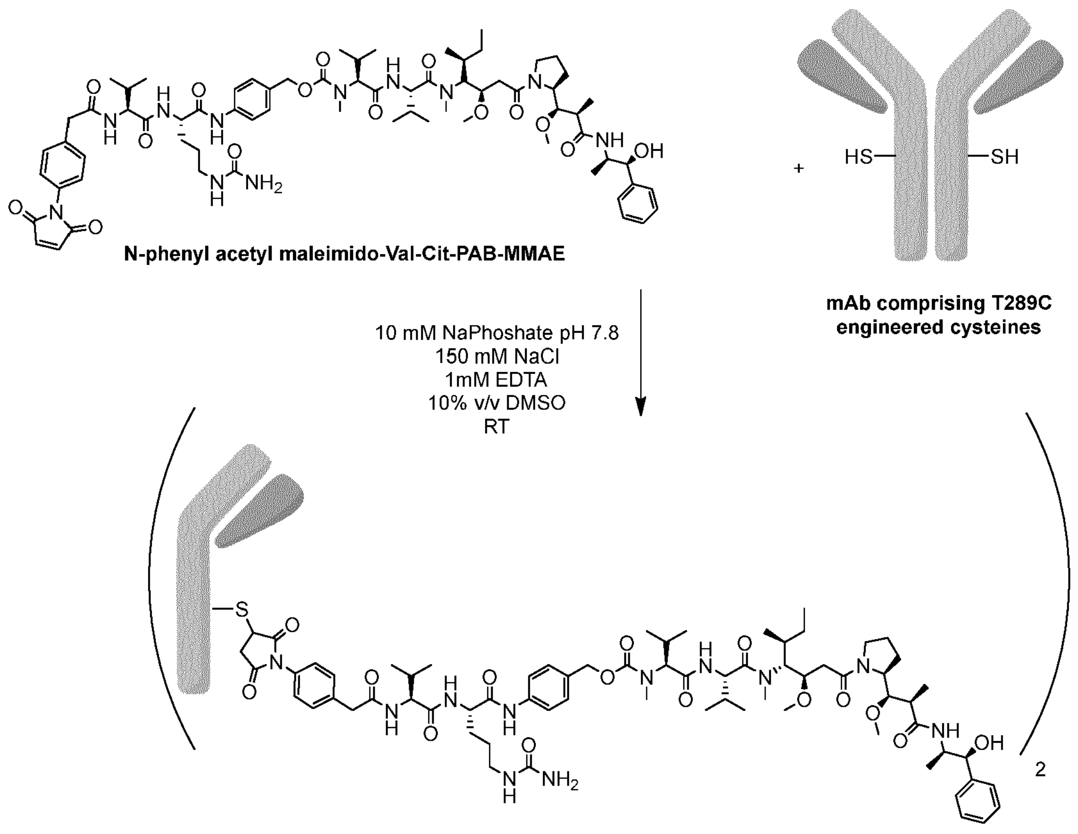


N-phenyl acetyl maleimido-Val-Cit-PAB-MMAE

mAb comprising T289C engineered cysteines

**MMAE-mAb conjugate linked by N-phenyl thiosuccinimide**

N-phenyl acetyl maleimido-Val-Cit-PAB-MMAE was conjugated to mAb (comprising a T289C mutation of the Fc) in several steps. First, mAbs were mildly reduced to generate free sulfhydryls by combining 5 mL of 1.6 mg/mL mAb solution in 10 mM PBS, pH 7.2 (8 mg mAb, 53.3 nM, 1 eq) was combined with 43 µL of 50 mM TCEP solution in water (2.15 µmol, 40 eq) followed by gentle mixing at 37 °C for 1 hr. Reduced mAb was transferred to a slide-a-lizer dialysis cassette (10K MWCO) and dialyzed against PBS, 1mM EDTA, pH 7.2-7.8, 4°C for 24 hr with several buffer changes. Reduced mAb was oxidized to reform internal disulfides by addition of dehydroascorbic acid (21 µL of 50 mM stock in DMSO, 1.1 µmol, 20 eq) followed by gentle mixing for 4 hr at room temperature. Oxidized mAb solution (2.5 mL, 27 nmol, 1 eq) was combined 10% v/v DMSO followed by addition of N-phenyl acetyl maleimido-Val-Cit-PAB-MMAE (27 µL of a 10 mM stock in DMSO, 270 nmol, 10 eq). The reaction proceeded at room temperature with mixing for 1 hr and then N-acetyl cysteine (21 µL of 100 mM stock in water, 2.2 µmol, 80 eq) was added to stop the reaction. The reaction mixture was then diluted 3-fold with distilled water and subjected to CHT chromatography to remove free unconjugated drug (Bio-Scale Mini Cartridge CHT Type 1140 µm media column). ADC was eluted with a gradient from buffer A (10 mM phoshate, pH 7.0) to buffer B (10 mM phosphate pH 7.0 containing 2M NaCl) over 25 minutes. After CHT chromatography the sample was buffer exchanged toIX PBS supplemented with 0.5 mM EDTA, pH 7.2 by dialysis in a slide-a-lyzer cassette at 4 °C. For samples subjected to mild hydrolysis, 10% v/v sodium phosphate solution (1M, dibasic) was added and the solution was incubated at 37 °C for 1 hr. Hydrolyzed samples were then buffer exchanged by dialysis to1X PBS (pH 7.2) supplemented with 0.5 mM EDTA. ADCs were characterized by reduced glycosylated LC/MS as described above. Conjugation efficiency and DAR was calculated using equation 1 and equation 2, respectively.

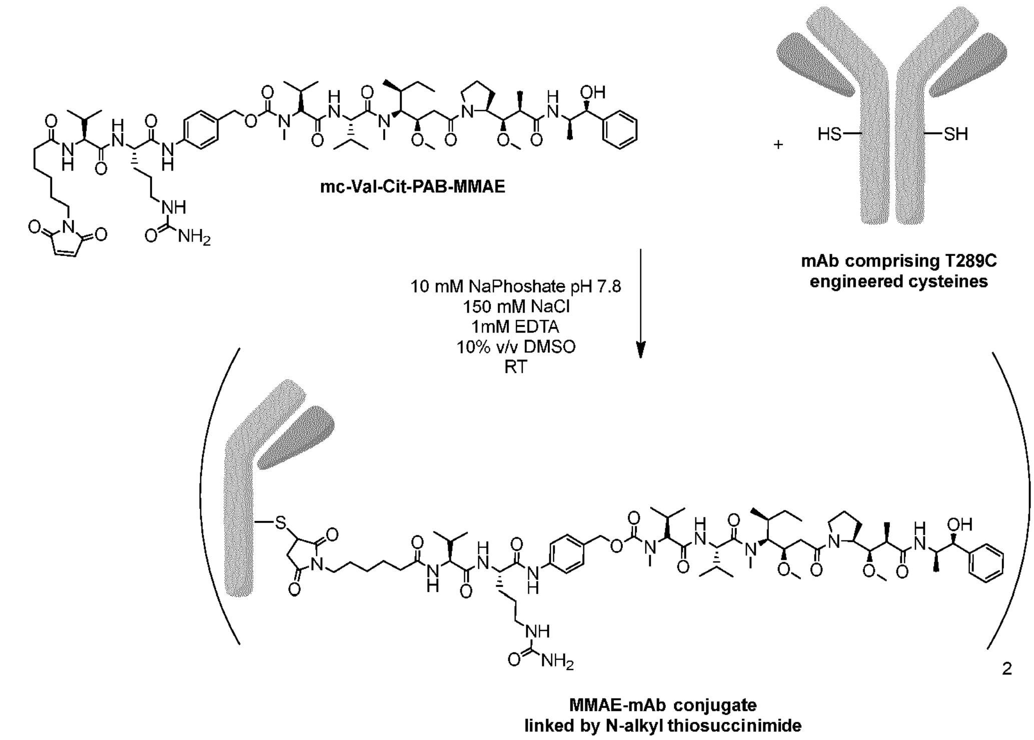


N-alkyl maleimido-Val-Cit-PAB-MMAE was conjugated to mAb (comprising a T289C mutation of the Fc) in in the same manner as N-phenyl maleimido-Val-Cit-PAB-MMAE (vide supra).

**Example 32 Analysis of mc-PAB-MMAE T289C mAb ADCs**

Representative reduced glycosylated mass spectrometry data for x-maleimide-Val-Cit-PAB-MMAE-T289C mAb conjugates is shown in Figure 22.

**Table 14**

| | **Unreacted mAb** | | **Alkyl maleimide-Val-Cit-PAB-MMAE conjugate** | | **Phenyl maleimide-Val-Cit-PAB-MMAE conjugate** | |
|---|---|---|---|---|---|---|
| | Peak | Intensity | Peak | Intensity | | |
| **Light Chain** | 23616.0 | 2241350 | 23616.0 | 638228 | 23615.9 | 681096 |
| **Heavy Chain (G0)** | 50756.6 | 2351111 | 50756.0 | 32756 | 50756.0 | 92886 |
| **Heavy Chain (GO+Na)** | 50776.3 | 693019 | Not observed | ----- | Not observed | ----- |
| **Heavy Chain (G0+2Na)** | 50798.4 | 357830 | Not observed | ----- | ----- | ----- |
| **Heavy Chain (G1)** | 50918.7 | 905663 | Not observed | ----- | ----- | ----- |
| **Heavy Chain (G1+Na)** | ---- | ----- | Not observed | ---- | ----- | ----- |
| **Heavy Chain (G0+1)** | ----- | ----- | 52072.9 | 509271 | 52093.2 | 547416 |
| **Heavy Chain (G0+1+Na/$H_2O$)** | ----- | ----- | 52094.1 | 142968 | 52110.9 | 725547 |
| **Heavy Chain (G0+1+Na/$H_2O$+Na** | ----- | ----- | 52115.8 | 132594 | 52133.4 | 275808 |

**Summary of MMAE T289C mAb Conjugation Data**

**Table 15** Conjugation 1

| | Conjugation (%) | DAR | Hydrolysis Pre-CHT | Hydrolysis Post CHT | Hydrolysis Post pH 8.6, 37 °C, 1 hr |
|---|---|---|---|---|---|
| **N-Phenyl Val-Cit-PAB-MMAE** | 91 | 1.82 | ND | ND | 100 |

**Table 16** Conjugation 2

| | Conjugation (%) | DAR | Hydrolysis Pre-CHT (%) | Hydrolysis Pre-CHT + pH 8.6, 37 °C, 1 hr (%) | Hydrolysis Post CHT (%) | Hydrolysis Post CHT +pH 8.6, 37 °C, 1 hr (%) |
|---|---|---|---|---|---|---|
| **N-Phenyl Val-Cit-PAB-MMAE** | 93 | 1.86 | ND | 95 | ND | 94 |
| **N-Alkyl Val-Cit-PAB-MMAE** | 96 | 1.91 | 9.6 | ND | 7.3 | ND |

**Table 17** Conjugation 3

| | Conjugation (%) | DAR | Hydrolysis Pre-CHT (%) | Hydrolysis Pre CHT + pH 8.6, 37 °C,1 hr (%) | Hydrolysis Post CHT (%) | Hydrolysis Post CHT +pH 8.6, 37 °C, 1 hr (%) |
|---|---|---|---|---|---|---|
| **N-Phenyl Val-Cit-PAB-MMAE** | 97 | 1.94 | ND | ND | 55 | 93 |
| **N-Alkyl Val-Cit-PAB-MMAE** | 97 | 1.94 | ND | ND | 1 | 2 |

**Example 32 Stability of MMAE-T289C mAb Conjugates in Buffer Containing Thiol**

MMAE-mAb conjugates were incubated in aqueous buffer containing β-mercaptoethanol (BME) to challenge the thiosuccinimide linkage towards thiol exchange reactions. MMAEs were conjugated to mAb containing the T289C mutation as described above. After conjugation, conjugates were immediately purified by CHT chromatography to remove unconjugated drug. Samples were then subjected to brief dialysis (Slide-a-lizer casette, 10 kDa MWCO, 4 °C, 2 hr) to exchange the buffer to 1X PBS, pH 7.2 containing 0.5 mM EDTA. After dialysis, samples were diluted to 0.2 mg/mL (1.33 μM mAb) with 1X PBS pH 7.2 containing 0.5 mM EDTA. For BME-containing samples, BME was added to a final concentration of 1% v/v (143 mM). Samples were further incubated at 37 °C under ambient atmosphere without stirring. Aliquots were removed at various time points, sterile filtered, reduced with DTT and then analyzed by LC/MS. Percent conjugated mAb and thiosuccinimide hydrolysis were determined from peak heights of mass spectra using Equation 1 and Equation 2, respectively. Deconjugated mAb and thiosuccinimide hydrolysis data were plotted as ln[concentration] vs. time (s) to obtain the psuedo-first order rate constants from the slope of the bestfit line.

**Example 33 Stability of MMAE T289C mAb Conjugates in Buffer Containing Thiol (BME)**

The results are shown in Figure 23.

**Table 18:** Kinetic parameters for deconjugation of alkyl thiosuccinimide conjugate

| | pH 7.2, 37 °C | | pH 7.2, 37 °C, +BME | |
|---|---|---|---|---|
| | Psuedo first order rate constant ($k_{obs}$, $s^{-1}$) | $T_{1/2}$ (hr) | Psuedo first order rate constant ($k_{obs}$, $s^{-1}$) | $T_{1/2}$ (hr) |
| N-alkyl maleimido-Val-Cit-PAB-MMAE | $1.4\times10^{-7}$ | 1375 | $2.1\times10^{-6}$ | 91.6 |

**Table 19:** Summary of initial hydrolysis and deconjugation data

| | Initial hydrolysis (%) | Max deconjugation -BME (%) | Max deconjugation +BME (%) |
|---|---|---|---|
| N-alkyl maleimido-Val-Cit-PAB-MMAE | 1 | 8 | 60 |
| N-phenyl maleimido-Val-Cit-PAB-MMAE | 55 | 7 | 12 |

This data shows that MMAE deconjugates in thiol-containing buffer and that conjugates prepared with a N-alkyl maleimide are less stable than MMAE conjugated with a N-phenyl maleimide.

**Example 34 Hydrolysis of MMAE-Thiosuccinimides in T289C mAb Conjugates**

The results are shown in Figure 24.

**Table 20:** Kinetic parameters for deconjugation of alkyl thiosuccinimide conjugate

| | pH 7.2, 37 °C | | pH 7.2, 37 °C, +BME | |
|---|---|---|---|---|
| | Psuedo first order rate constant ($k_{obs}$, $s^{-1}$) | $T_{1/2}$ (hr) | Psuedo first order rate constant ($k_{obs}$, $s^{-1}$) | $T_{1/2}$ (hr) |
| N-alkyl maleimido-Val-Cit-PAB-MMAE | $1.3\times10^{-6}$ | 148 | $1.4\times10^{-6}$ | 138 |

This data shows that MMAE conjugated with a N-alkyl maleimide is less stable than MMAE conjugated with a N-phenyl maleimide in the presence of free thiols. Ln plots used to calculate rate constants are not shown.

**Example 35 Stability of MMAE T289C mAb Conjugates in Buffer Containing Thiol (BME)**

The results are shown in Figure 25.

**Table 21:** Summary of initial hydrolysis and deconjugation data for MMAE conjugates

| | Intentional hydrolysis | Initial hydrolysis (%) | Max deconjugation -BME (%) | Max deconjugation +BME (%) |
|---|---|---|---|---|
| N-alkyl maleimido-Val-Cit-PAB-MMAE | No | 1 | 16 | 61 |
| N-phenyl maleimido-Val-Cit-PAB-MMAE | No | 57 | 4 | 15 |
| N-alkyl maleimido-Val-Cit-PAB-MMAE | Yes | 5 | 23 | 68 |
| N-phenyl maleimido-Val-Cit-PAB-MMAE | Yes | 93 | 3 | 8 |

This data shows that 1) MMAE conjugated with a N-alkyl maleimide is less stable than MMAE conjugated with aN-phenyl maleimide in the presence of free thiols, 2) N-alkyl maleimide MMAE conjugates are less sensitive to mild hydrolysis than the PEG-biotin analogue, 3) Mild hydrolysis does not improve the stability of N-alkyl maleimide-MMAE conjugates, 4) N-phenyl maleimide-MMAE conjugates are very responsive to mild hydrolysis, but this step is not necessary to achieve high stability, 5) The N-phenyl maleimide spontaneously hydrolyzes significantly more than N-alkyl maleimide when subjected to identical purification processes.

**Example 36 Comparison of PEG-biotin and MMAE Thiosuccinimide Hydrolysis Observed in the BME Challenge**

The results are shown in Figure 26.

**Table 22:** Thiosuccinimide hydrolysis kinetics for T289C mAb conjugate

| | pH 7.2, 37 °C | | pH 7.2, 37 °C, +BME | |
|---|---|---|---|---|
| **Conjugated Compound** | **Thiosuccinimide hydrolysis psuedo first order rate constant ($k_{obs}$, $s^{-1}$)** | **$T_{1/2}$ (hr)** | **Thiosuccinimide hydrolysis psuedo first order rate constant ($k_{obs}$, $s^{-1}$)** | **$T_{1/2}$ (hr)** |
| **N-alkyl maleimide-Val-Cit-PAB-MMAE** | $1.3\times10^{-6}$ | 148 | $1.4\times10^{-6}$ | 138 |
| **N-alkyl maleimide biotin** | $7.1\times10^{-6}$ | 27 | $6.5\times10^{-6}$ | 31 |

This data shows that slowly-hydrolysing thiosuccinimides are significantly affected by the chemistry upstream of the maleimide. The hydrophilic PEG-biotin chemistry increased the hydrolysis rate ~3-5 fold compared to the hydrophobic MMAE payload.

**Example 37 Comparison of Deconjugation in the Presence of β-Mercaptoethanol: PEG-Biotin vs. MMAE payload**

The results are shown in Figure 27.

**Table 23:** Kinetic parameters for thiosuccinimide hydrolysis and deconjugation determined from the BME assay

| Conjugated species | Deconjugation psuedo first order rate constant ($k_{obs}$, $s^{-1}$) | Max deconjugation (%) |
|---|---|---|
| **N-Alkyl maleimide Val-Cit-PAB-MMAE** | $1.9\times10^{-6}$ | 60 |
| **N-Alkyl maleimide-PEG-biotin** | $1.6\times10^{-6}$ | 35 |

**Example 38 Stability of MMAE-T289C ADCs in Mouse Serum**

MMAE ADCs were incubated in mouse serum to challenge the thiosuccinimide linkage towards deconjugation. MMAEs were conjugated to mAb containing the T289C mutation to produce the desired ADC as described above. After drug conjugation, ADCs were immediately purified by CHT chromatography to remove unconjugated drug. Samples were then subjected to brief dialysis (slide-a-lyzer casette, 10 kDa MWCO, 4 °C, 2 hr) to exchange the buffer to 1X PBS, pH 7.2 containing 0.5 mM EDTA. After dialysis, mAb concentrations were determined by A280 measurement (NanoDrop) and then added to normal mouse serum (Jackson Immunoresearch) to achieve a final concentration of 0.2 mg/mL (1.33 μM mAb). The total volume of ADC added to serum was less than 10%. The ADC-serum mixture was sterile filtered and incubated at 37 °C in a sealed container without stirring. Aliquots were removed at various time points, and frozen. Conjugated and unconjugated human antibody was recovered from mouse serum by immunoprecipitation using FC-specific anti-human IgG-agarose resin (Sigma-Aldrich). First, resin was rinsed twice with PBS, once with IgG elution buffer, and then twice more with PBS. ADC-mouse serum samples were then combined with anti-human IgG resin (100 μL of ADC-serum mixture, 50 μL resin slurry) and gently mixed for 15 minutes at room temperature. Resin was recovered by centrifugation and then washed twice with PBS. The resin pellet was resuspended in 100 μL IgG elution buffer (Thermoscientific) and further incubated for 5 minutes at room temperature. Resin was removed by centrifugation and

then 20 μL of 1M Tris, pH 8.0 was added to the supematent. Recovered human antibody solution was sterile filtered, reduced with DTT and then analyzed by LC/MS. Percent conjugated mAb and thiosuccinimide hydrolysis were determined from peak heights of mass spectra using Equation 1 and Equation 2, respectively. Deconjugated mAb and thiosuccinimide hydrolysis data were plotted as ln[concentration] vs. time (s) to obtain the psuedo-first order rate constants from the slope of the bestfit line.

The results are shown in Figure 28.

**Table 24:** Kinetic parameters for thiosuccinimide hydrolysis and deconjugation determined from the mouse serum stability assay

| | Intentional hydrolysis | Initial thiosuccinimide hydrolysis (%) | Max deconjugation (%) | Deconjugation psuedo first order rate constant ($k_{obs}$, $s^{-1}$) | Thiosuccinimide hydrolysis psuedo first order rate constant ($k_{obs}$, $s^{-1}$) |
|---|---|---|---|---|---|
| **N-alkyl maleimido-Val-Cit-PAB-MMAE** | No | 1 | 67 | $1.8\times10^{-6}$ | $7.8\times10^{-6}$ |
| **N-phenyl maleimido-Val-Cit-PAB-MMAE** | No | 57 | 5 | ND | ND |

This data shoes that 1) ADCs prepared with N-phenyl maleimide are more stable than ADCs prepared with N-alkyl maleimides, 2) Hydrolysis of N-phenyl thiosuccinide occurs faster than hydrolysis of N-alkyl thiosuccinimide, 3) thiol deconjugation occurs slightly slower than thiosuccinimide hydrolysis for N-alkyl thiosuccinimide, thus complete deconjugation is not observed, rates are similar for soluble and insoluble payloads. In the case of soluble payloads, the maximum deconjugation observed is lower, presumable due to the higher thiosuccinimide hydrolysis rate.

Figure 29A shows alkyl thiosuccinimide deconjugation and hydrolysis, pH 7.2 37∘C plus BME. This data shoes that deconjugation is linked to thiosuccinimide hydrolysis. Deconjugation plateaus after maximum thiosuccinimide hydrolysis is achieved, which is consistent with BME challenge experiments.

Figure 29B shows ADC deconjugation in mouse serum 7 days at 37∘C as an average +/standard deviation (n=3). This experiment is the same serum stability assay described above, performed in triplicate fore one timepoint. Data shows that N-alkyl maleimide Val-Cit-PAB-MMAE ADC is less stable than N-phenyl maleimide Val-Cit-PAB-MMAE ADC. The relative error for this experiment was typically less than 10%.

**Example 39 Activity of ADCs towards MDA-MB-361 cancer cells after incubation in mouse serum**

MDA-MB-361 breast cancer cells with high 5T4 expression were used in these studies. Cells were plated in 80 μL of RPMI1640 with 10% FBS into 96-well flat-bottomed plates at 2,000 MDA-MB-361 cells/well. Cells were allowed to adhere overnight. A 5X concentration of each ADC was prepared by diluting the test articles in culture medium. Twenty microliters of each test article was added to cells in duplicate such that the final dose curve range of 50 ng/mL down to 0.76 pg/mL in a stepwise 1:4 serial dilution series. The treated cells were cultured at 37°C/5% $CO_2$ for 6 days and cell viability was assessed with the CellTiter-Glo Luminescent Viability Assay from Promega. 100 μL of reconstituted CTG reagent was added each well, mildly shaken for 10 minutes at room temperature, and the absorbance of each sample at 560 nm was read using a Perkin Elmer EnVision luminometer. The percent cell viability was calculated by the following formula: (average luminescence of treated samples/average luminescence of untreated control samples) x 100. $IC_{50}$ values were determined using logistic non-linear regression analysis with GraphPad Prism software.

The results are shown in Figure 30 shown as the average ± relative error, n=2. This data shoes that ADC potency is preserved for stable (N-phenyl) ADCs and lost for unstable (N-alkyl maleimide) ADCs.

**Example 40 Heterobifunctional Linkers for Thiosuccinimide Stabilization and Payload Conjugation**

1) N-fluorophenyl maleimido-PEG4-BCN

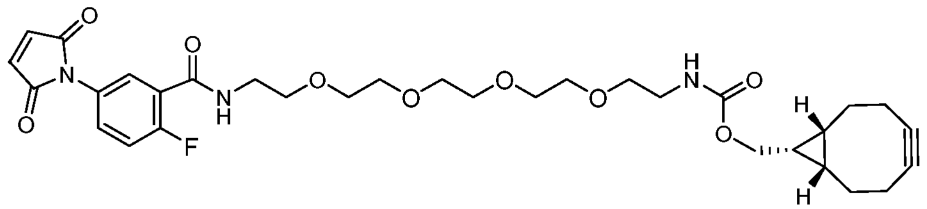

Chemical Formula: $C_{32}H_{40}FN_3O_9$
Molecular Weight: 629.68

;

2) N-phenyl maleimido-PEG4-BCN

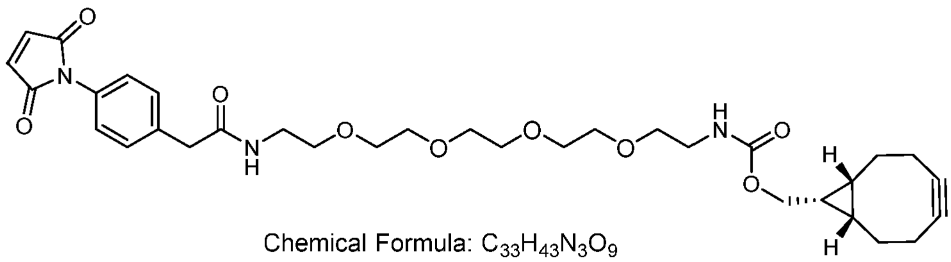

Chemical Formula: $C_{33}H_{43}N_3O_9$
Molecular Weight: 625.72

.

3) N-alkyl maleimido-PEG4-BCN SynChem catalogue product #SC50094 95% purity

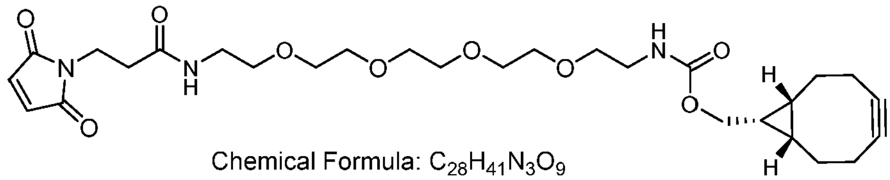

Chemical Formula: $C_{28}H_{41}N_3O_9$
Molecular Weight: 563.65

.

A series of heterobifunctional linkers comprising both alkyl- or N-aryl based maleimides and PEG-BCN functionality were prepared. The BCN group is reactive towards azides in a reaction known as "copper-free click conjugation".

**Example 41 Synthesis of N-fluorophenyl Maleimido-PEG-BCN**

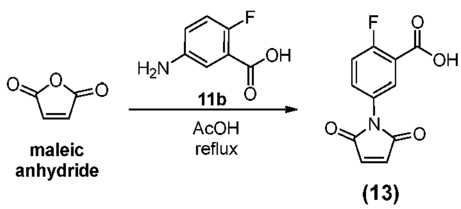


maleic anhydride

(13)

Reaction of maleic anhydride (**10a**, 0.313 g, 3.19 mmol) and 5-amino-2-fluorobenzoic acid (11b, 0.5 g, 3.22 mmol) in glacial AcOH under reflux furnished crude **9c** (0.72 g) as yellow solid after work up. The crud product was further purified by silica gel column chromatography to afford 0.21 g (0.89 mmol, 28% yield) of **9c** as a light yellow solid.

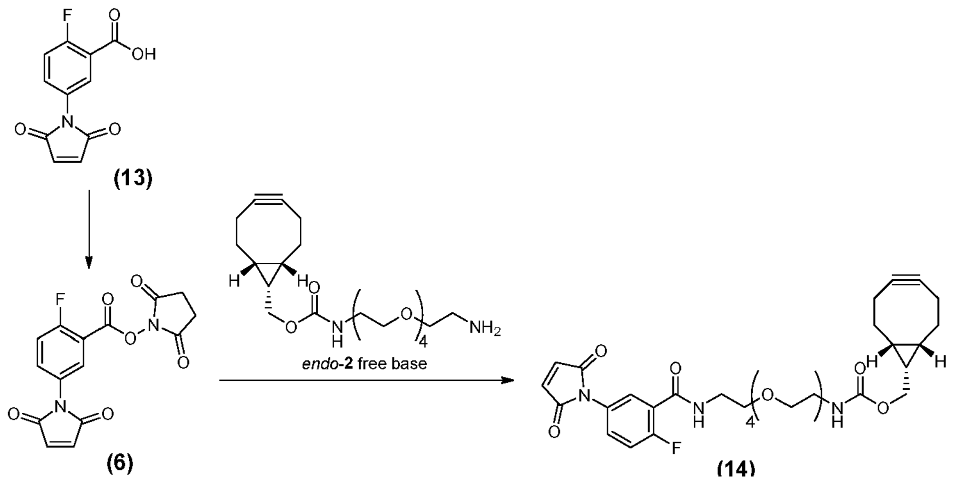


(13) (6) (14)

The NHS-activated maleimide (**6**) was prepared in situ from (**13**) and used without purification. Thus, (**13**) (0.137 g, 0.582 mmol) was reacted with N-hydroxysuccinimide (0.074 g, 0.64 mmol) in the presence of DCC (0.144 g, 0.698 mmol) in DME (5 mL) at room temperature for 1 h. After removal of the precipitated solid (DCU) by filtration, the filtrate containing the activated ester (**6**) was added dropwise into a solution of endo-**2** (0.2 g, 0.485 mmol) in DME (2 mL) at room temperature while stirring under $N_2$. After 1h, TLC and MS analyses indicated reaction completion. Excess DME was concentrated under vacuum and the crude residue obtained was purified by silica gel column chromatography under $N_2$, eluting with ethyl acetate followed by 4% MeOH in DCM, to furnish (**14**) (0.24 g, 0.38 mmol, 78.6% yield) as light yellow oil. The $^1H$ NMR and MS data confirmed the chemical structure; HPLC indicated 94% pure. MS (ESI) m/z 629.3 (M+1), 647.8 (M+$H_2O$), 652.8 (M+Na, base peak).

**Example 42 Synthesis of N-phenyl maleimido-PEG-BCN**

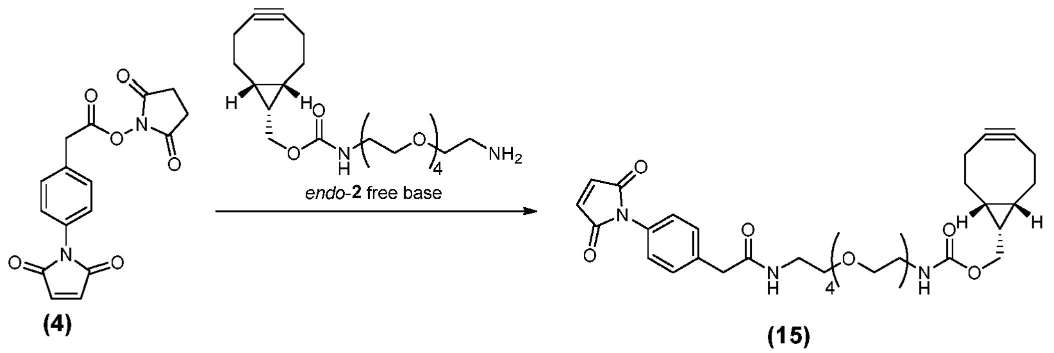


(4) (15)

Reaction between (4) (0.191 g, 0.582 mmol) and *endo-2* (0.2 g, 0.485 mmol) in DME (5 mL) was completed in 1 h at room temperature under $N_2$, as indicated by both TLC and MS. Excess DME was removed under vacuum and the crude residue was purified by silica gel column chromatography under $N_2$ pressure, eluting with ethyl acetate followed by 4% MeOH in DCM. The fractions containing the product were pooled and concentrated under vacuum. The residue was dissolved into DCM and transferred to a vial under $N_2$. Excess solvent was removed by $N_2$ bubbling to provide (15) (0.26 g, 85% yield) as light yellow oil. The $^1H$ NMR and MS data confirmed the chemical structure, with contaminant of residual solvents. MS (ESI) m/z 626.5 (M+1), 643.5 (M+$H_2O$), 648.8 (M+Na, base peak).

**Example 43 General Procedure for Conjugating x-Maleimide-PEG-BCNs to mAbs**

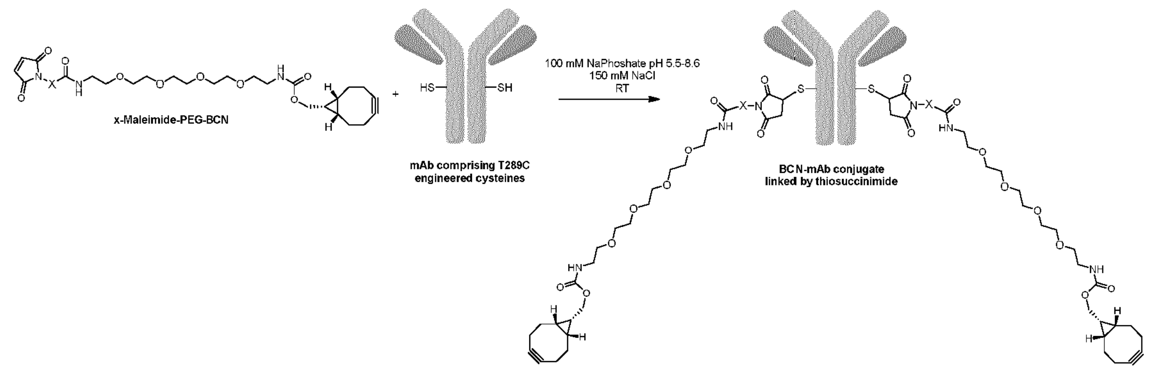


x-Maleimide-PEG-BCNs were conjugated to mAb (comprising a T289C mutation of the Fc) in several steps. First, mAbs were mildly reduced to generate free sulfhydryls by combining 5 mL of 1.6 mg/mL mAb solution in 10 mM PBS, pH 7.2 (8 mg mAb, 53.3 nM, 1 eq) was combined with 43 μL of 50 mM TCEP solution in water (2.15 μmol, 40 eq) followed by gentle mixing at 37 °C for 1 hr. Reduced mAb was transferred to a slide-a-lizer dialysis cassette (10K MWCO) and dialyzed against PBS, 1mM EDTA, pH 7.2-7.8, 4°C for 24 hr with several buffer changes. Reduced mAb was oxidized to reform internal disulfides by addition of dehydroascorbic acid (21 μL of 50 mM stock in DMSO, 1.1 μmol, 20 eq) followed by gentle mixing for 4 hr at room temperature. Oxidized mAb solution was adjusted to1.3 mg/mL mAb by addition of PBS. Next, 1.15 mL of mAb solution (1.5 mg, 10 nmol, 1 eq) was aliquoted into a vial followed by addition of x-maleimide-PEG-BCN stock solution (10 mM stock colution in DMAc, 20 nmol, 2 eq). The reaction mixture was briefly vortexed and incubated for 1 hr at 22 °C followed by addition of N-acetly cysteine (10 μL of a 100 mM solution in water, 1 μmol, 100 eq) and further incubation for 15 minutes to quench unreacted maleimide. All conjugation reactions were performed at room temperature (22 °C) under ambient atmosphere. Note that 2 eq x-maleimide-PEG-BCN relative to mAb = 1 eq x-maleimide-PEG-BCN relative to free cysteine contained in the mAb. This general procedure was modified as needed to achieve desired reaction stoichiometry (i.e. different maleimide:mAb feeds). Conjugates were analyzed by reduced glycolsylated mass spectrometry and conjugation efficiency was determined using equation 1.

**Example 44 Mass Spectrometry Analysis of N-phenyl Maleimide-PEG-BCN-mAb Conjugate**

Representative mass spectrometry data for N-phenyl-PEG-BCN-mAb conjugate prepared at 4 equivalents cross-linker are shown in Figure 31 and Table 25:

**Table 25**

| | Unreacted mAb | | N-phenyl maleimide-PEG-BCN-mAb conjugate | |
|---|---|---|---|---|
| | Peak | Intensity | Peak | Intensity |
| Light Chain | 23745.4 | 2241350 | 23745.3 | 1859865 |
| Heavy Chain (G0) | 50756.4 | 2520546 | 50757 | ----- |
| Heavy Chain (G0+Na) | 50777.3 | 703916 | N/D | ----- |
| Heavy Chain (G1) | 50918.7 | 954244 | N/D | ------ |
| Heavy Chain (G1+Na) | 50939.3 | 288623 | N/D | ------ |
| Heavy Chain (G0+1) | ------ | ------- | 51382.1 | 1026822 |
| Heavy Chain (G0+1+Na/$H_2O$) | ------ | ------ | 51400.8 | 515263 |
| Heavy Chain (G0+$H_2O$+Na) | | | 51421.9 | 188687 |

N-phenyl-maleimide-PEG-BCN conjugated efficiently and specifically to mAb comprising the T289C mutation. No additional conjugation was observed on the heavy chain or light chain up to 10 molar equivalents crosslinker.

**Example 45 Mass Spectrometry Analysis of N-Fluorophenyl Maleimide-PEG-BCN-mAb Conjugate**

Representative mass spectrometry data for N-fluorophenyl-PEG-BCN-mAb conjugate prepared at 4 equivalents

cross-linker in Figure 32 and Table 26:

**Table 26**

| | Unreacted mAb | | N-fluorophenyl maleimide-PEG-BCN-mAb conjugate | |
|---|---|---|---|---|
| | Peak | Intensity | Peak | Intensity |
| **Light Chain** | 23745.4 | 2241350 | 23745.3 | 1859865 |
| **Heavy Chain (G0)** | 50756.4 | 2520546 | 50757 | ---- |
| **Heavy Chain (G0+Na)** | 50777.3 | 703916 | N/D | ---- |
| **Heavy Chain (G1)** | 50918.7 | 954244 | N/D | ---- |
| **Heavy Chain (G1+Na)** | 50939.3 | 288623 | N/D | ---- |
| **Heavy Chain (G0+1)** | ---- | ---- | 51385.9 | 689372 |
| **Heavy Chain (G0+l+Na/$H_2O$)** | ---- | ---- | 51404.4 | 476344 |

N-fluorophenyl-maleimide-PEG-BCN conjugated efficiently and specifically to mAb comprising the T289C mutation. No additional conjugation was observed on the heavy chain or light chain up to 10 molar equivalents crosslinker.

**Example 46 Mass Spectrometry Analysis of N-Alkyl Maleimide-PEG-BCN-mAb Conjugate**

Summary of mass spectrometry data for N-alkyl-PEG-BCN-mAb conjugate prepared at 4 equivalents cross-linker in Figure 33 and below:

**Table 27**

| | Unreacted mAb | | N-alkyl maleimide-PEG-BCN-mAb conjugate | |
|---|---|---|---|---|
| | Peak | Intensity | Peak | Intensity |
| **Light Chain** | 23615.9 | 501138 | 23615.9 | 350163 |
| **Heavy Chain (G0)** | 50756.3 | 566918 | 50757 | ---- |
| **Heavy Chain (G0+Na)** | 50777.5 | 208504 | N/D | ---- |
| **Heavy Chain (G1)** | 50918.4 | 337042 | N/D | ---- |
| **Heavy Chain (G1+Na)** | 50939.6 | 126619 | N/D | ---- |
| **Heavy Chain (G0+1)** | ---- | ---- | 51320.2 | 244596 |
| **Heavy Chain (G0+1+Na/$H_2O$)** | ---- | ---- | 51340.4 | 122749 |

N-alkyl-maleimide-PEG-BCN conjugated efficiently and specifically to mAb comprising the T289C mutation. No additional conjugation was observed on the heavy chain or light chain up to 10 molar equivalents crosslinker.

**Example 47 Conjugation Efficiency of x-Maleimide-PEG-BCNs to T289C mAb**

The results are shown in Figure 35. All cross-linkers conjugated efficiently to the mAb comprising the T289C mutation. Complete conjugation is observed above 2 equivalents of maleimide.

**Example 48 Thiosuccinimide Hydrolysis Kinetics for PEG-BCN conjugates**

x-Maleimide-PEG-BCN-mAb conjugates were incubated in buffer solutions and monitored by LC/MS over time to observe thiosuccinimide hydrolysis. Conjugated T289C mAbs were diluted to 0.22 mg/mL with 1X PBS containing 0.5 mM EDTA, pH 7.2 and then dithiothreitol was added (0.5 M stock in water) to achieve a final concentration of 42 mM. Samples were placed into the LC/MS autosampler at 22 °C and injected periodically. Thiosuccinimide hydrolysis was confirmed by addition of 18 amu to the mAb conjugate peak in the mass spectrum. Semi-quantitative analysis of

hydrolysis was performed using peak intensities in deconvoluted mass spectra and equation 3, which includes background subtraction using the T=0 measurement. Data was then converted into loss of thiosuccinimide (M) over time and plotted as ln[thiosuccinimide] vs seconds. The slope of the best fit line yielded the psuedo first-order rate constant for thiosuccinimide hydrolysis. There results are shown in Figure 36 and Table 28.

**Table 28:** Kinetic constants for thiosuccinimide hydrolysis of mAb-PEG-BCN conjugates at T289C

| | pH 7.2, 22 °C | |
|---|---|---|
| | **Psuedo first order rate constant ($k_{obs}$, $s^{-1}$)** | **Half life (hr)** |
| **Alkyl thiosuccinimide** | $1.6 \times 10^{-6}$ | 120.3 |
| **Phenyl thiosuccinimide** | $1.9 \times 10^{-5}$ | 10.1 |
| **F-phenyl thiosuccinimide** | $3.1 \times 10^{-5}$ | 6.2 |

X-Maleimide-PEG-BCN conjugates hydrolyze in a similar trend as that observed for PEG-biotin conjugates; i.e. F-phenyl thiosuccinimide>phenyl thiosuccinimide»alkyl thiosuccinimide.

**Example 49 Sequential Addition of Payloads to T289C mAb**

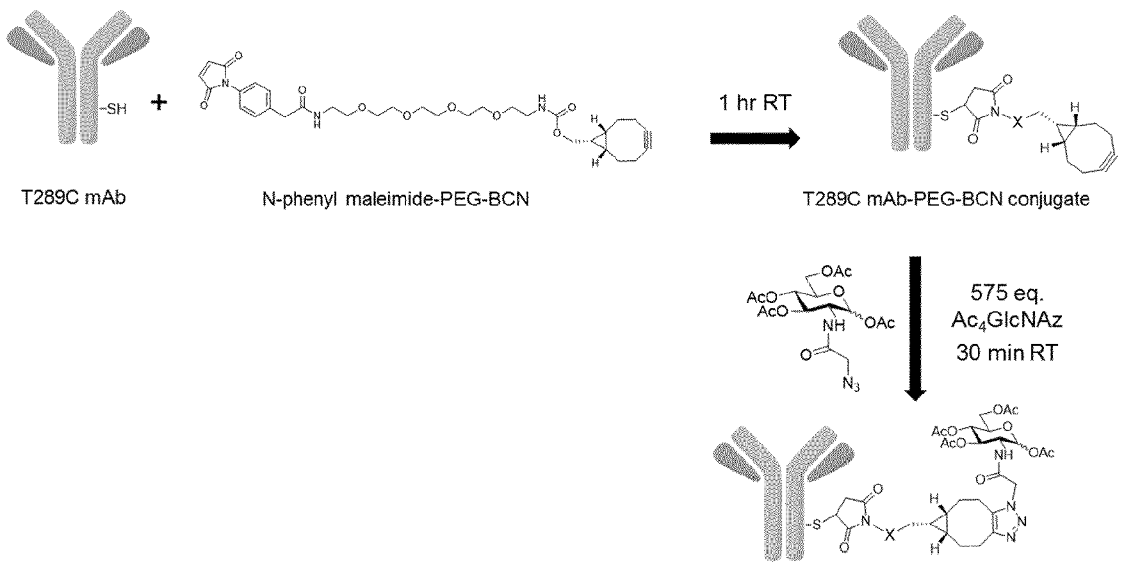


T289C mAb

N-phenyl maleimide-PEG-BCN

T289C mAb-PEG-BCN conjugate

T289C mAb-PEG-BCN-$AC_4GlcNAz$ conjugate

N-phenyl-maleimide-PEG-BCN-mAb conjugate (100 µL of 1.3, mg/mL solution in PBS with 0.5 mM EDTA, 0.87 nmol) was combined with $AC_4GlcNAz$ (1 µL of 500 mM stock in DMSO, 500 nmol). The reaction solution was incubated at room temperature without stirring for 1 hr at 22 °C. For long-term reactivity studies, mAb-BCN conjugate was stored at 4 oC in PBS with 0.5 mM EDTA, pH 7.2 and aliquots were removed and reacted with $AC_4GlcNAz$ as described above. Conjugates were analyzed by reduced glycosylated mass spectrometry and conjugation efficiency was determined using equation 1.

Analysis of mAb-BCN-Ac4GlcNAz conjugates are shown in Figure 37. BCN groups reacted completely with $AC_4GlcNAz$.

**Example 50 Reactivity of mAb-BCN Conjugate After Storage**

The results are shown in Figure 38 and Table 29.

**Table 29:** Summary of BCN reactivity after cross-linker conjugation to mAb and storage at 4 °C

| Days storage at 4 °C | Azide conjugation |
|---|---|
| 1 | quantitative |
| 4 | quantitative |
| 7 | quantitative |
| 10 | quantitative |
| 15 | quantitative |
| 21 | quantitative |

Quantitative reaction of BCN was observed for all samples. No unreacted mAb-BCN could be detected by mass spectrometry. BCN groups show no loss of reactivity for > 21 days after conjugation and storage at 4 oC.

**Example 51 Conjugation of azido-PBD to BCN-modified mAb**

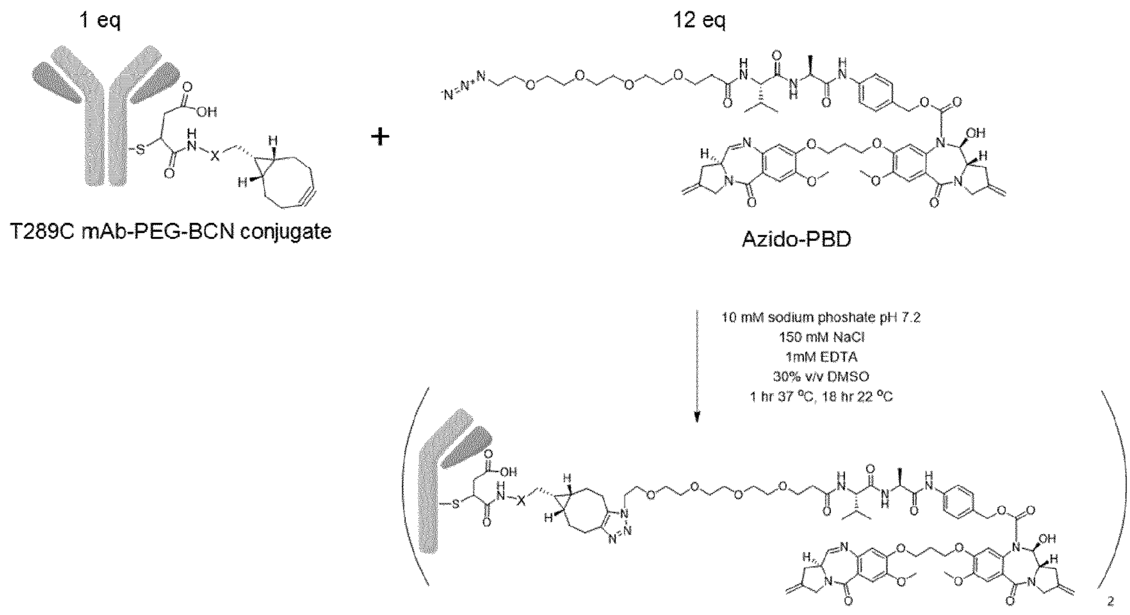


1 eq

12 eq

T289C mAb-PEG-BCN conjugate

Azido-PBD

PBD-mAb conjugate linked by thioether and triazole

N-phenyl-maleimide-PEG-BCN-T289C mAb conjugates were prepared as described above (at 1.5 mg/mL mAb) and stored at 4 °C until reaction with azido-PBD. For PBD conjugation, mAb solution was first combined with DMSO to achieve a final concentration of 30% v/v DMSO and 1.05 mg/mL mAb-BCN. Next, mAb-BCN/DMSO solution (1.89 mL, 13 nmol mAb, 1 eq.) was transferred to a vial and azido-PBD was added (16 $\mu$L of 10 mM stock in DMSO, 160 nmol, 12 eq.). The reaction mixture was incubated at 37 °C for one hour and then 22 °C for 18 hr. The reaction mixture was first purified by dialysis (10K MWCO slide-a-lyzer cassette, 1X PBS with 0.5 mM EDTA, pH 7.2, 4 °C, 18 hr) and then CHT chromatography as described above. Reaction products were analyzed by reduced glycosylated mass spectrometry. Conjugation efficiency and DAR was calculated using equation 1 and equation 2, respectively.

**Example 52 Analysis of mAb-PBD Conjugates**

Representative mass spectrometry data for 5T4-targeted T289C mAb - N-phenyl maleimide-PEG-BCN conjugate are shown in Figure 38.

**Table 30:** Summary of mAb-PBD Conjugates Prepared by Sequential Addition to T289C

| | **N-phenyl maleimide-PEG-BCN mAb conjugate** | | **N-phenyl maleimide-PEG-BCN -PBD mAb conjugate** | |
|---|---|---|---|---|
| | **Conjugation (%)** | **DAR** | **Conjugation [a] (%)** | **DAR [b]** |
| **5T4-targeted T289C mAb** | 94 | 1.87 | 81 | 1.52 |
| **Dummy T289C mAb** | 90 | 1.80 | 81 | 1.45 |
| a) *Calculated relative to BCN groups*<br>b) *Calculated relative to mAb. Unreacted mAb and mAb+BCN were considered as unconjugated* | | | | |

**Example 53 *In vitro* Activity of mAb-BCN-PBD ADCs Towards MDA-MB-361 Breast Cancer Cells**

MDA-MB-361 breast cancer cells with high ST4 expression were used in these studies. Cells were plated in 80 μL of RPM11640 with 10% FBS into 96-well flat-bottomed plates at 2,000 MDA-MB-361 cells/well. Cells were allowed to adhere overnight. A 5X concentration of each ADC was prepared by diluting the test articles in culture medium. Twenty microliters of each test article was added to cells in triplicate such that the final dose curve range of 50 ng/mL down to 0.76 pg/mL in a stepwise 1:4 serial dilution series. The treated cells were cultured at 37°C/5% $CO_2$ for 6 days and cell viability was assessed with the CellTiter-Glo Luminescent Viability Assay from Promega. 100 μL of reconstituted CTG reagent was added each well, mildly shaken for 10 minutes at room temperature, and the absorbance of each sample at 560 nm was read using a Perkin Elmer EnVision luminometer. The percent cell viability was calculated by the following formula: (average luminescence of treated samples/average luminescence of untreated control samples) × 100. $IC_{50}$ values were determined using logistic non-linear regression analysis with GraphPad Prism software.

The results are shown in Figure 40 and Table 25.

**Table 31:** Summary of PBD conjugate DAR Values and Potency *in vitro*

| | **PBD DAR** | **$IC_{50}$ ng/mL** |
|---|---|---|
| **Dummy mAb T289C-BCN** | 0 | >10,000 |
| **5T4 mAb T289C-BCN** | 0 | >10,000 |
| **Dummy mAb T289C-BCN-PBD** | 1.45 | -10,000 |
| **5T4 mAb T289C-BCN-PBD** | 1.52 | 4.6 |

## Claims

**1.** A method for preparing an antibody conjugated to a payload comprising the step of:

a) performing a Michael addition reaction with the maleimide entity in the molecule of formula (I):

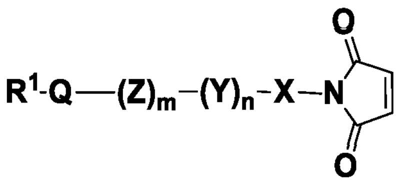

(I)

and an antibody molecule comprising at least one thiol group wherein:

n is 0 or 1;
m is 0 or 1;
q is 0 or an integer in the range 1 to 12, for example 1 to 6, such as 2 or 3;
Q is a bond or a residue from a conjugation component;

X is $C_{0-18}$ alkylene$C_{6-36}$ Aryl,
wherein the aryl has 0, 1, 2, 3 or 4 substituent independently selected from the group comprising halogen, hydroxyl, $C_{1-6}$ alkyl, $C_{1-6}$ alkoxy, $-COOR^3$, - $COR^3$, -CN, $-CF_3$, $-NO_2$, $-SO_2$, $-SO_3$, $-NR^4R^5$, $-PO_4$, and $-(OCH_2CH_2)_q-OR^3$;
Y is C(O)
Z is a saturated or unsaturated branched or unbranched $C_{1-30}$ alkylene chain, wherein one or more carbons (such as 1, 2, 3, 4, 5, 6, 7 or 8) are optionally independently replaced by -O-, N and the chain is optionally bears one or more (such as 1, 2, 3 or 4) C(O) substituents;
$R^1$ is H, a solid surface or a payload molecule;
$R^3$ is H or $C_{1-6}$ alkyl;
$R^4$ is H or $C_{1-6}$ alkyl;
$R^5$ is H or $C_{1-6}$ alkyl;

b) wherein $R^1$ is a payload molecule or a solid surface hydrolysing the resultant thio-succinimide entity formed by the reaction of compound of formula (I) and the antibody, or
c) wherein $R^1$ is H performing a conjugation with a payload, a conjugation component or solid surface, followed by hydrolysing the thio-succinimide entity formed by the reaction of compound of formula (I) and the antibody.

2. A method according to claim 1, wherein X is $C_{0-15}$ alkylene$C_{6-10}$ Aryl.

3. A method according to claim 1 or 2, wherein n is 1 of formula (II):

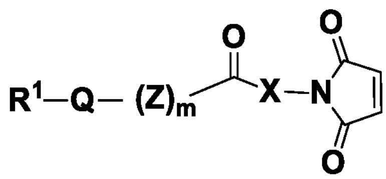
(II)

and pharmaceutically acceptable salts thereof wherein $R^1$, Q, Z, X and m are defined above for compounds of formula (I).

4. A method according to claim 1 or 2, wherein n is 0 of formula (III):

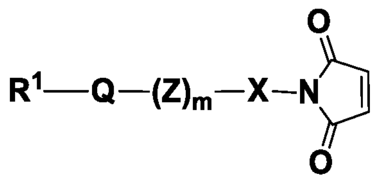
(III)

and pharmaceutically acceptable salts thereof wherein $R^1$, Q, Z, X and m are defined above for compounds of formula (I).

5. A method according to claim 1 or 3, wherein the maleimide molecule is in a compound of formula (IIa):

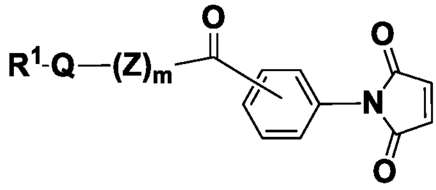
(IIa)

and pharmaceutically acceptable salts thereof wherein $R^1$, Q, Z and m are defined above for compounds of formula (I), and the phenyl has 0, 1, 2, 3 or 4 substituent independently selected from the group comprising halogen, hydroxyl,

$C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**6.** A method according to claim 5, wherein the group $R^1Q(Z)_mC(O)$- is in the para position as shown in the compound of formula (**IIaa**):

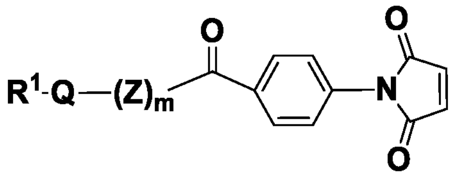
(IIaa)

and pharmaceutically acceptable salts thereof wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**7.** A method according to claim 5, wherein group $R^1Q(Z)_mC(O)$- is in the meta position as shown in the compound of formula (**IIaa'**):

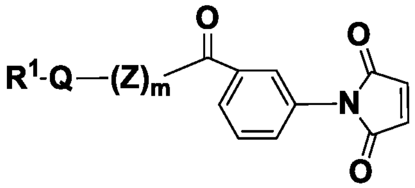
(IIaa')

and pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**8.** A method according to claim 1, 3 or 5, wherein the maleimide molecule is in a compound of formula (**IIaaa**):

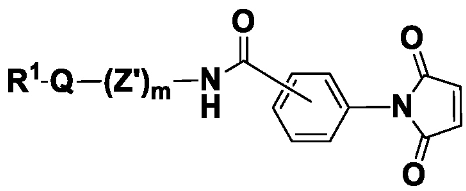
(IIaaa)

and pharmaceutically acceptable salts thereof, wherein $R^1$, Q, and m are defined above for compounds of formula (**I**),
the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$, and Z' is a saturated or unsaturated branched or unbranched $C_{1-24}$ alkylene chain, wherein one or more carbons are optionally independently replaced by -O-, N and the chain is optionally bears one or more C(O) substituents.

**9.** A method according to claim 8, wherein the group $R^1Q(Z')mNC(O)$- is in the meta or para position.

**10.** A method according to claim 1 or -3 wherein the maleimide entity is in a molecule of formula (**IIb**):

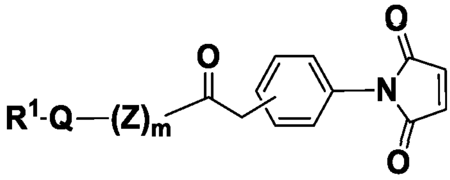

(IIb)

and pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**11.** A method according to claim 10, wherein the group $R^1Q(Z)_mC(O)$- is in the para position as shown in the compound of formula (**IIbb**):

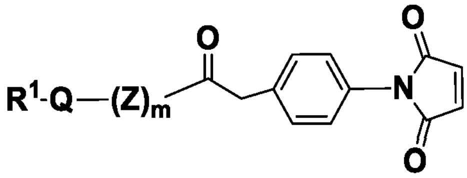

(IIbb)

and pharmaceutically acceptable salts thereof, wherein n, Q, Z, X, $R^1$ and m are defined above in claim 1.

**12.** A method according to claim 10, wherein the group $R^1Q(Z)_mC(O)$- is in the meta position as shown in the compound of formula (**IIbb**'):

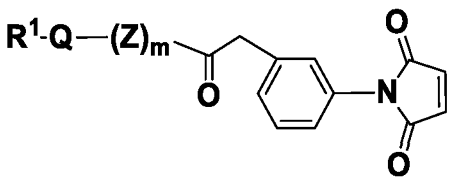

(IIbb')

and pharmaceutically acceptable salts thereof, wherein Q, Z, X, $R^1$ and m are defined above in claim 1.

**13.** A method according to claim 1, 3 or 10, wherein the maleimide entity is in a molecule of formula (**IIbbb**):

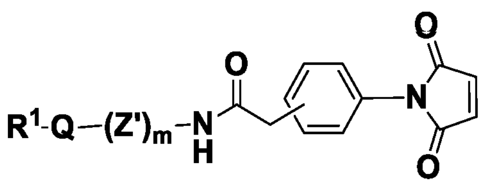

(IIbbb)

wherein $R^1$, Q and m are defined above for compounds of formula (**I**) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$
Z' is a saturated or unsaturated branched or unbranched $C_{1-24}$ alkylene chain,
wherein one or more carbons are optionally independently replaced by -O-, N and the chain is optionally bears one or more C(O) substituents.

**14.** A method according to claim 13, wherein the group $R^1Q(Z')mNC(O)$- is in the meta or para position of the phenyl ring.

**15.** A method according to claim 1 or 4, wherein the maleimide molecule is in a compound of formula (**IIIa**):

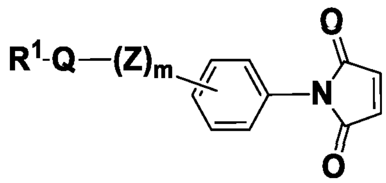

(IIIa)

and pharmaceutically acceptable salts thereof, wherein $R^1$, Q, Z and m are defined above for compounds of formula (I), and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$- and $-(OCH_2CH_2)_q-OR^3$.

**16.** A method according to claim 1 or 4, wherein the group $R^1Q(Z)_m$- is in the para position as shown in the compound of formula (**IIIaa**):

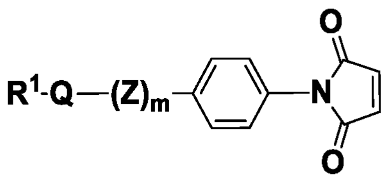

(IIIaa)

and pharmaceutically acceptable salts thereof wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**17.** A method according to claim 1 or 4, the group $R^1Q(Z)_m$- is in the meta position as shown in the compound of formula (**IIIaa**'):

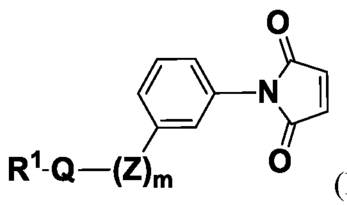

(IIIaa')

and pharmaceutically acceptable salts thereof, wherein $R^1$, Q, Z and m are defined above for compounds of formula (I) and the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$.

**18.** A method according to claim 1, 3 or 14, wherein the maleimide molecule is in a compound of formula (**IIIaaa**):

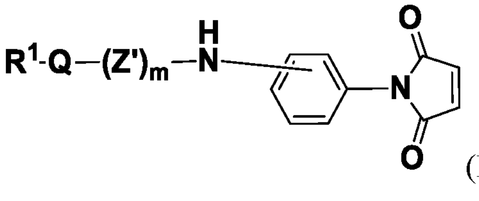

(IIIaaa)

and pharmaceutically acceptable salts thereof wherein $R^1$, Q, and m are defined above for compounds of formula (**I**),
the phenyl has 0, 1, 2, 3 or 4 substituent independently selected from the group comprising halogen, hydroxyl, $C_{1-6}$ alkyl, $C_{1-6}$ alkoxy, $-COOR^3$, $-COR^3$, -CN, $-CF_3$, $-NO_2$, $-SO_2$, $-SO_3$, $-NR^4R^5$, $-PO_4$ and $-(OCH_2CH_2)_q-OR^3$, and Z' is a saturated or unsaturated branched or unbranched $C_{1-24}$ alkylene chain, wherein one or more carbons are optionally independently replaced by -O-, N and the chain is optionally bears one or more C(O) substituents.

**19.** A method according to any one of claims 1 to 18, wherein Z or Z' represents $-C_{1-12}$ alkylene- or $-(CH_2CH_2O)_{1-8}-$.

**20.** A method according to any one of claims 1 to 19, wherein $R^1$ is selected from the group comprising a toxin, a drug molecule (such as cytotoxic agent), a polymer, an antibody or binding fragment thereof.

**21.** A method according to claim 20, wherein the drug molecule is selected from the comprising an auristatin, for example selected from the group comprising a tubulysin,a pyrrolobenzodiazepine (PBD), MMAE (monomethyl auristatin E) and MMAF (monomethyl auristatin F).

## Patentansprüche

**1.** Verfahren zur Herstellung eines Antikörpers, der mit einer Nutzlast konjugiert ist, wobei es den folgenden Schritt umfasst:

a) Durchführen einer Michael-Additionsreaktion mit der Maleimid-Einheit in dem Molekül der Formel (I):

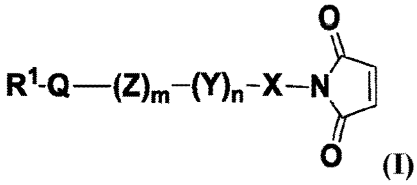

(I)

und einem Antikörpermolekül, das mindestens eine Thiolgruppe umfasst, wobei:

n gleich 0 oder 1 ist;
m gleich 0 oder 1 ist;
q gleich null oder einer ganzen Zahl im Bereich von 1 bis 12, beispielsweise 1 bis 6, wie etwa 2 oder 3, ist;
Q für eine Bindung oder einen Rückstand aus einem Konjugationsbestandteil steht;
X für $C_{0-18}$-Alkylen-$C_{6-36}$-aryl steht,
wobei das Aryl mit 0, 1, 2, 3 oder 4 Substituenten versehen ist, die auf unabhängige Weise aus der Gruppe ausgewählt sind, die Halogen, Hydroxyl, $C_{1-6}$-Alkyl, $C_{1-6}$-Alkoxy, $-COOR^3$, $-COR^3$, -CN, $-CF_3$, $-NO_2$, $-SO_2$, $-SO_3$, $-NR^4R^5$, $-PO_4$ und $-(OCH_2CH_2)_q-OR^3$ umfasst;
Y Für C(O) steht
Z für eine gesättigte oder ungesättigte verzweigte oder unverzweigte $C_{1-30}$-Alkylenkette steht, wobei ein oder mehrere Kohlenstoffe (wie etwa 1, 2, 3, 4, 5, 6, 7 oder 8) möglicherweise unabhängig voneinander durch -O-, N ersetzt sind und die Kette möglicherweise mit einem oder mehreren (wie etwa 3 oder 4) C(O)-Substituenten versehen ist;
$R^1$ für H, eine feststoffliche Oberfläche oder ein Nutzlastmolekül steht;
$R^3$ für H oder $C_{1-6}$-Alkyl steht;
$R^4$ für H oder $C_{1-6}$-Alkyl steht;
$R^5$ für H oder $C_{1-6}$-Alkyl steht;

b) wobei $R^1$ für ein Nutzlastmolekül oder für eine feststoffliche Oberfläche steht, welche die entstehende Thiosuccinmid-Einheit hydrolysiert, wie sie durch die Reaktion der Verbindung der Formel (I) mit dem Antikörper gebildet wird, oder
c) wobei $R^1$ für H steht, welches eine Konjugation mit einer Nutzlast, einem Konjugationsbestandteil oder einer feststofflichen Oberfläche erfährt, woraufhin die Thiosuccinmid-Einheit, wie sie durch die Reaktion der Verbindung der Formel (I) mit dem Antikörper gebildet wird, hydrolysiert wird.

**2.** Verfahren nach Anspruch 1, wobei X für $C_{0-15}$-Alkylen-$C_{6-10}$-aryl steht.

**3.** Verfahren nach Anspruch 1 oder 2, wobei n gemäß der folgenden Formel **(II)** gleich 1 ist:

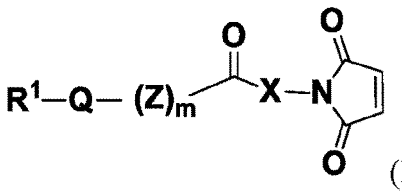

(II)

und pharmazeutisch annehmbare Salze davon, wobei $R^1$, Q, Z, X und m den obigen Begriffsbestimmungen für Verbindungen der Formel (I) entsprechen.

**4.** Verfahren nach Anspruch 1 oder 2, wobei n der folgenden Formel **(III)** gleich 0 ist:

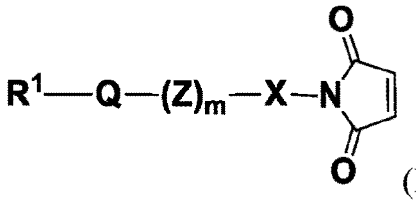

(III)

und pharmazeutisch annehmbare Salze davon, wobei $R^1$, Q, Z, X und m den obigen Begriffsbestimmungen für Verbindungen der Formel (I) entsprechen.

**5.** Verfahren nach Anspruch 1 oder 3, wobei es sich bei dem Maleimid-Molekül um eine Verbindung der Formel (**IIa**) handelt:

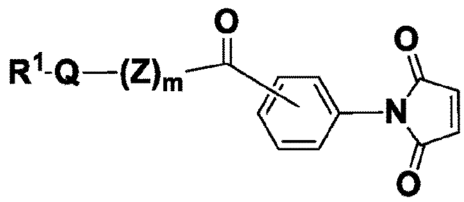

(IIa)

und pharmazeutisch annehmbare Salze davon, wobei $R^1$, Q, Z und m den obigen Begriffsbestimmungen für Verbindungen der Formel **(I)** entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, $-COOR^3$, - $COR^3$, -CN, $-CF_3$, $-NO_2$, $-SO_2$, $-SO_3$, $-NR^4R^5$, $-PO_4$ und - $(OCH_2CH_2)_q$-$OR^3$ umfasst.

**6.** Verfahren nach Anspruch 5, wobei die Gruppe $R^1Q(Z)_mC(O)$- die para-Stellung einnimmt, wie es in der Verbindung der Formel **(IIaa)** dargestellt ist:

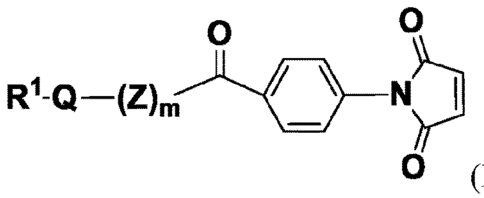

(IIaa)

und pharmazeutisch annehmbare Salze davon, wobei $R^1$, Q, Z und m den obigen Begriffsbestimmungen für Verbindungen der Formel (I) entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, $-COOR^3$, - $COR^3$, -CN, $-CF_3$, $-NO_2$, $-SO_2$, $-SO_3$, $-NR^4R^5$, $-PO_4$ und - $(OCH_2CH_2)_q$-$OR^3$ umfasst.

**7.** Verfahren nach Anspruch 5, wobei die Gruppe $R^1Q(Z)_mC(O)$- die meta-Stellung einnimmt, wie es der Verbindung der Formel (IIaa') dargestellt ist:

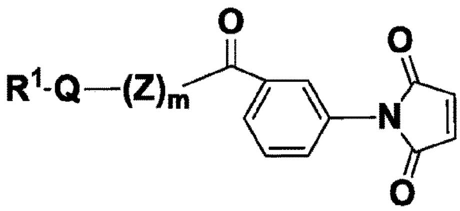

(IIaa')

und pharmazeutisch annehmbare Salze davon, wobei $R^1$, Q, Z und m den obigen Begriffsbestimmungen für Verbindungen der Formel (I) entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, -$COOR^3$, - $COR^3$, -CN, -$CF_3$, -$NO_2$, -$SO_2$, -$SO_3$, -$NR^4R^5$, -$PO_4$ und - $(OCH_2CH_2)_q$-$OR^3$ umfasst.

**8.** Verfahren nach Anspruch 1, 3 oder 5, wobei das Maleimid-Molekül in einer Verbindung der Formel **(IIaaa)** vorliegt:

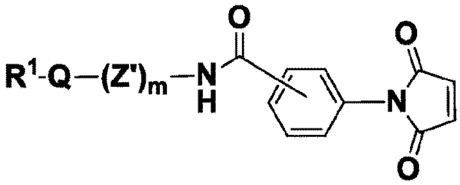

(IIaaa)

und pharmazeutisch annehmbare Salze davon, wobei $R^1$, Q und m den obigen Begriffsbestimmungen für Verbindungen der Formel **(I)** entsprechen,
wobei das Phenyl mit 0, 1, 2, 3 oder 4 Substituenten versehen ist, die auf unabhängige Weise aus der Gruppe ausgewählt sind, die Halogen, Hydroxyl, $C_{1-6}$-Alkyl, $C_{1-6}$-Alkoxy, -$COOR^3$, -$COR^3$, -CN, -$CF_3$, -$NO_2$, -$SO_2$, -$SO_3$, -$NR^4R^5$, -$PO_4$ und - $(OCH_2CH_2)_q$-$OR^3$ umfasst, und
Z' für eine gesättigte oder ungesättigte verzweigte oder unverzweigte $C_{1-24}$-Alkylenkette steht, wobei ein oder mehrere Kohlenstoffe möglicherweise unabhängig voneinander durch -O-, N ersetzt sind und die Kette möglicherweise mit einem oder mehreren C(O)-Substituenten versehen ist.

**9.** Verfahren nach Anspruch 8, wobei die Gruppe $R^1$Q(Z')mNC(O)- die meta- oder para-Stellung einnimmt.

**10.** Verfahren nach Anspruch 1 oder -3, wobei die Maleimid-Einheit in einem Molekül der Formel **(IIb)** vorliegt:

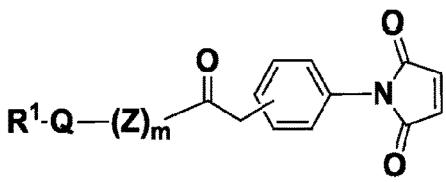

(IIb)

und pharmazeutisch annehmbare Salze davon, wobei $R^1$, Q, Z und m den obigen Begriffsbestimmungen für Verbindungen der Formel **(I)** entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, -$COOR^3$, - $COR^3$, -CN, -$CF_3$, -$NO_2$, -$SO_2$, -$SO_3$, -$NR^4R^5$, -$PO_4$ und - $(OCH_2CH_2)_q$-$OR^3$ umfasst.

**11.** Verfahren nach Anspruch 10, wobei die Gruppe $R^1Q(Z)_mC(O)$- die para-Stellung einnimmt, wie es in der Verbindung der Formel (**IIbb**) dargestellt ist:

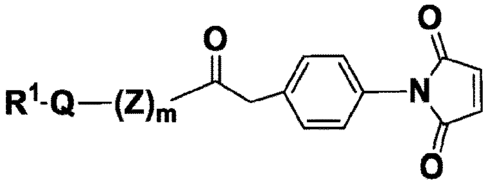

(IIbb)

und pharmazeutisch annehmbare Salze davon, wobei n, Q, Z, X, $R^1$ und m den obigen Begriffsbestimmungen in Anspruch 1 entsprechen.

**12.** Verfahren nach Anspruch 10, wobei die Gruppe $R^1Q(Z)_mC(O)$- die meta-Stellung einnimmt, wie es in der Verbindung der Formel **(IIbb')** dargestellt ist:

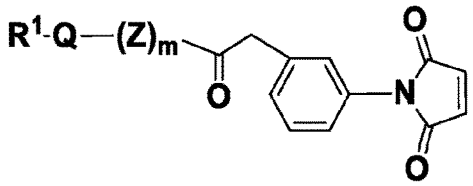

(IIbb')

und pharmazeutisch annehmbare Salze davon, wobei Q, Z, X, $R^1$ und m den obigen Begriffsbestimmungen in Anspruch entsprechen.

**13.** Verfahren nach Anspruch 1, 3 oder 10, wobei die Maleimid-Einheit in einem Molekül der Formel **(IIbbb)** vorliegt:

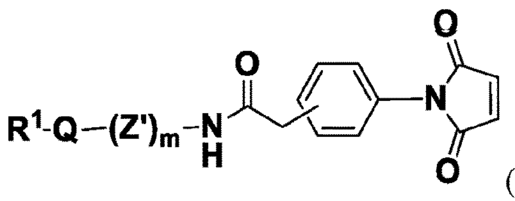

(IIbbb)

wobei $R^1$, Q und m den obigen Begriffsbestimmungen für Verbindungen der Formel **(I)** entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, - $COOR^3$, $-COR^3$, -CN, $-CF_3$, $-NO_2$, $-SO_2$, $-SO_3$, $-NR^4R^5$, $-PO_4$ und - $(OCH_2CH_2)_q$-$OR^3$ umfasst,
Z' für eine gesättigte oder ungesättigte verzweigte oder unverzweigte $C_{1-24}$-Alkylenkette steht, wobei ein oder mehrere Kohlenstoffe möglicherweise unabhängig voneinander durch -O-, N ersetzt sind und die Kette möglicherweise mit einem oder mehreren C(O)-Substituenten versehen ist.

**14.** Verfahren nach Anspruch 13, wobei die Gruppe $R^1$Q(Z')mNC(O)- die meta- oder para-Stellung des Phenylrings einnimmt.

**15.** Verfahren nach Anspruch 1 oder 4, wobei es sich bei dem Maleimid-Molekül um eine Verbindung der Formel **(IIIa)** handelt:

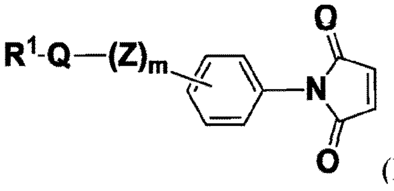

(IIIa)

und pharmazeutisch annehmbare Salze davon, wobei $R^1$, Q, Z und m den obigen Begriffsbestimmungen für Verbindungen der Formel (I) entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, -COOR$^3$, - COR$^3$, -CN, -$CF_3$, -$NO_2$, -$SO_2$, -$SO_3$, -NR$^4$R$^5$, -$PO_4$- und - $(OCH_2CH_2)_q$-OR$^3$ umfasst.

**16.** Verfahren nach Anspruch 1 oder 4, wobei die Gruppe $R^1Q(Z)_m$- die para-Stellung einnimmt, wie es in der Verbindung der Formel **(IIIaa)** dargestellt ist:

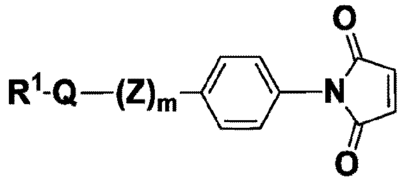

(IIIaa)

und pharmazeutisch annehmbare Salze davon, wobei R$^1$, Q, Z und m den obigen Begriffsbestimmungen für Verbindungen der Formel (I) entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, -COOR$^3$, - COR$^3$, -CN, -$CF_3$, -$NO_2$, -$SO_2$, -$SO_3$, -NR$^4$R$^5$, -$PO_4$ und - $(OCH_2CH_2)_q$-OR$^3$ umfasst.

**17.** Verfahren nach Anspruch 1 oder 4, wobei die Gruppe $R^1Q(Z)_m$- die meta-Stellung einnimmt, wie es in der Verbindung der Formel **(IIIaa')** dargestellt ist:

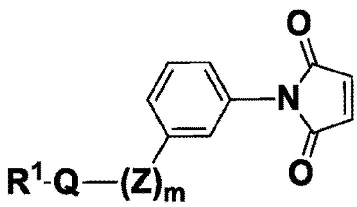

(IIIaa')

und pharmazeutisch annehmbare Salze davon, wobei R$^1$, Q, Z und m den obigen Begriffsbestimmungen für Verbindungen der Formel (I) entsprechen und das Phenyl mit 0, 1, 2, 3 oder 4 Substituenten versehen ist, die unabhängig voneinander aus der Gruppe ausgewählt sind, die Halogen, Hydroxyl, $C_{1-6}$-Alkyl, $C_{1-6}$-Alkoxy, -COOR$^3$, - COR$^3$, -CN, -$CF_3$, -$NO_2$, -$SO_2$, -$SO_3$, -NR$^4$R$^5$, -$PO_4$ und - $(OCH_2CH_2)_q$-OR$^3$ umfasst.

**18.** Verfahren nach Anspruch 1, 3 oder 14, wobei das Maleimid-Molekül in einer Verbindung der Formel **(IIIaaa)** vorliegt:

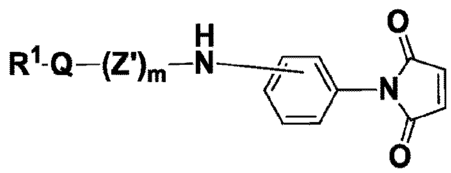

(IIIaaa)

und pharmazeutisch annehmbare Salze davon, wobei R$^1$, Q und m den obigen Begriffsbestimmungen für Verbindungen der Formel **(I)** entsprechen,
wobei das Phenyl mit 0, 1, 2, 3 oder 4 Substituenten versehen ist, die auf unabhängige Weise aus der Gruppe ausgewählt sind, die Halogen, Hydroxyl, $C_{1-6}$-Alkyl, $C_{1-6}$-Alkoxy, -COOR$^3$, -COR$^3$, -CN, -$CF_3$, -$NO_2$, -$SO_2$, -$SO_3$, -NR$^4$R$^5$, -$PO_4$ und -$(OCH_2CH_2)_q$-OR$^3$ umfasst, und
Z' für eine gesättigte oder ungesättigte verzweigte oder unverzweigte $C_{1-24}$-Alkylenkette steht, wobei ein oder mehrere Kohlenstoffe möglicherweise unabhängig voneinander durch -O-, N ersetzt sind und die Kette möglicherweise mit einem oder mehreren C(O)-Substituenten versehen ist.

**19.** Verfahren nach einem beliebigen der Ansprüche 1 bis 18, wobei Z oder Z' für -$C_{1-12}$-Alkylen- oder -$(CH_2CH_2O)_{1-8}$-stehen.

**20.** Verfahren nach einem beliebigen der Ansprüche 1 bis 19, wobei R$^1$ aus der Gruppe ausgewählt ist, die ein Toxin,

ein Arzneistoffmolekül (wie etwa ein zytotoxisches Mittel), ein Polymer, einen Antikörper oder ein Bindungsfragment eines solchen umfasst.

**21.** Verfahren nach Anspruch 20, wobei das Arzneistoffmolekül aus derjenigen ausgewählt ist, die ein Auristatin umfasst, wobei es beispielsweise aus der Gruppe ausgewählt ist, die ein Tubulysin, ein Pyrrolobenzodiazepin (PBD), MMAE (Monomethylauristatin E) und MMAE (Monomethylauristatin F) umfasst.

## Revendications

**1.** Procédé pour la préparation d'un anticorps conjugué à une charge utile comprenant l'étape de :

a) réalisation d'une réaction d'addition de Michael avec le motif de maléimide dans la molécule de formule (I) :

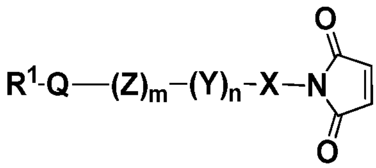

(I)

et une molécule d'anticorps comprenant au moins un groupe thiol :

n étant 0 ou 1 ;
m étant 0 ou 1 ;
q étant 0 ou un entier dans la plage de 1 à 12, par exemple 1 à 6, tel que 2 ou 3 ;
Q étant une liaison ou un radical d'un composant de conjugaison ;
X étant $C_{0-18}$ alkylène$C_{6-36}$ aryle,
l'aryle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, - $COR^3$, -CN, $-CF_3$, $-NO_2$, $-SO_2$, $-SO_3$, $-NR^4R^5$, $-PO_4$ et - $(OCH_2CH_2)_q-OR^3$ ;
Y étant C(O) ;
Z étant une chaîne $C_1$-$C_{30}$ alkylène saturée ou insaturée ramifiée ou non ramifiée, un ou plusieurs carbones (tel que 1, 2, 3, 4, 5, 6, 7 ou 8) étant éventuellement indépendamment remplacés par -O-, N et la chaîne portant éventuellement un ou plusieurs (tel que 1, 2, 3 ou 4) substituants C(O) ;
$R^1$ étant H, une surface solide ou une molécule de charge utile ;
$R^3$ étant H ou $C_{1-6}$ alkyle ;
$R^4$ étant H ou $C_{1-6}$ alkyle ;
$R^5$ étant H ou $C_{1-6}$ alkyle ;

b) $R^1$ étant une molécule de charge utile ou une surface solide hydrolysant le motif de thio-succinimide résultant formé par la réaction du composé de formule (I) et de l'anticorps, ou
c) $R^1$ étant H réalisant une conjugaison avec une charge utile, un composant de conjugaison ou une surface solide, suivie par l'hydrolyse du motif de thio-succinimide formé par la réaction du composé de formule (I) et de l'anticorps.

**2.** Procédé selon la revendication 1, X étant $C_{0-15}$ alkylène$C_{6-10}$ aryle.

**3.** Procédé selon la revendication 1 ou 2, n étant 1 de formule (II) :

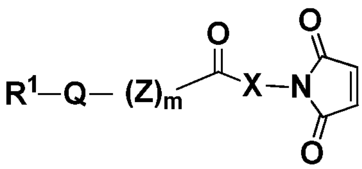

(II)

et des sels pharmaceutiquement acceptables correspondants, $R^1$, Q, Z, X et m étant définis ci-dessus pour des

composés de formule (I).

**4.** Procédé selon la revendication 1 ou 2, n étant 0 de formule (III) :

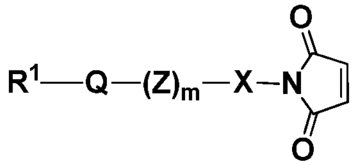
(III)

et des sels pharmaceutiquement acceptables correspondants, $R^1$, Q, Z, X et m étant définis ci-dessus pour des composés de formule (I).

**5.** Procédé selon la revendication 1 ou 3, la molécule de maléimide étant dans un composé de formule (IIa) :

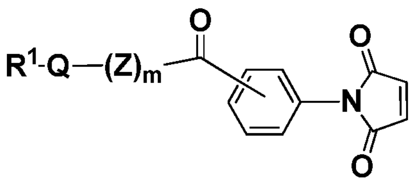
(IIa)

et des sels pharmaceutiquement acceptables correspondants, $R^1$, Q, Z et m étant définis ci-dessus pour des composés de formule (I), et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et - $(OCH_2CH_2)_q-OR^3$.

**6.** Procédé selon la revendication 5, le groupe $R^1Q(Z)_mC(O)$- étant dans la position para comme présenté dans le composé de formule (IIaa) :

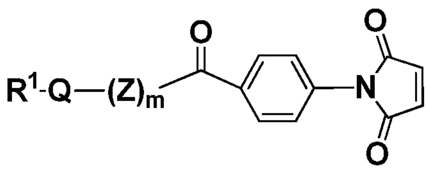
(IIaa)

et des sels pharmaceutiquement acceptables correspondants, $R^1$, Q, Z et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et - $(OCH_2CH_2)_q-OR^3$.

**7.** Procédé selon la revendication 5, le groupe $R^1Q(Z)_mC(O)$- étant dans la position méta comme présenté dans le composé de formule (IIaa') :

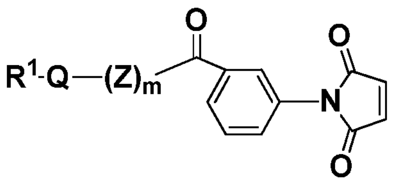
(IIaa')

et des sels pharmaceutiquement acceptables correspondants, $R^1$, Q, Z et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et - $(OCH_2CH_2)_q-OR^3$.

8. Procédé selon la revendication 1, 3 ou 5, la molécule de maléimide étant dans un composé de formule (IIaaa) :

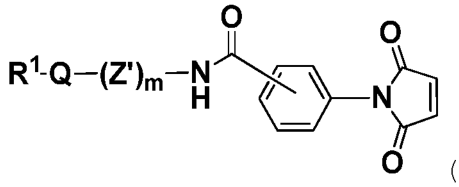

(IIaaa)

et des sels pharmaceutiquement acceptables correspondants, $R^1$, Q et m étant définis ci-dessus pour des composés de formule (I),
le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $- COR^3$, -CN, $-CF_3$, $-NO_2$, $-SO_2$, $-SO_3$, $-NR^4R^5$, $-PO_4$ et $-(OCH_2CH_2)_q-OR^3$, et
Z' étant une chaîne $C_{1-24}$ alkylène saturée ou insaturée ramifiée ou non ramifiée, un ou plusieurs carbones étant éventuellement remplacés par -O-, N et la chaîne portant éventuellement un ou plusieurs substituants C(O).

9. Procédé selon la revendication 8, le groupe $R^1Q(Z')_mNC(O)$- étant dans la position méta ou para.

10. Procédé selon la revendication 1 ou 3, le motif de maléimide étant dans une molécule de formule (IIb) :

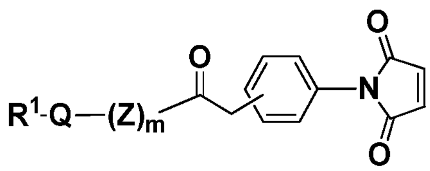

(IIb)

et des sels pharmaceutiquement acceptables correspondants, $R^1$, Q, Z et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et $- (OCH_2CH_2)_q-OR^3$.

11. Procédé selon la revendication 10, le groupe $R^1Q(Z)_mC(O)$- étant dans la position para comme présenté dans le composé de formule (IIbb) :

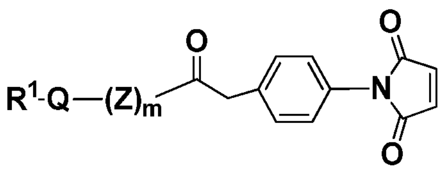

(IIbb)

et des sels pharmaceutiquement acceptables correspondants, n, Q, Z, X, $R^1$ et m étant définis ci-dessus dans la revendication 1.

12. Procédé selon la revendication 10, le groupe $R^1Q(Z)_mC(O)$- étant dans la position méta comme présenté dans le composé de formule (IIbb') :

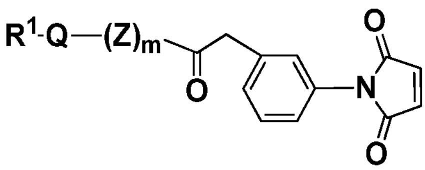

(IIbb')

et des sels pharmaceutiquement acceptables correspondants, Q, Z, X, $R^1$ et m étant définis ci-dessus dans la

revendication 1.

**13.** Procédé selon la revendication 1, 3 ou 10, le motif de maléimide étant dans une molécule de formule (IIbbb) :

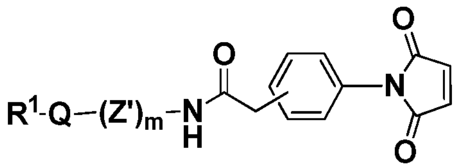
(IIbbb)

$R^1$, Q et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et - $(OCH_2CH_2)_q-OR^3$
Z' étant une chaîne $C_{1-24}$ alkylène saturée ou insaturée ramifiée ou non ramifiée, un ou plusieurs carbones étant éventuellement remplacés par -O-, N et la chaîne portant éventuellement un ou plusieurs substituants C(O).

**14.** Procédé selon la revendication 13, le groupe $R^1Q(Z')_mNC(O)$ - étant dans la position méta ou para du cycle phényle.

**15.** Procédé selon la revendication 1 ou 4, la molécule de maléimide étant dans un composé de formule (IIIa) :

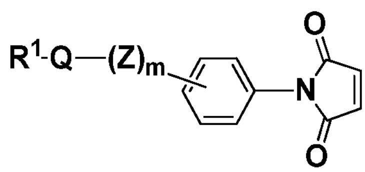
(IIIa)

et des sels pharmaceutiquement acceptables correspondants, $R^1$, Q, Z et m étant définis ci-dessus pour des composés de formule (I), et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$- et $-(OCH_2CH_2)_q-OR^3$.

**16.** Procédé selon la revendication 1 ou 4, le groupe $R^1Q(Z)_m$- étant dans la position para comme présenté dans le composé de formule (IIIaa) :

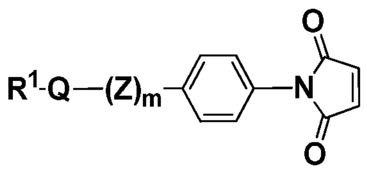
(IIIaa)

et des sels pharmaceutiquement acceptables correspondants, $R^1$, Q, Z et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et $-(OCH_2CH_2)_q-OR^3$.

**17.** Procédé selon la revendication 1 ou 4, le groupe $R^1Q(Z)_m$- étant dans la position méta comme présenté dans le composé de formule (IIIaa') :

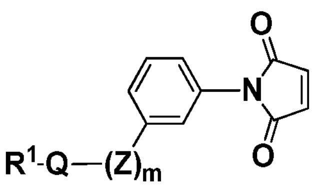
(IIIaa')

et des sels pharmaceutiquement acceptables correspondants, $R^1$, Q, Z et m étant définis ci-dessus pour des composés de formule (I) et le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, $-COR^3$, -CN, $-CF_3$, $-NO_2$, $-SO_2$, $-SO_3$, $-NR^4R^5$, $-PO_4$ et $-(OCH_2CH_2)_q-OR^3$.

**18.** Procédé selon la revendication 1, 3 ou 14, la molécule de maléimide étant dans un composé de formule (IIIaaa) :

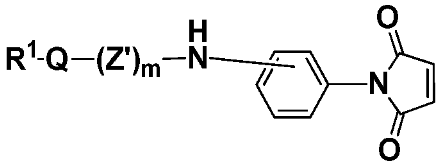

(IIIaaa)

et des sels pharmaceutiquement acceptables correspondants, $R^1$, Q et m étant définis ci-dessus pour des composés de formule (I),
le phényle ayant 0, 1, 2, 3 ou 4 substituants indépendamment choisis dans le groupe comprenant halogène, hydroxyle, $C_{1-6}$ alkyle, $C_{1-6}$ alcoxy, $-COOR^3$, - $COR^3$, -CN, $-CF_3$, $-NO_2$, $-SO_2$, $-SO_3$, $-NR^4R^5$, $-PO_4$ et - $(OCH_2CH_2)_q-OR^3$, et
Z' étant une chaîne $C_{1-24}$ alkylène saturée ou insaturée ramifiée ou non ramifiée, un ou plusieurs carbones étant éventuellement indépendamment remplacés par -O-, N et la chaîne portant éventuellement un ou plusieurs substituants C(O).

**19.** Procédé selon l'une quelconque des revendications 1 à 18, Z ou Z' représentant $-C_{1-12}$ alkylène- ou $-(CH_2CH_2O)_1-8^-$.

**20.** Procédé selon l'une quelconque des revendications 1 à 19, $R^1$ étant choisi dans le groupe comprenant une toxine, une molécule de médicament (tel qu'un agent cytotoxique), un polymère, un anticorps ou un fragment de liaison de celui-ci.

**21.** Procédé selon la revendication 20, la molécule de médicament étant choisie dans le groupe comprenant une auristatine, par exemple choisie dans le groupe comprenant une tubulysine, une pyrrolobenzodiazépine (PBD), MMAE (monométhyl-auristatine E) et MMAF (monométhyl-auristatine F).

**FIGURE 1 A schematic representation of hydrolysis of compounds of the disclosure**

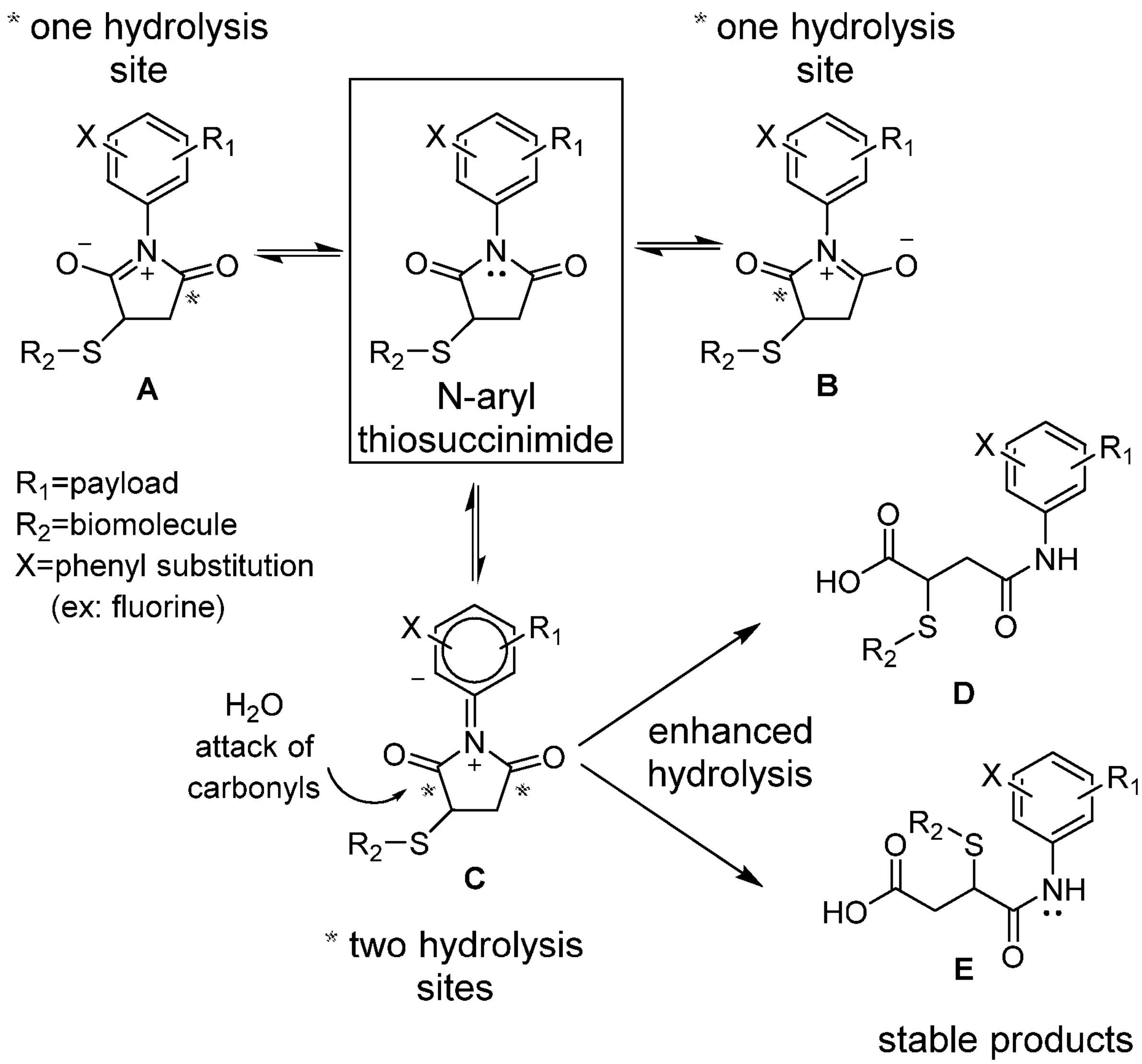

**Figure 1A Spectra of Unreacted mAb**

**Full Spectrum**

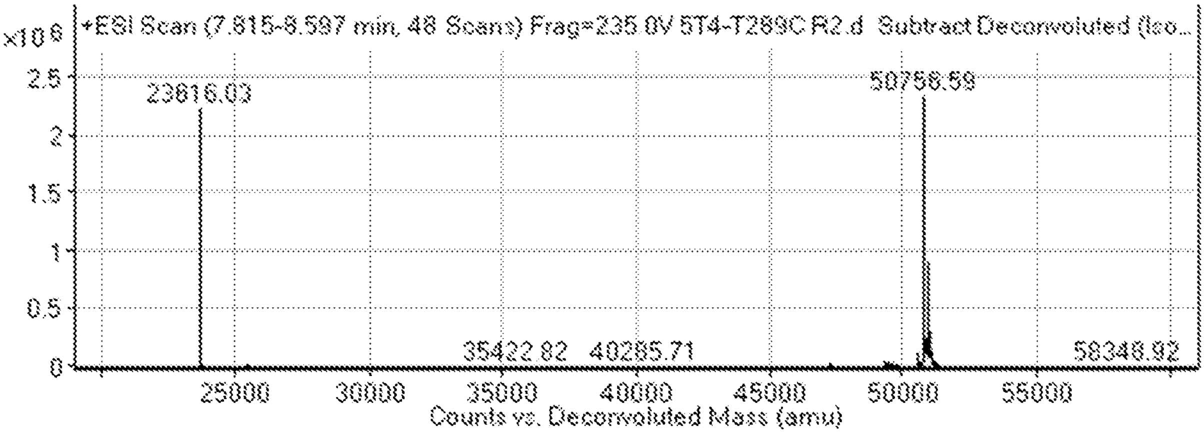


**Heavy Chain Zoom**

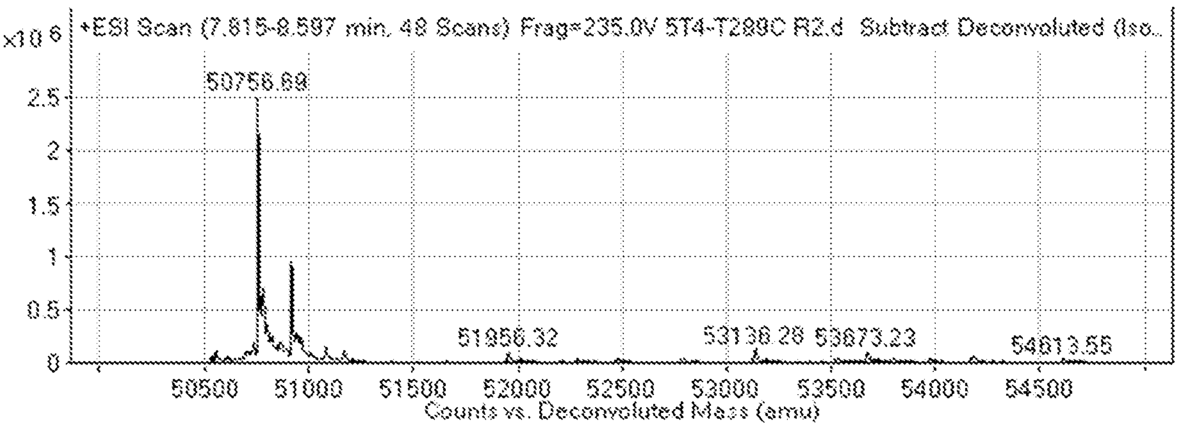

**Figure 1B Spectra of Alkyl maleimide-PEG-biotin conjugate**

**Full Spectrum**

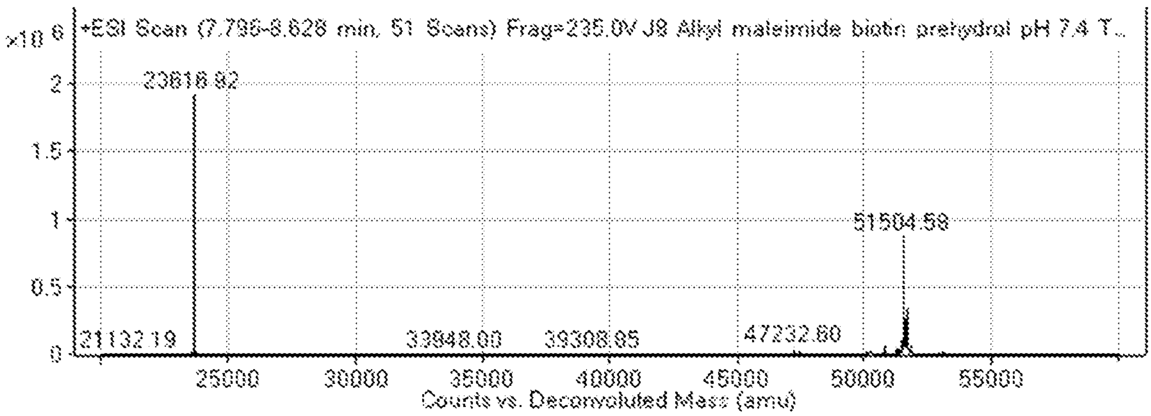


**Heavy Chain Zoom**

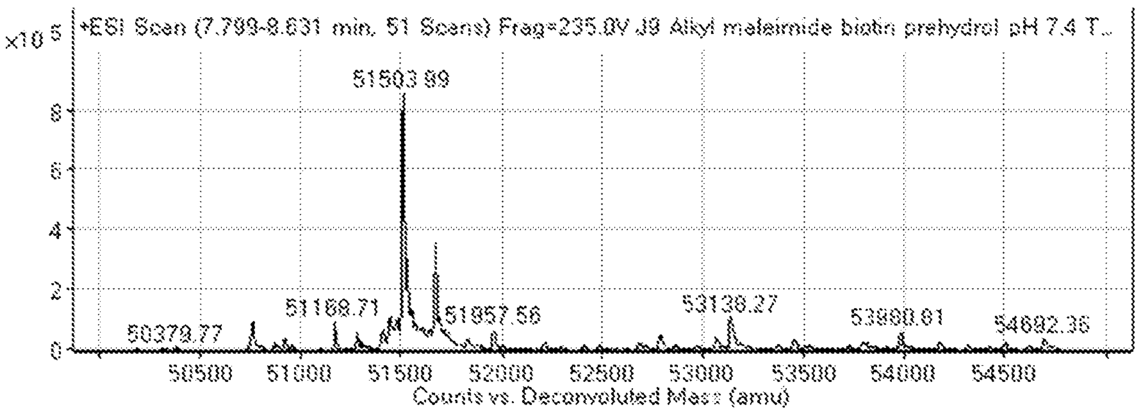

**Figure 2A** **Spectrum of Alkyl maleimide-PEG-biotin (comparator)**

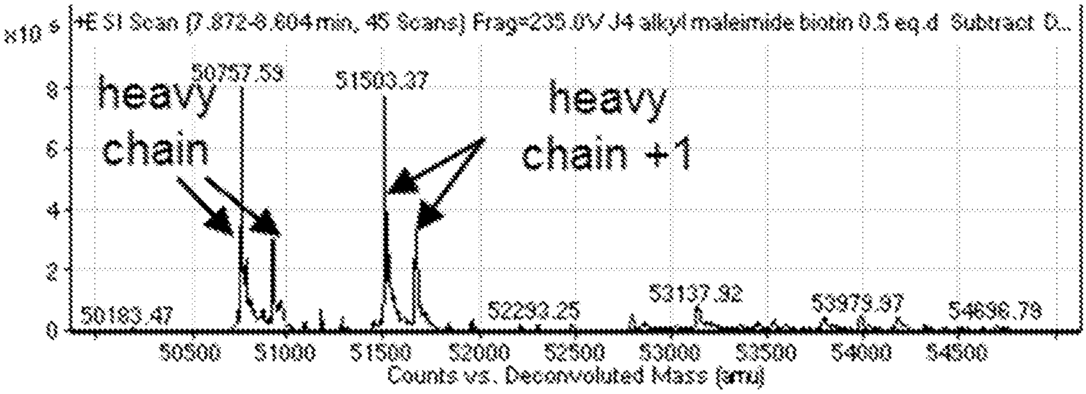


**Figure 2B** **Spectrum of Phenyl maleimide-PEG-biotin**

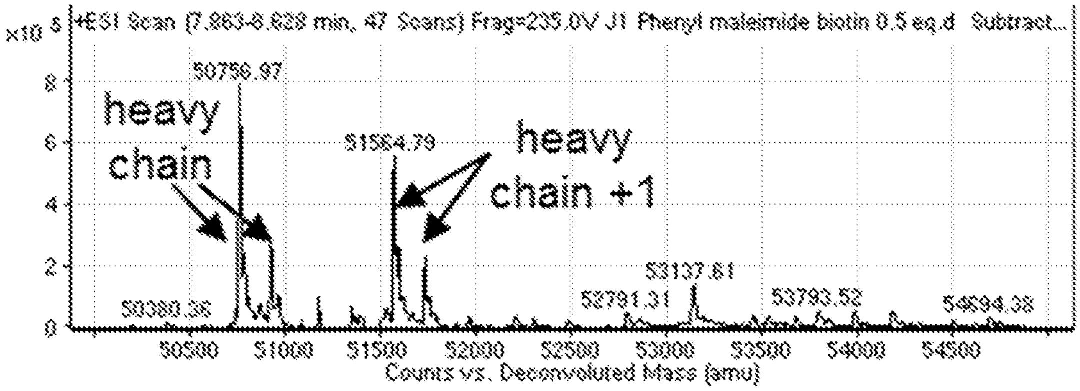


**Figure 2C** **Spectrum of Fluorophenyl maleimide-PEG-biotin**

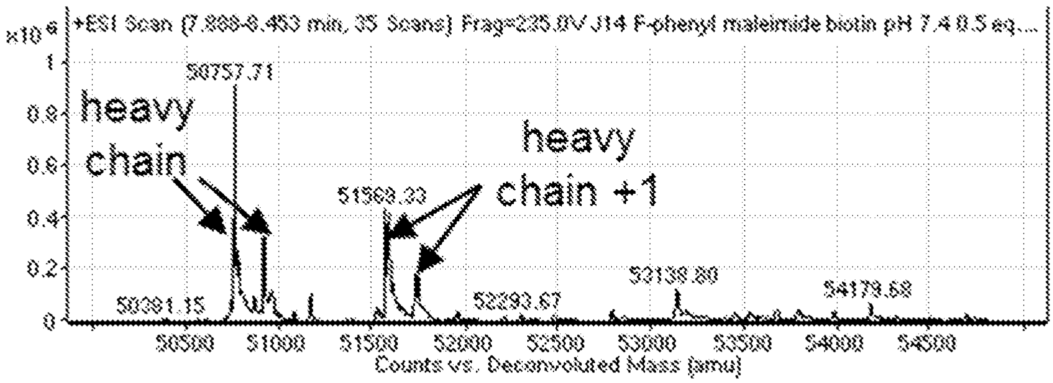

**Figure 3 Conjugation Efficiency of x-Maleimide-PEG-biotins to T289C mAb at 22 oC**

A

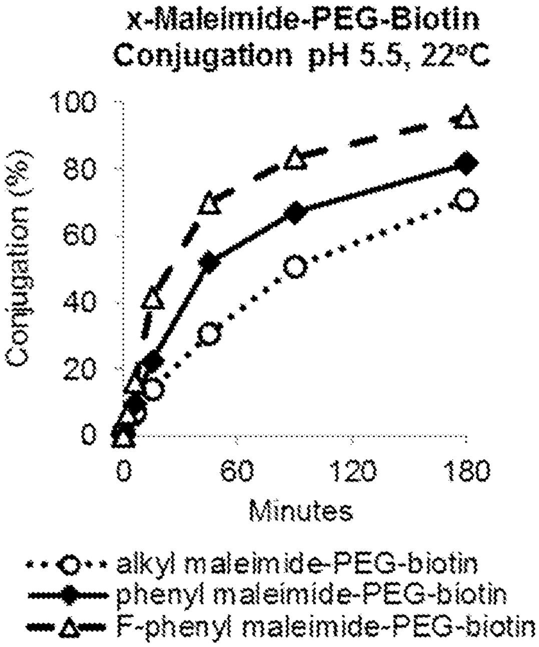


B

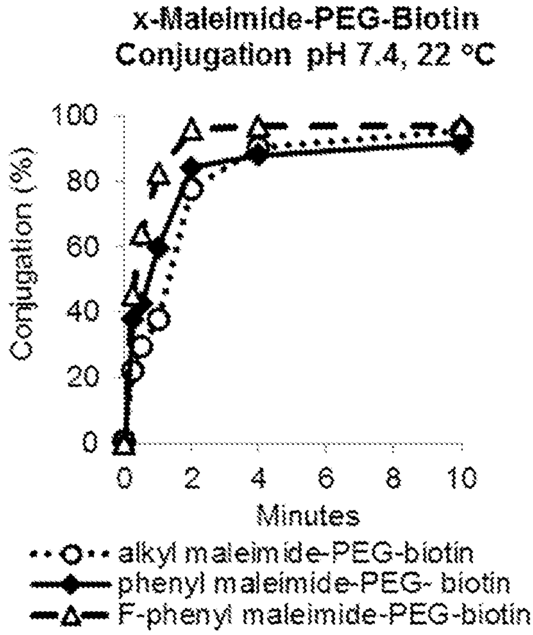


C

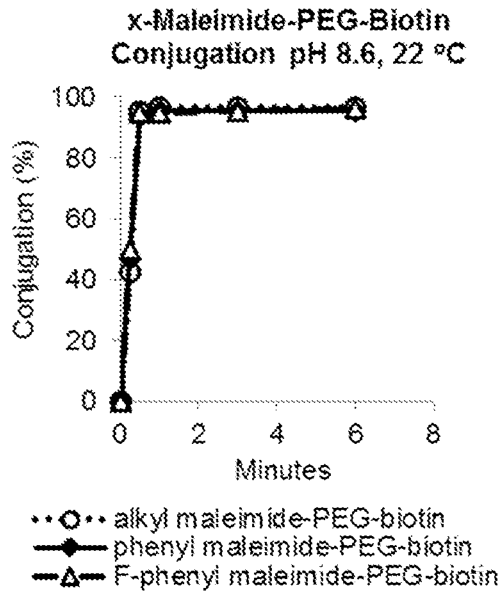

**Figure 4 Conjugation Efficiency of x-Maleimide-PEG-biotins to T289C mAb at 22 oC**

A

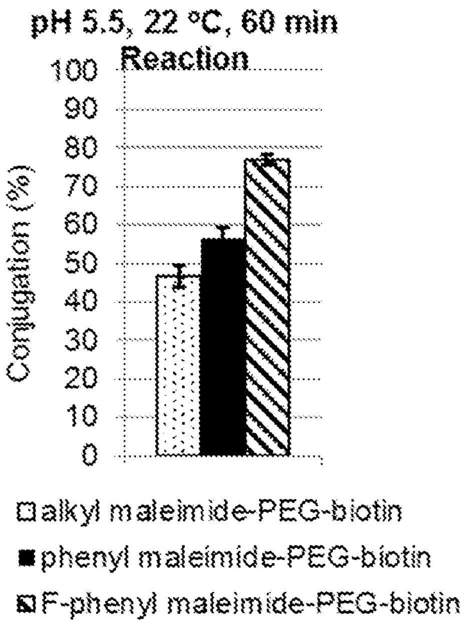


B

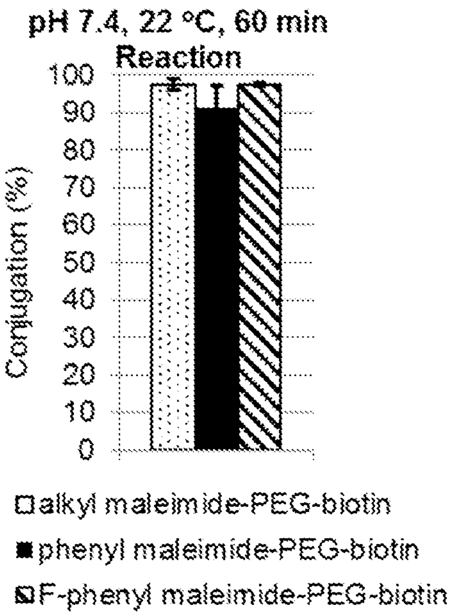


C

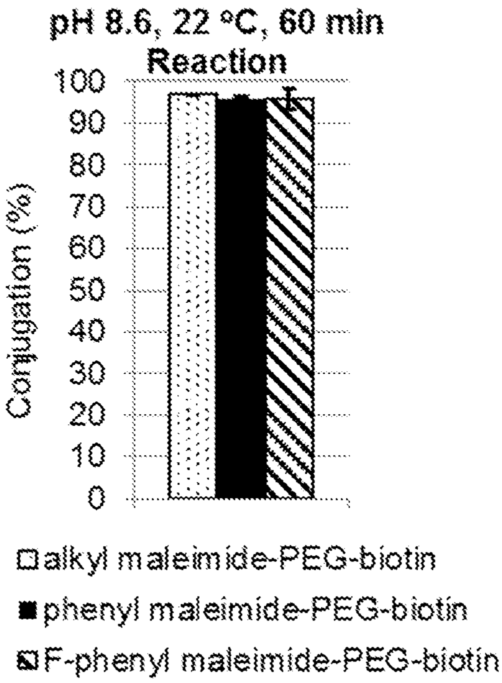

**Figure 5** **Conjugation Kinetics of x-Maleimide-PEG-biotins to T289C mAb, pH 5.5, at 22 oC**

**A**

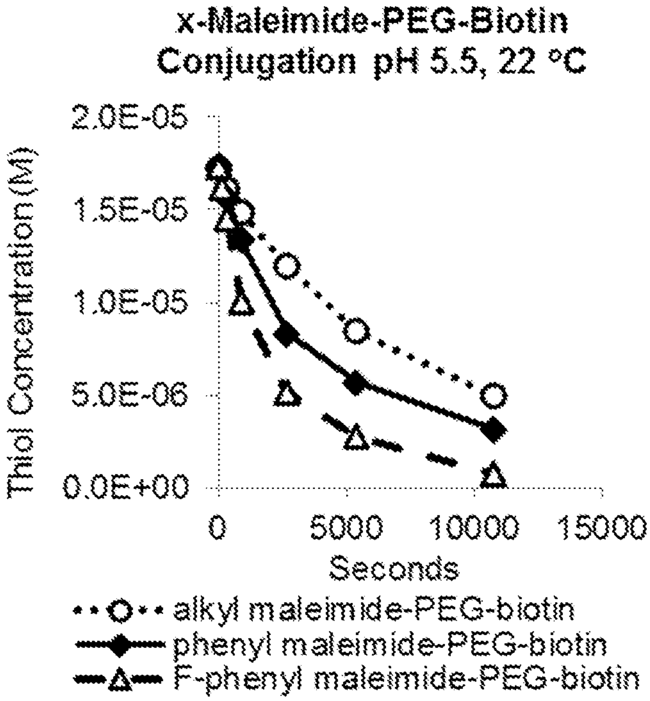


**B**

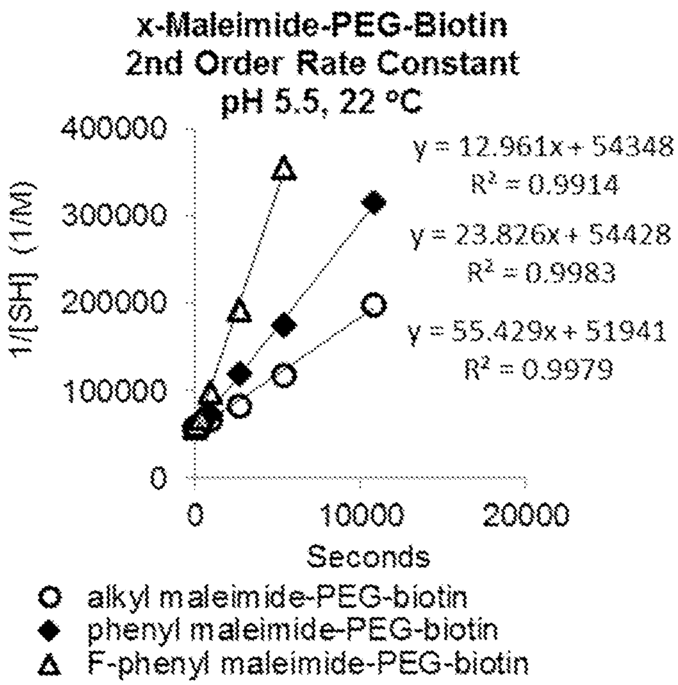


***All trendlines listed in the same order as legend***

**Figure 6** **Conjugation Kinetics of x-Maleimide-PEG-biotins to T289C mAb, pH 7.4, at 22◦C**

**A**

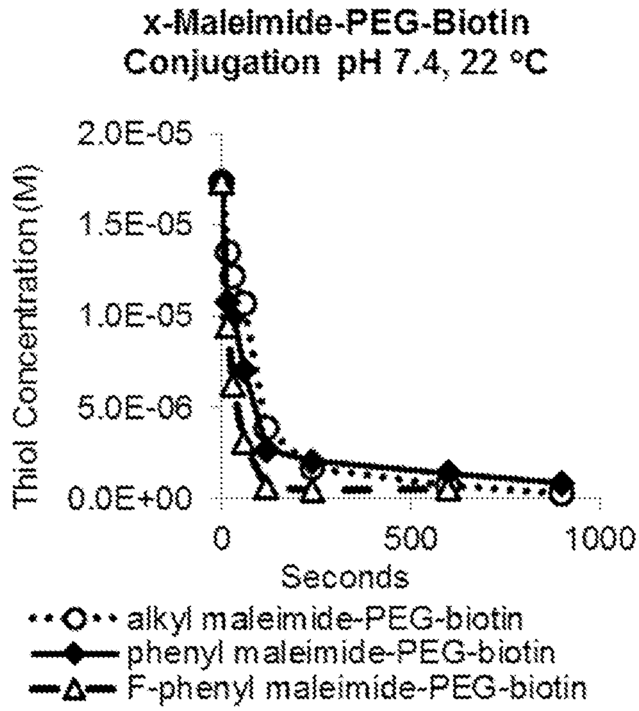


**B**

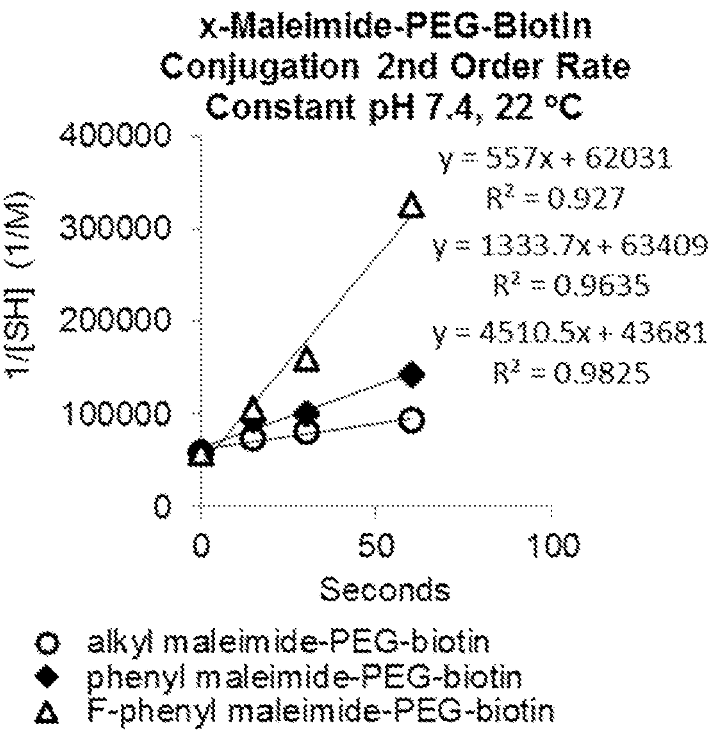


***All trendlines listed in the same order as legend***

**Figure 7** **Conjugation Kinetics of x-Maleimide-PEG-biotins to T289C mAb, pH 8.6, at 22◦C**

**A**

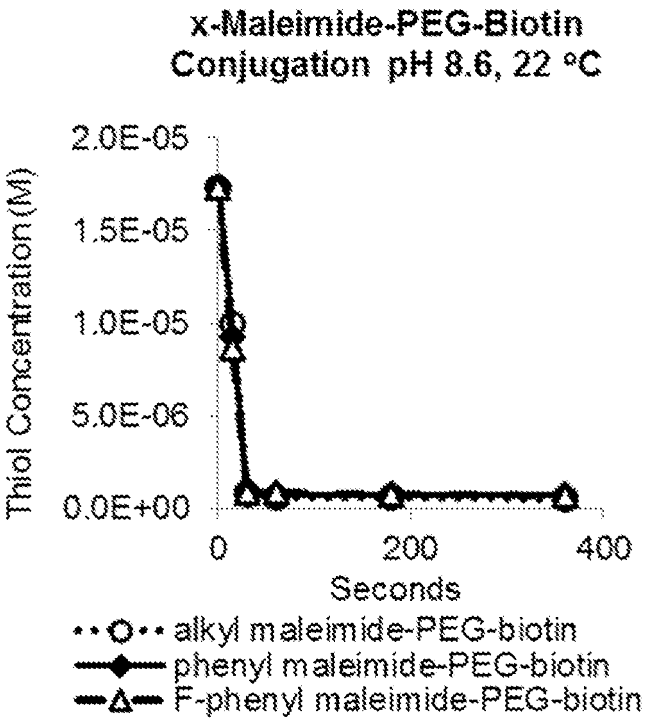


**B**

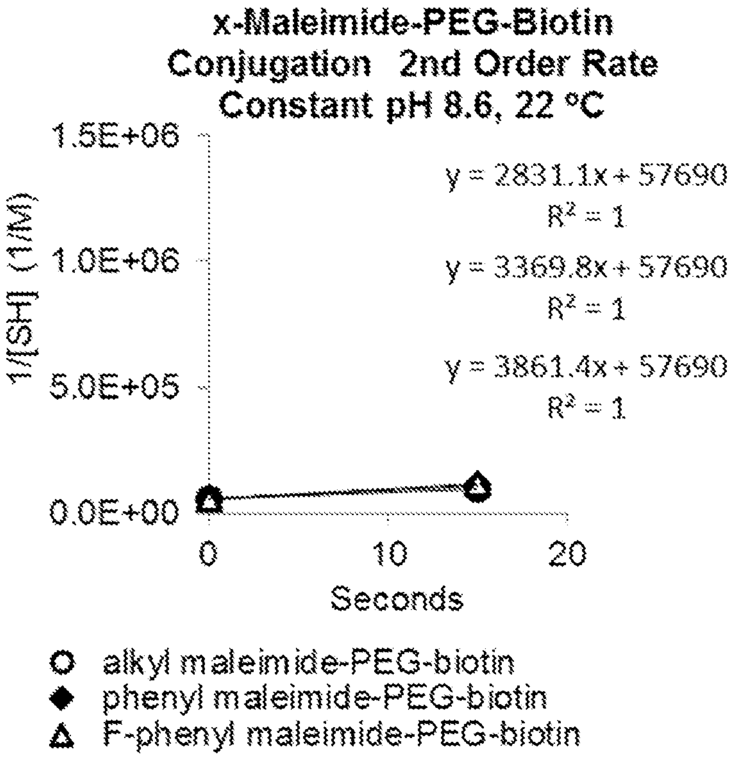


*All trendlines listed in the same order as legend*

**Figure 8** **Reaction Kinetics of x-Maleimide-PEG-Biotins with T289C mAb Following Pre-Incubation: Indirect Measurement of Maleimide Hydrolysis**

A

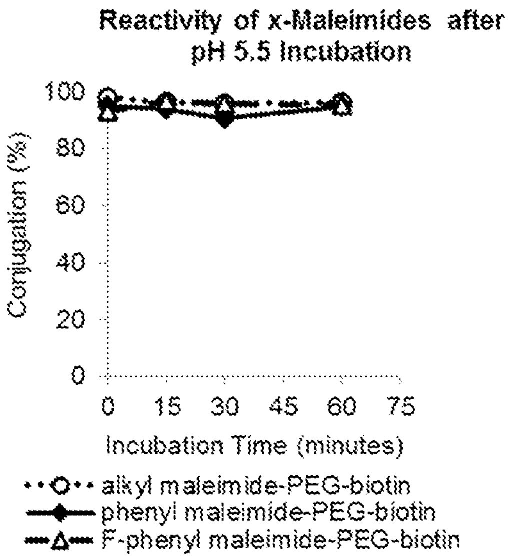


B

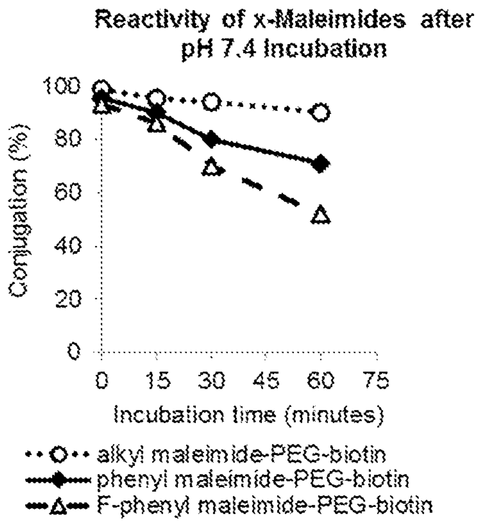


C

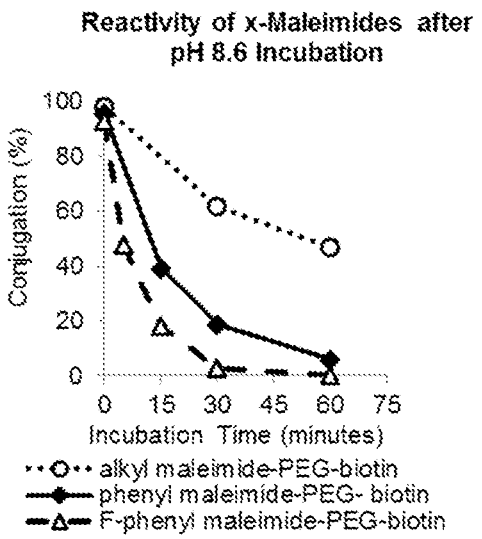

**Figure 9** **Maleimide Hydrolysis Kinetics pH 5.5, 22 oC**

A

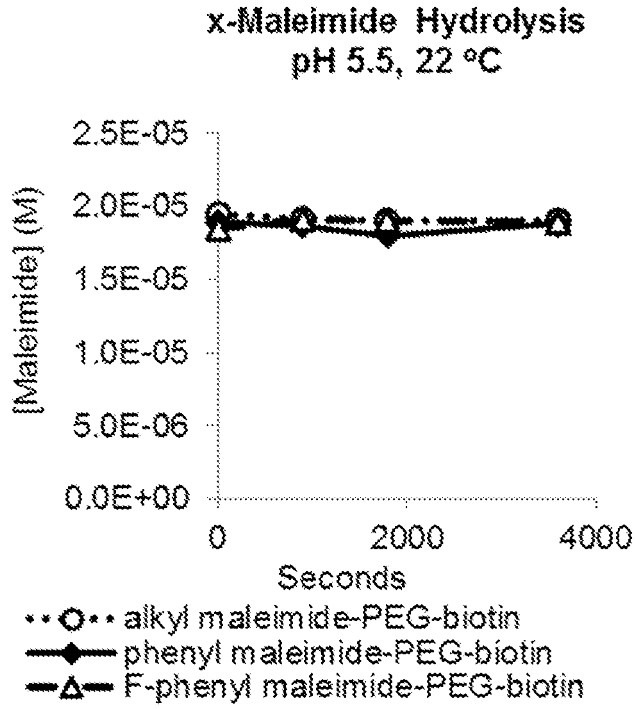


B

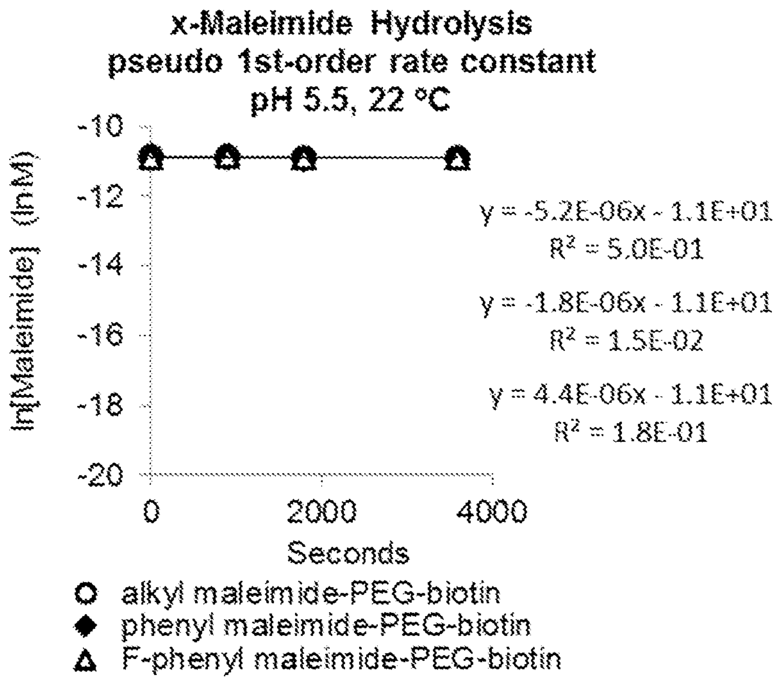


***All trendlines listed in the same order as legend***

**Figure 10 Maleimide Hydrolysis Kinetics pH 7.4, 22 oC**

**A**

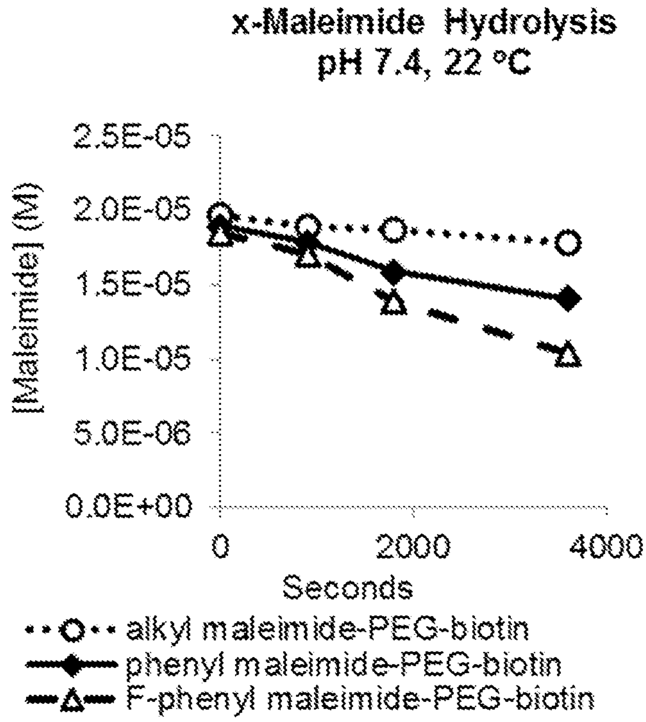


**B**

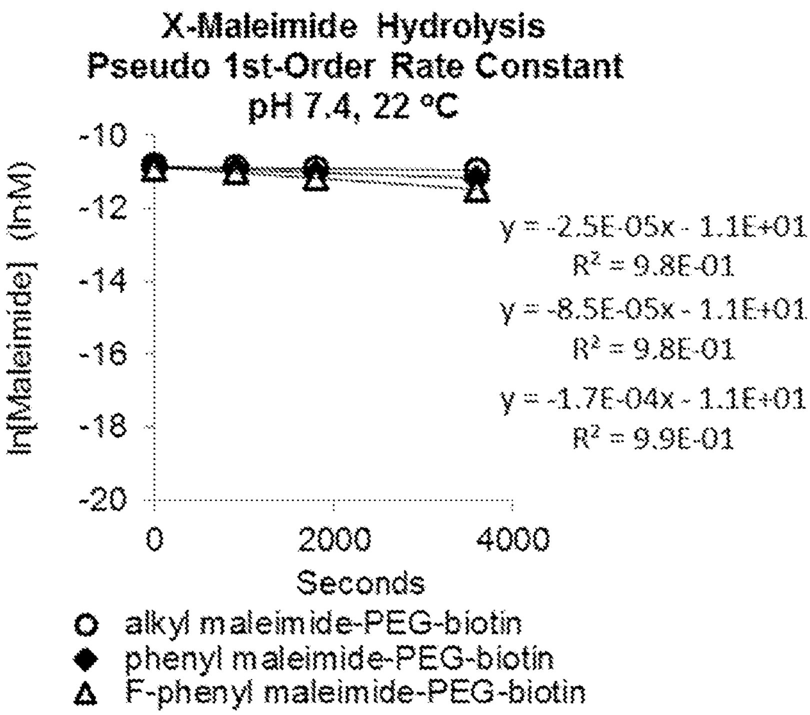


***All trendlines listed in the same order as legend***

**Figure 11** **Maleimide Hydrolysis Kinetics pH 8.6, 22 oC**

**A**

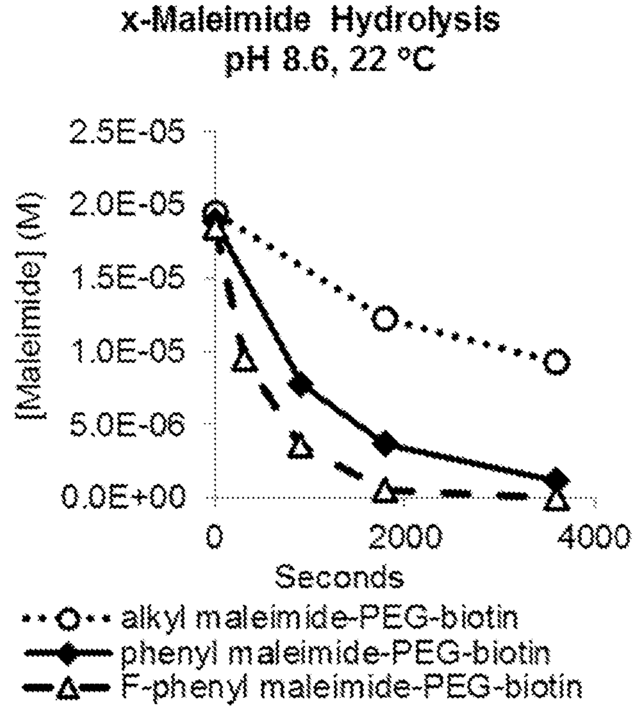


**B**

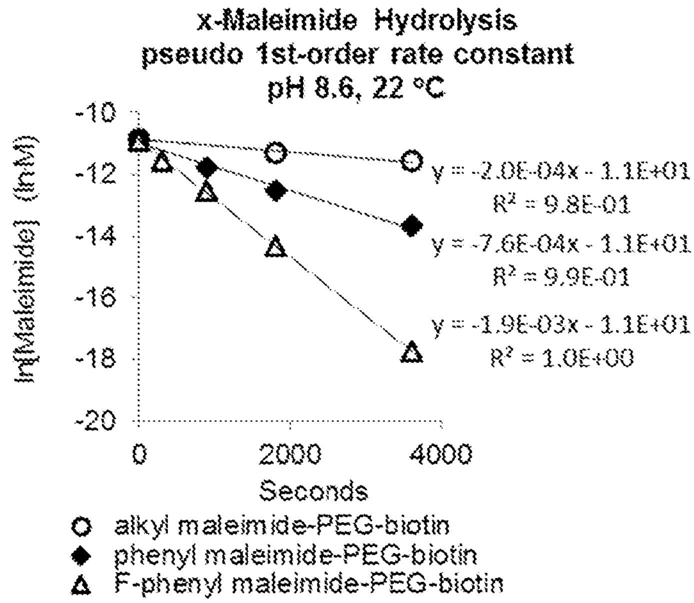

**Figure 12** **Thiosuccinimide Hydrolysis pH 7.4, 22 oC, for PEG-biotin T289C mAb Conjugates**

**A**

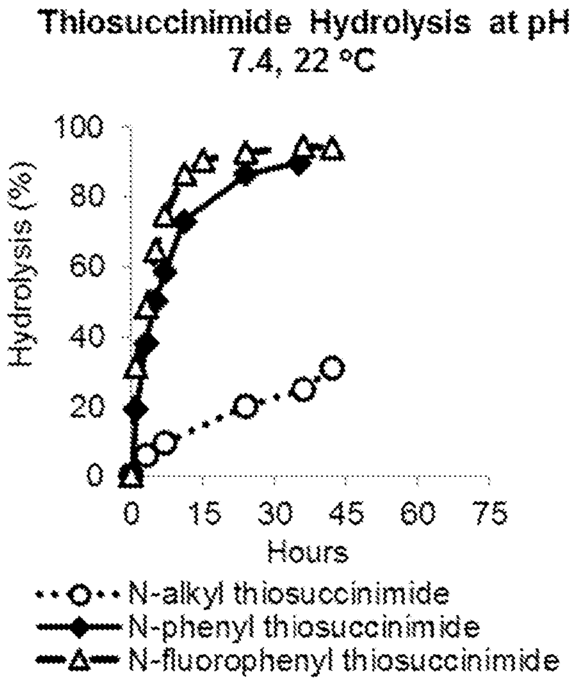


**B**

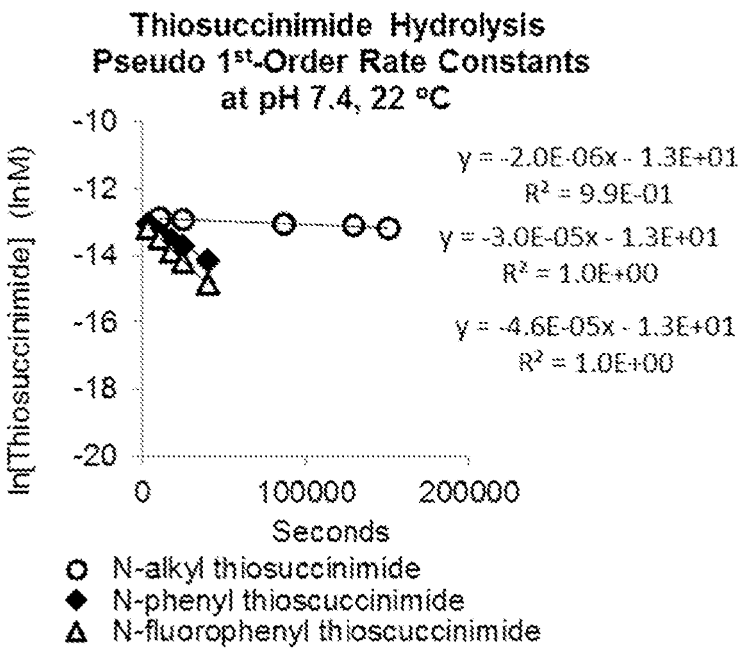


*All trendlines listed in the same order as legend*

**Figure 13** **Thiosuccinimide Hydrolysis pH 7.4, 37 oC, for PEG-biotin T289C mAb**

A

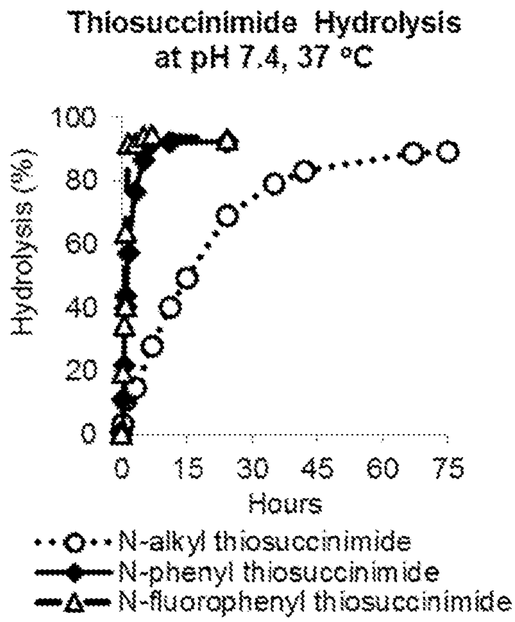


B

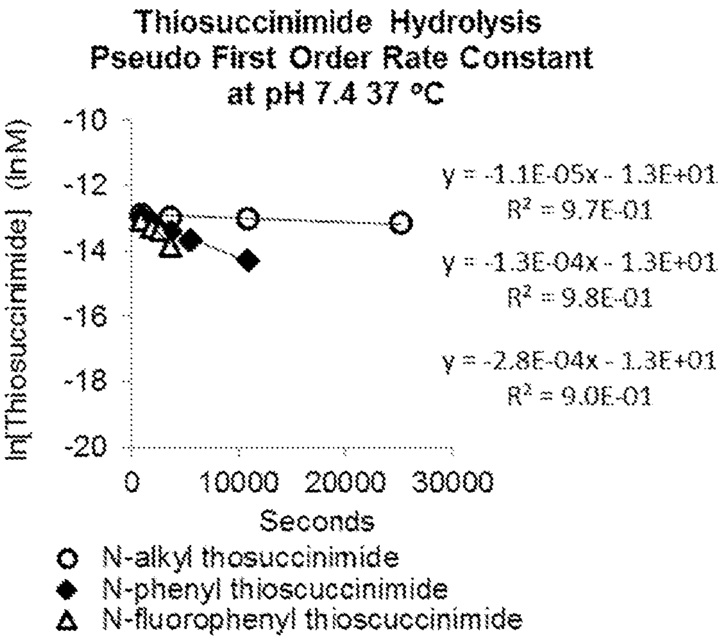

**Figure 14 Thiosuccinimide Hydrolysis pH 8.6, 22 oC, for PEG-biotin T289C mAb**

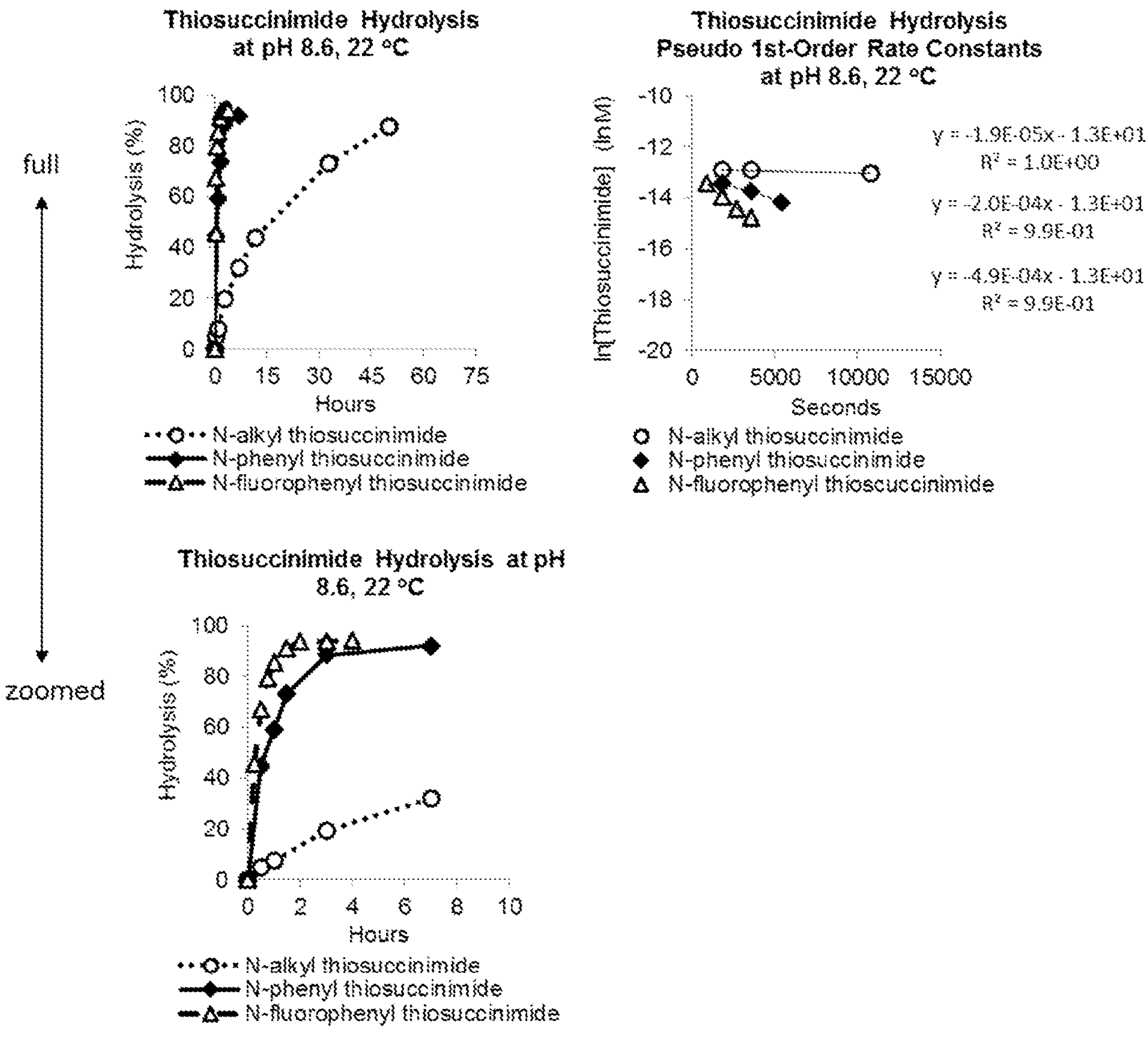

**Figure 15** **x-Thiosuccinimide Hydrolysis for PEG-biotin T289C-mAb Conjugates After 1 Hour Incubation, n=3**

**A**

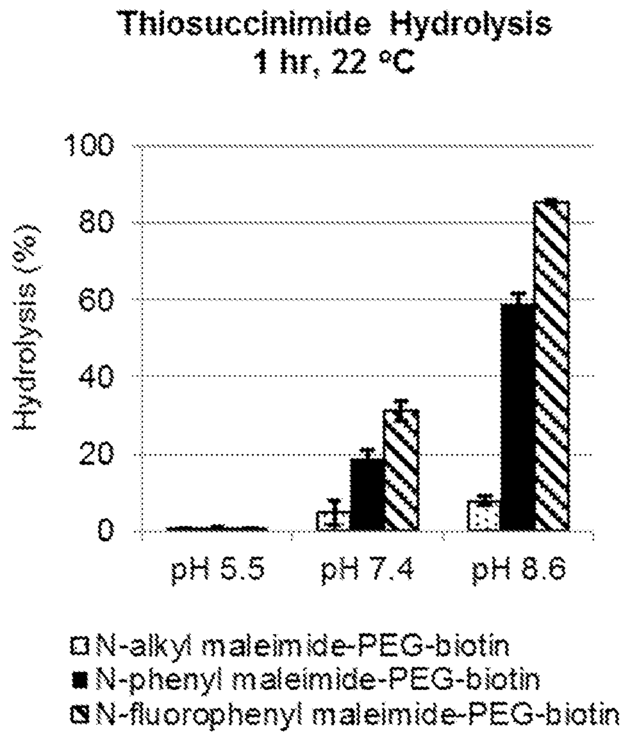


**B**

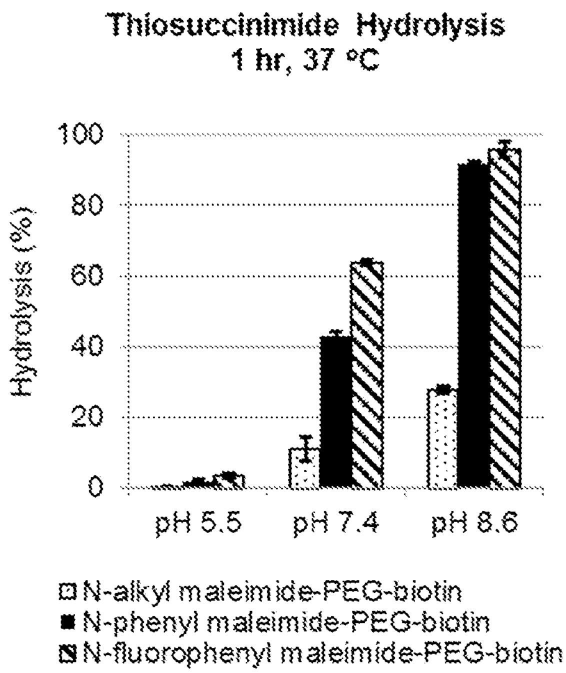

**Figure 16** **x-Thiosuccinimide-PEG-Biotin Hydrolysis for PEG-Biotin T289C mAb Conjugates in the Presence of Sodium Molybdate**

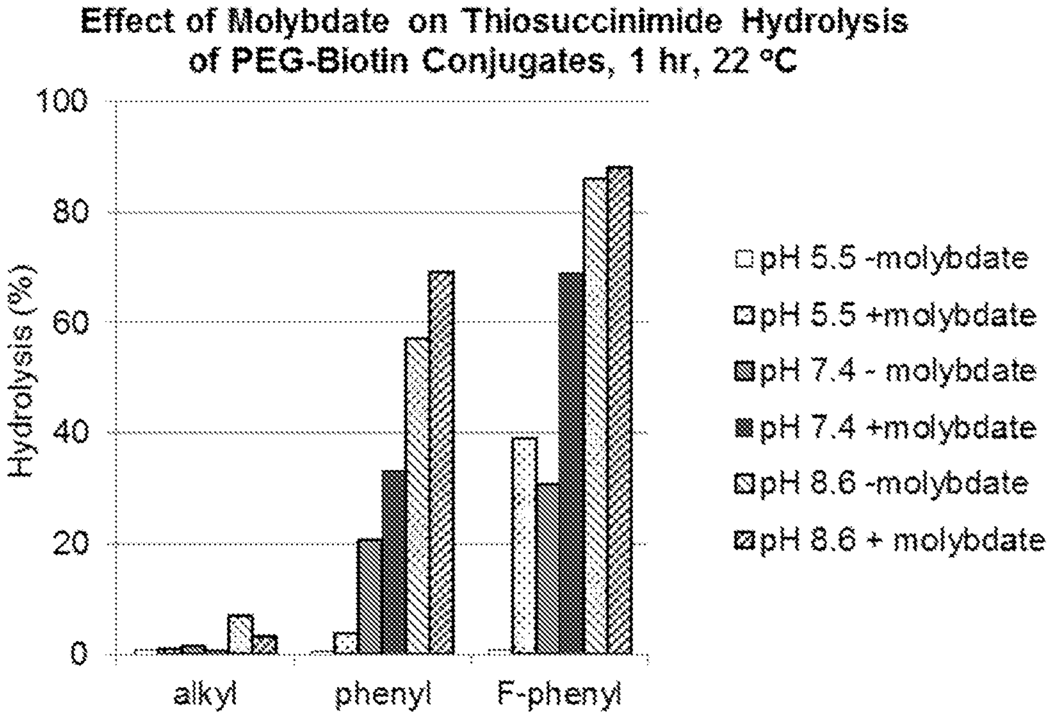

**Figure 17** **Sensitivity of T289C mAb Conjugates to Thiol Exchange in Buffer Containing Thiols**

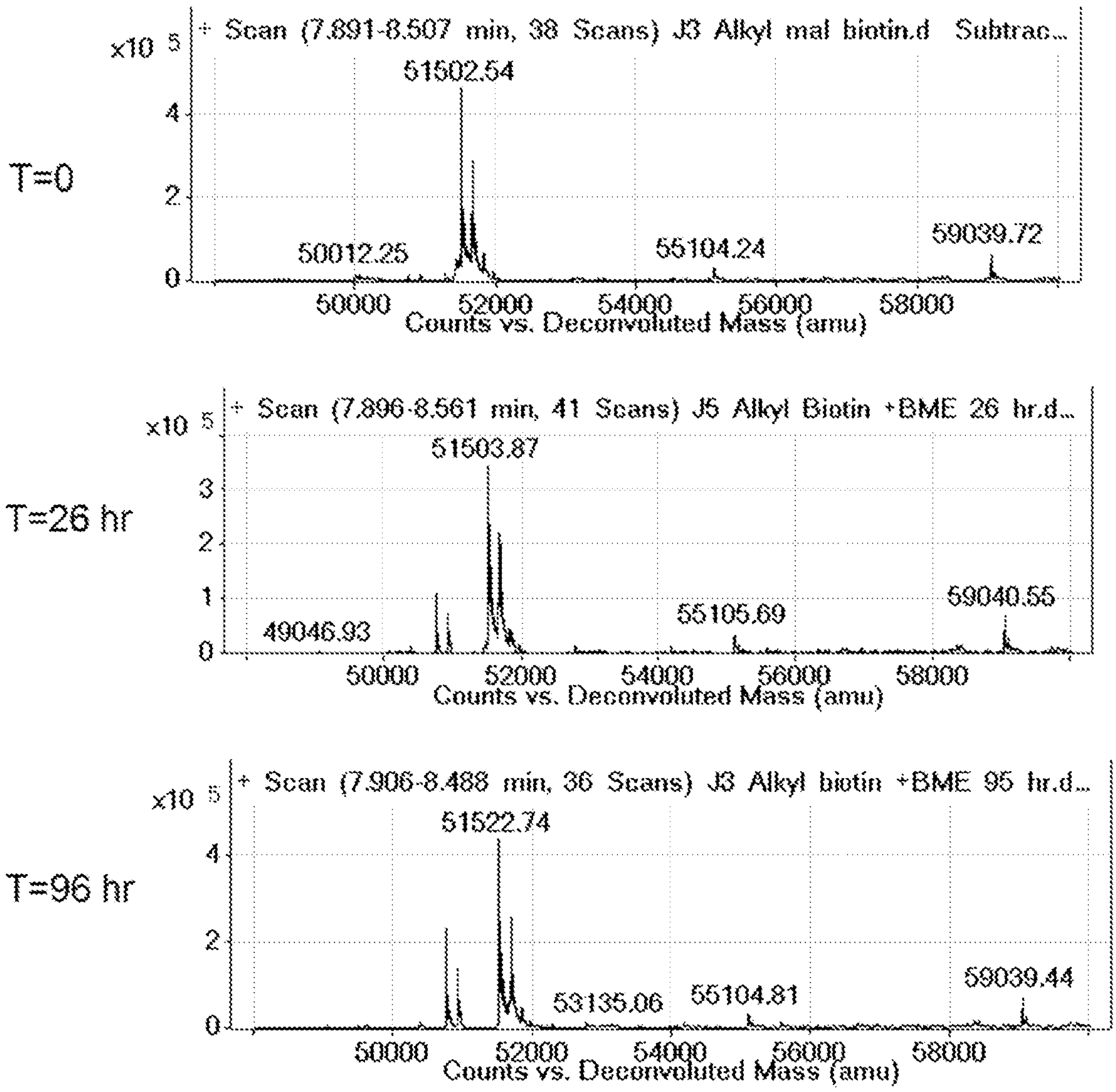


Representative mass spectometry data: BME challenge of alkyl-maleimide-biotin-mAb conjugate

**Figure 18 Stability of PEG-Biotin T289C mAb Conjugates in Buffer Containing BME**

A

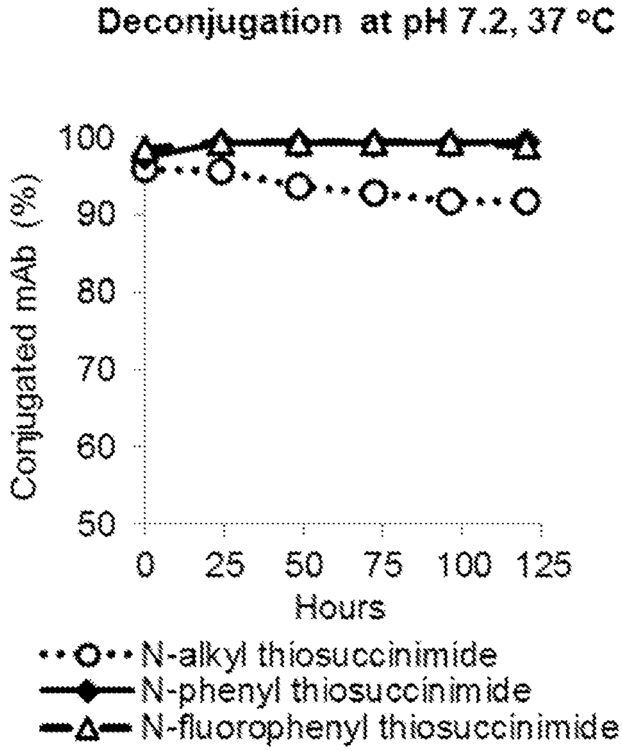


B

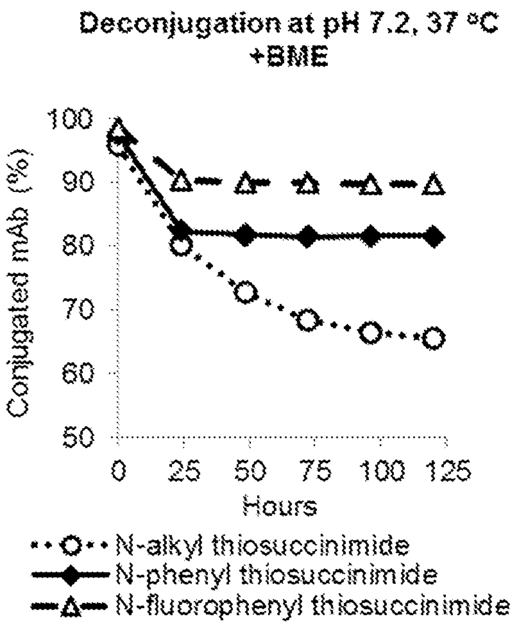

**Figure 19** **Thiosuccinimide Hydrolysis at pH 7.2, 37 oC for PEG-Biotin T289C mAb Conjugates Observed in BME Challenge Assay**

**A**

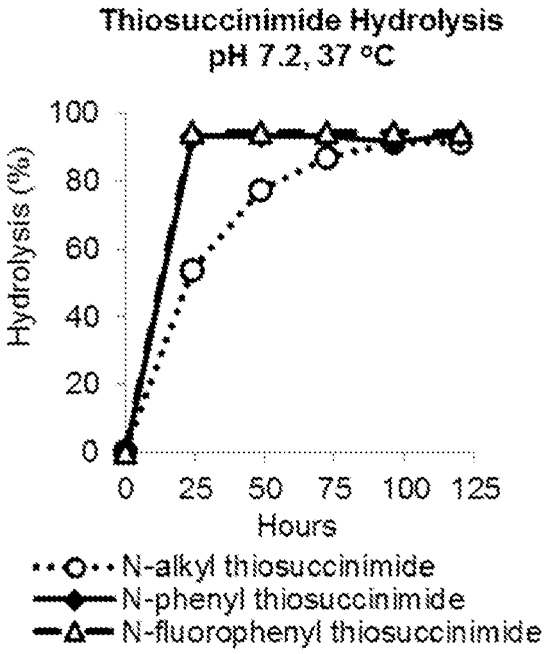


**B**

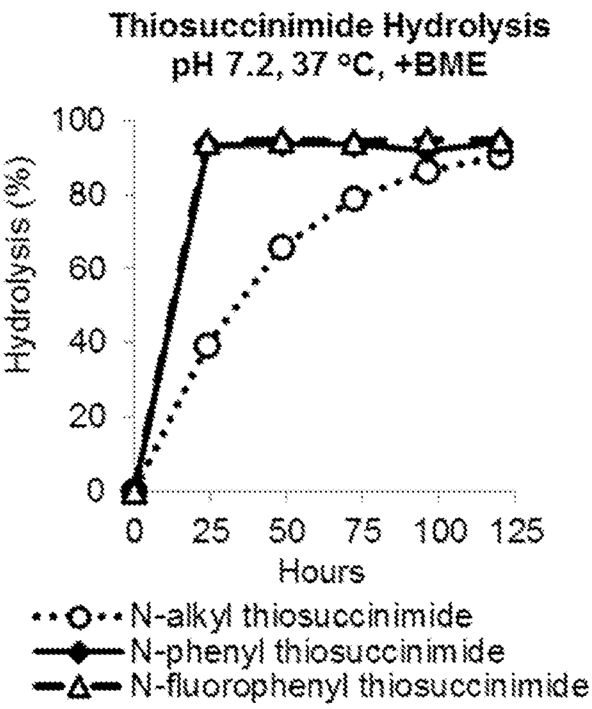

**Figure 20 Relationship Between Thiosuccinimide Deconjugation and Hydrolysis Observed in the BME Challenge Assay**

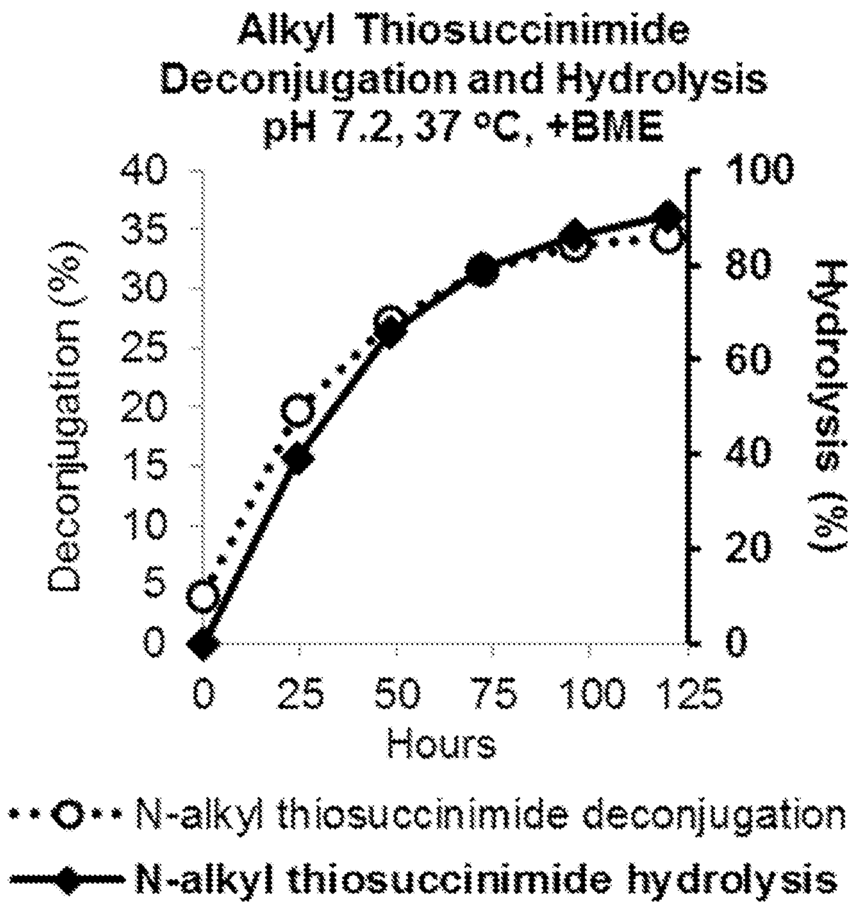

**Figure 21** **Sensitivity of T289C mAb Conjugates to Thiol Exchange in Buffer after Mild Hydrolysis**

**A**

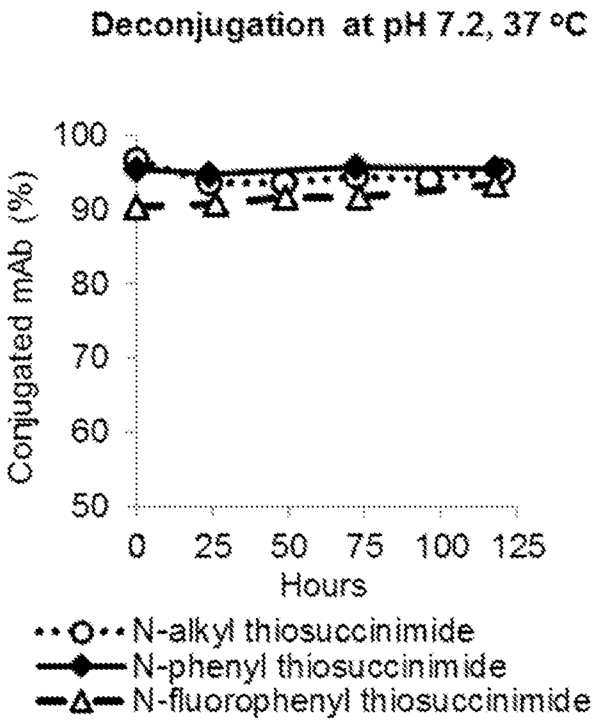


**B**

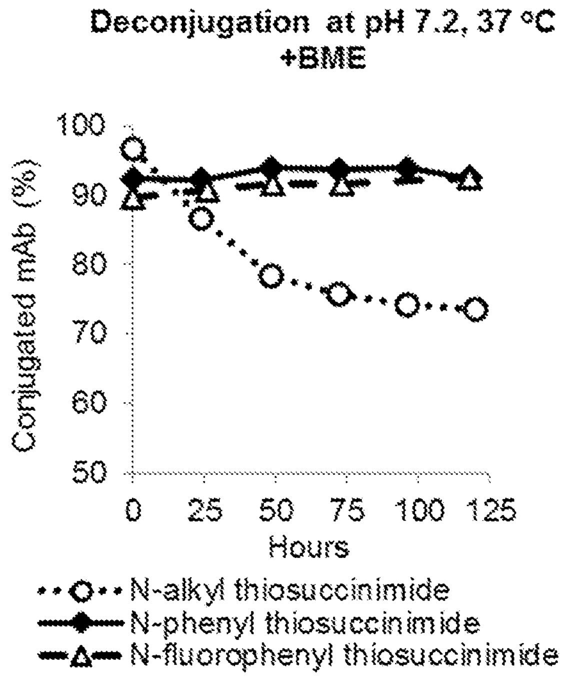

**Figure 22 Analysis of mc-PAB-MMAE T289C mAb ADCs**

**Unreacted T289C mAb**

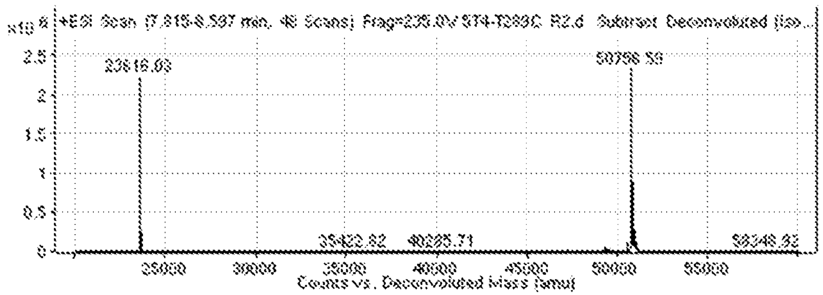


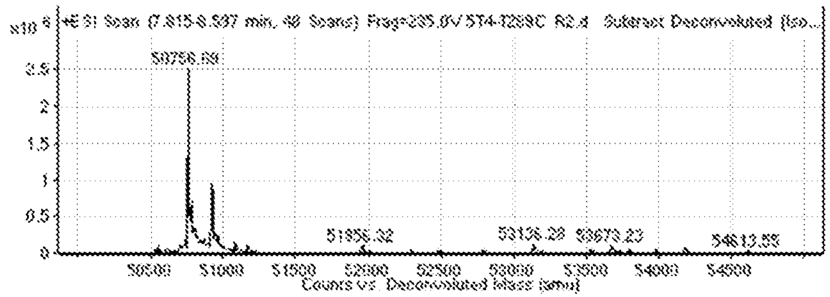


**N-alkyl maleimido-Val-Cit-PAB-MMAE**

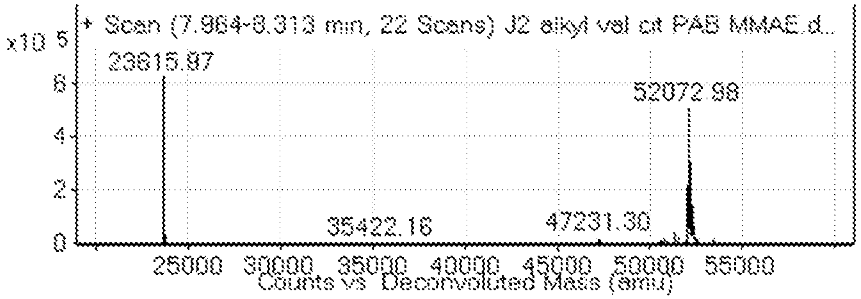


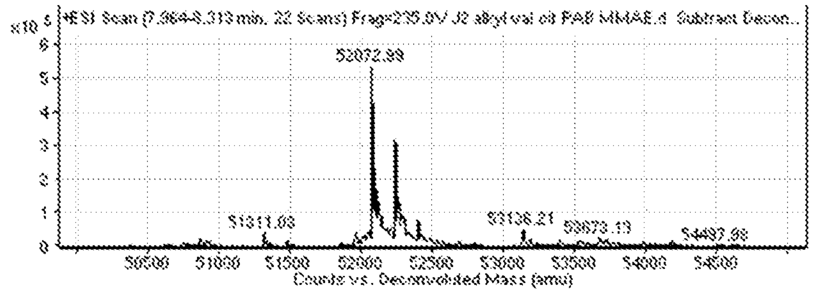


**N-phenyl maleimido-Val-Cit-PAB-MMAE**

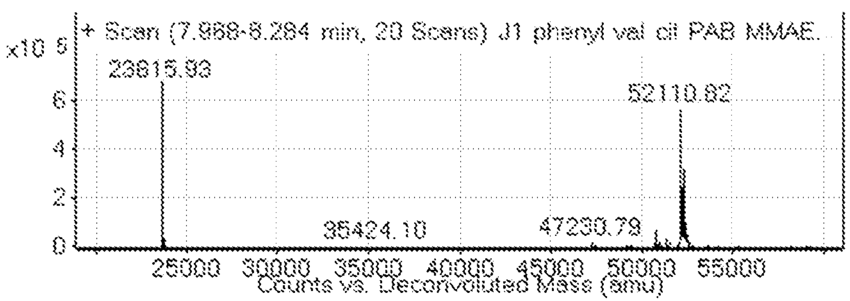


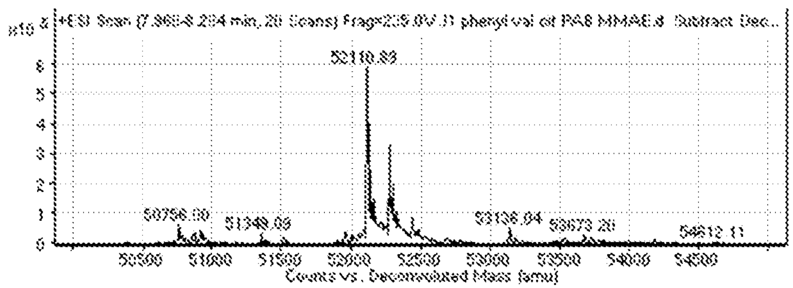

**Figure 23 Stability of MMAE T289C mAb Conjugates in Buffer Containing Thiol (BME)**

**A**

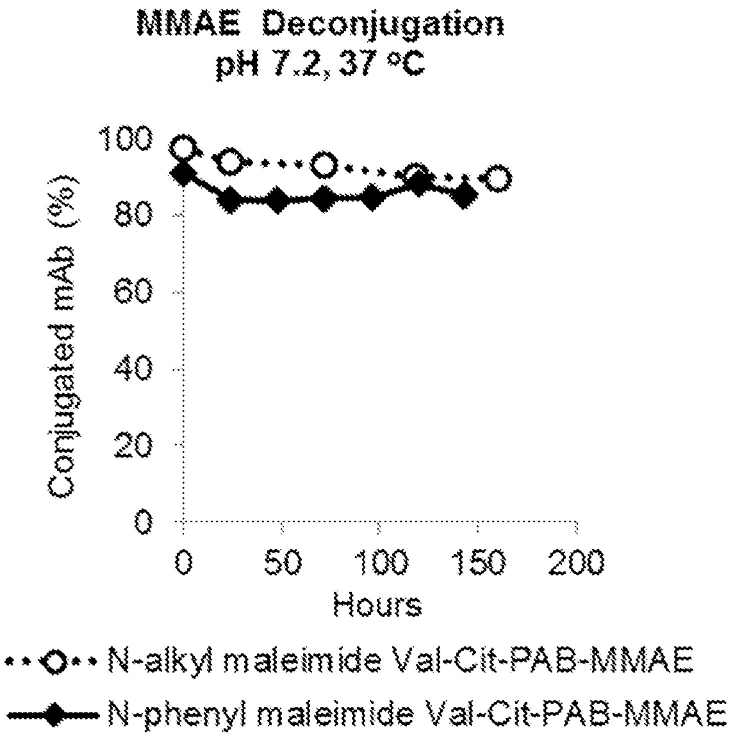


**B**

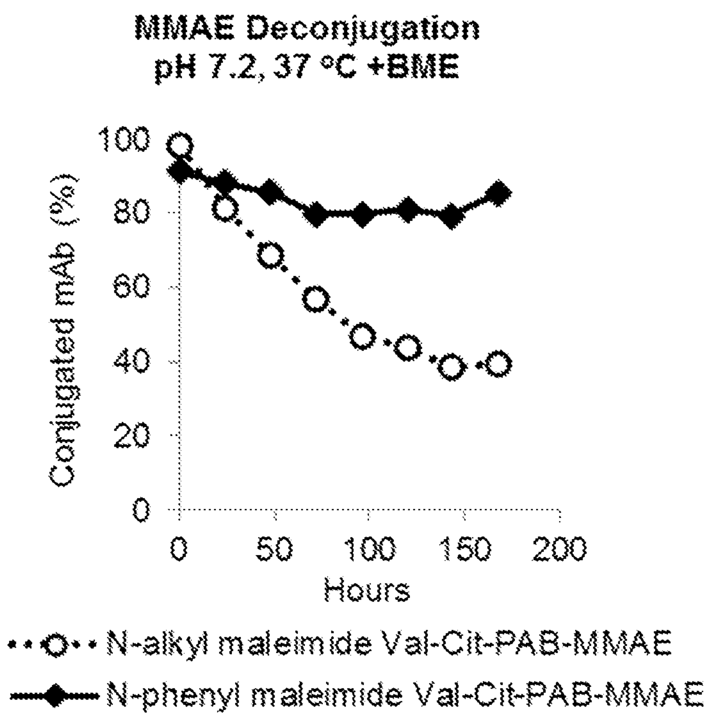

**Figure 24 Hydrolysis of MMAE-Thiosuccinimides in T289C mAb Conjugates**

**A**

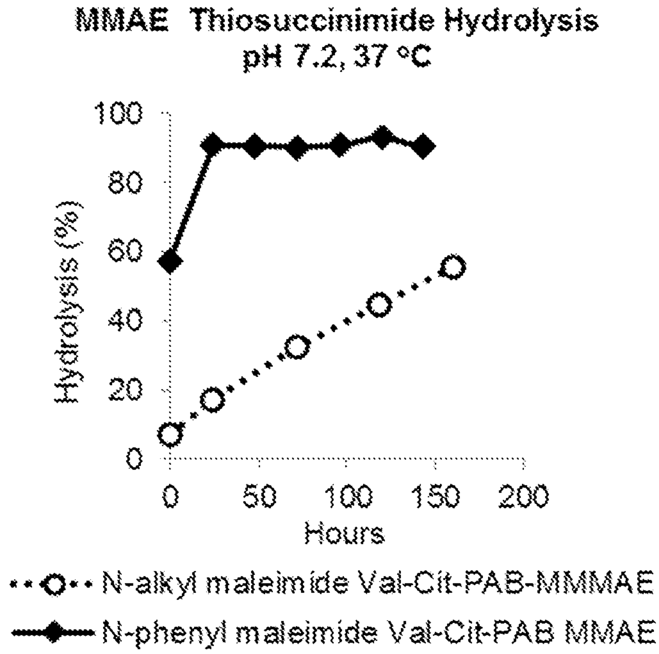


**B**

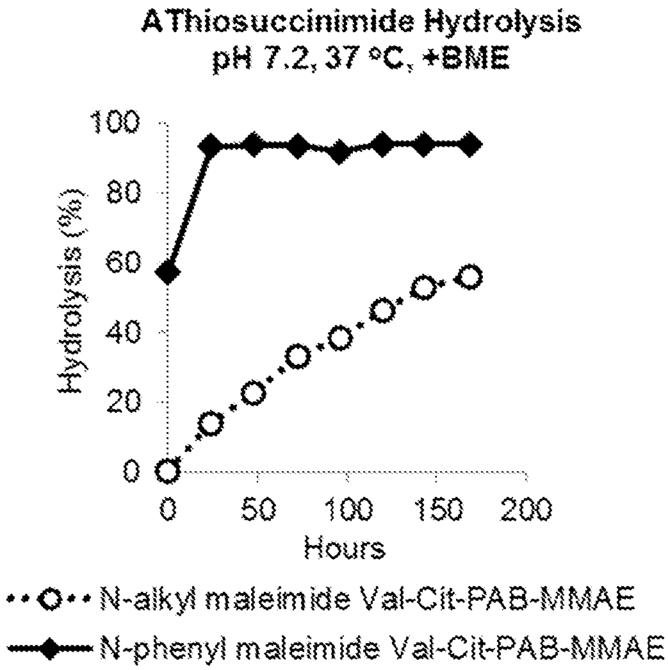

Figure 25

**Stability of MMAE T289C mAb Conjugates in Buffer Containing Thiol (BME)**

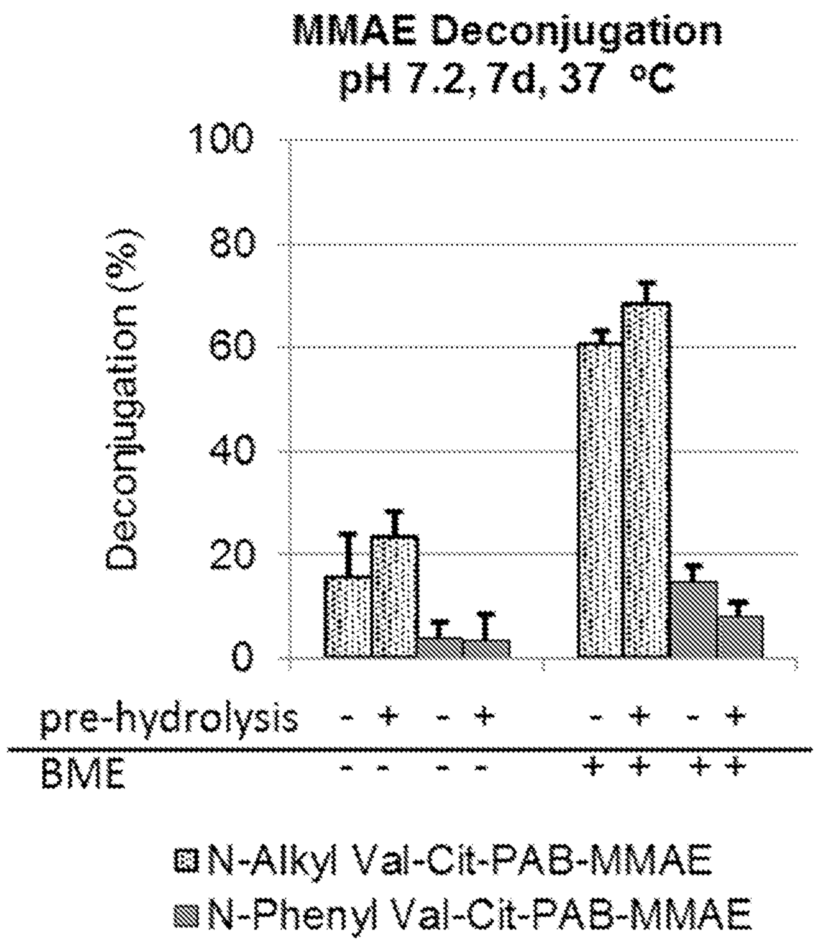

**Figure 26** **Comparison of PEG-biotin and MMAE Thiosuccinimide Hydrolysis Observed in the BME Challenge**

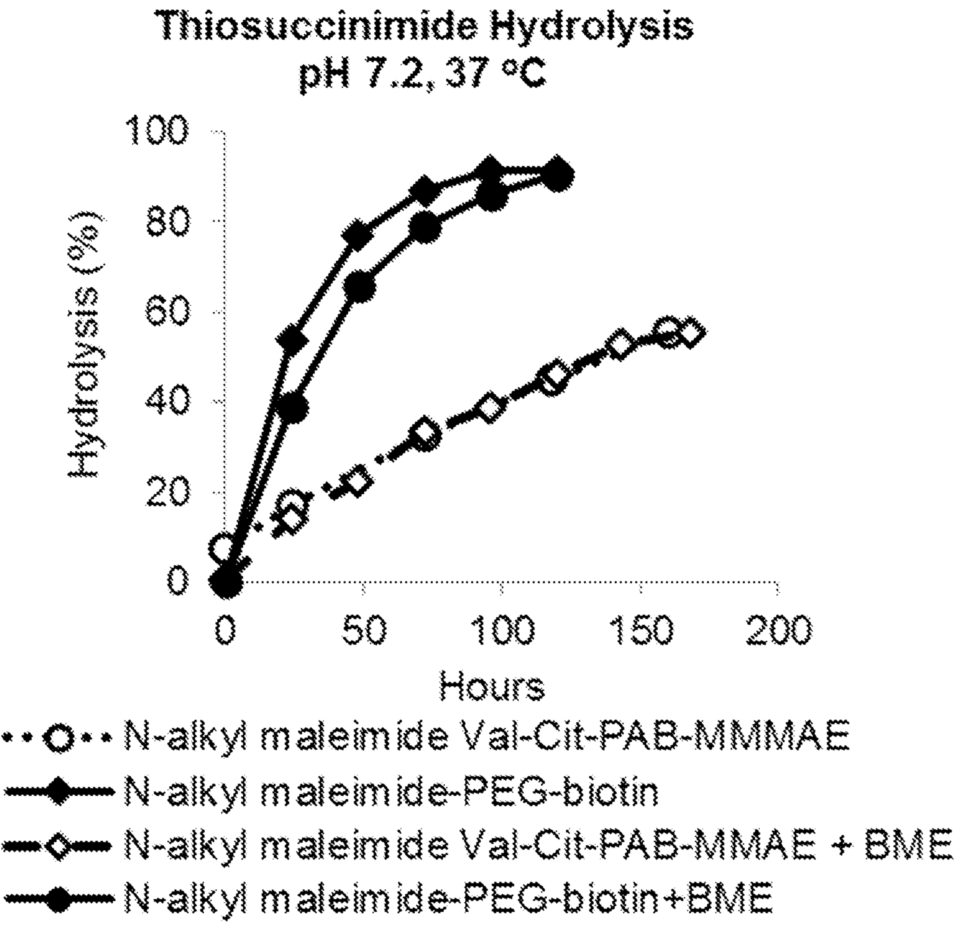

**Figure 27 Comparison of Deconjugation in the Presence of β-Mercaptoethanol: PEG-Biotin vs. MMAE payload**

**A**

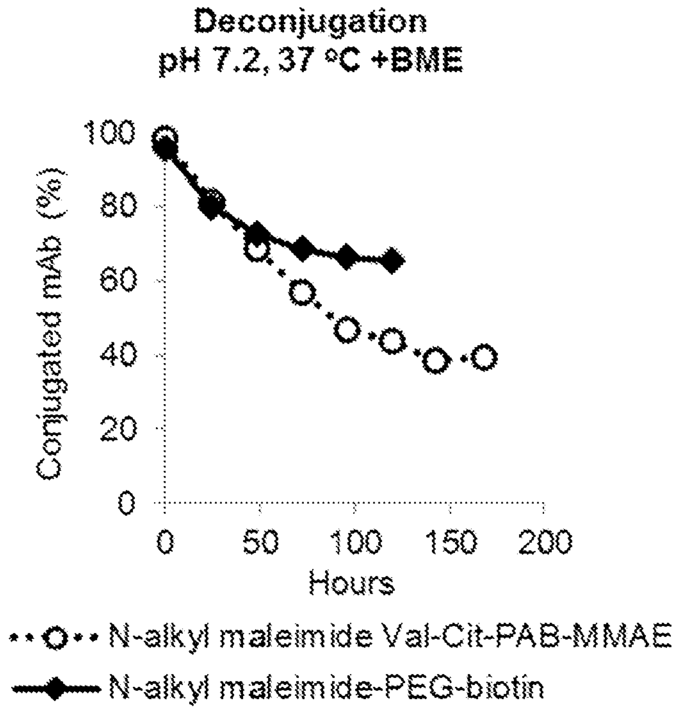


**B**

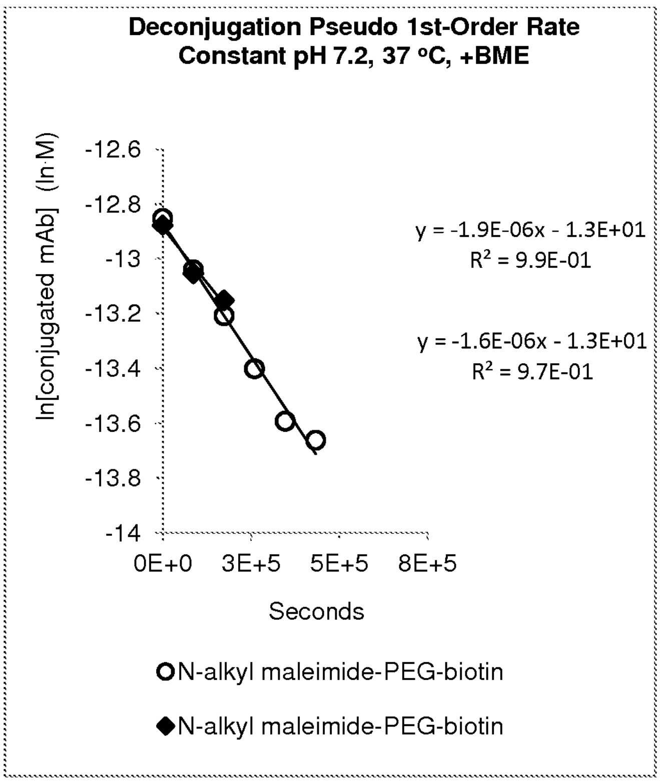

**Figure 28 Stability of MMAE-T289C ADCs in Mouse Serum**

A

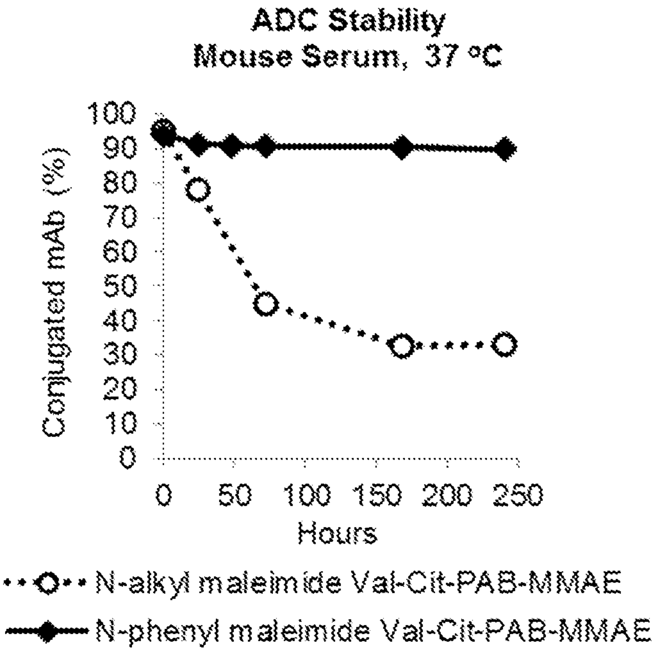


B

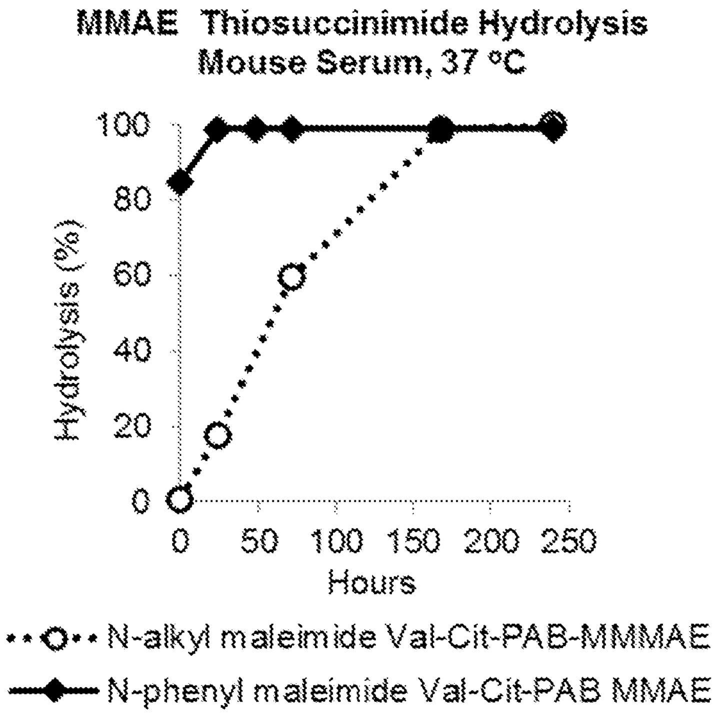

**Figure 29 Stability of MMAE-T289C ADCs in Mouse Serum**

**A**

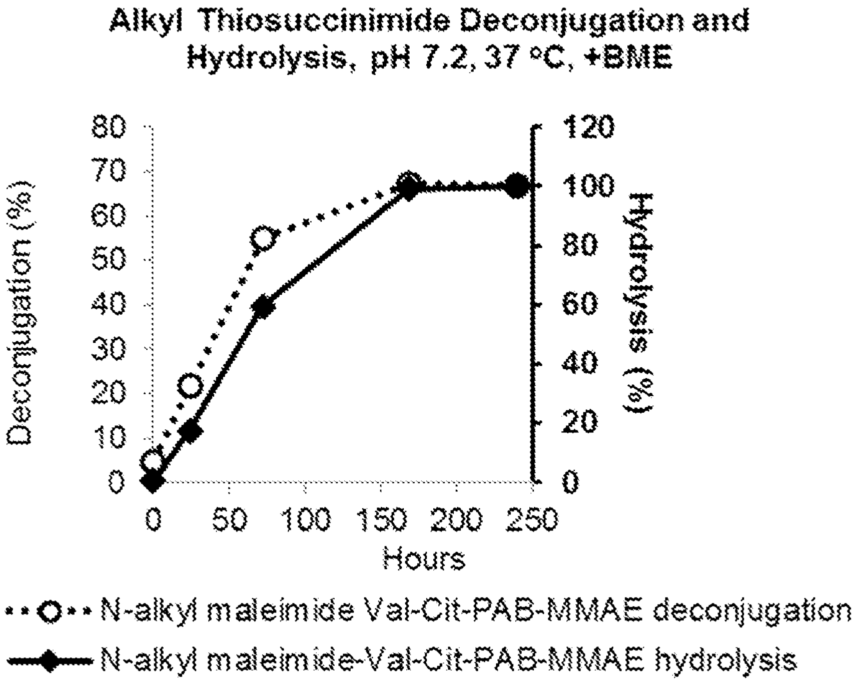


**B**

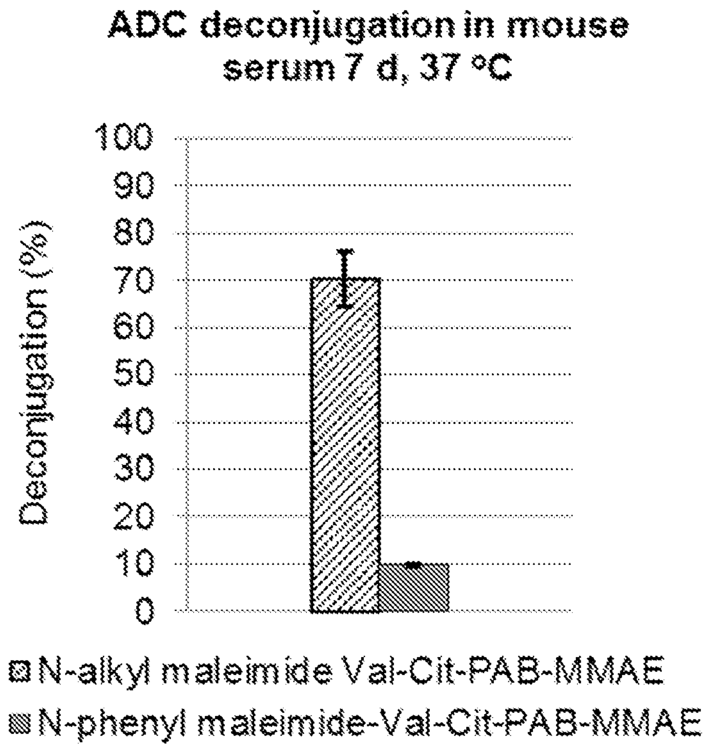

**Figure 30** **Activity of ADCs towards MDA-MB-361 cancer cells after incubation in mouse serum**

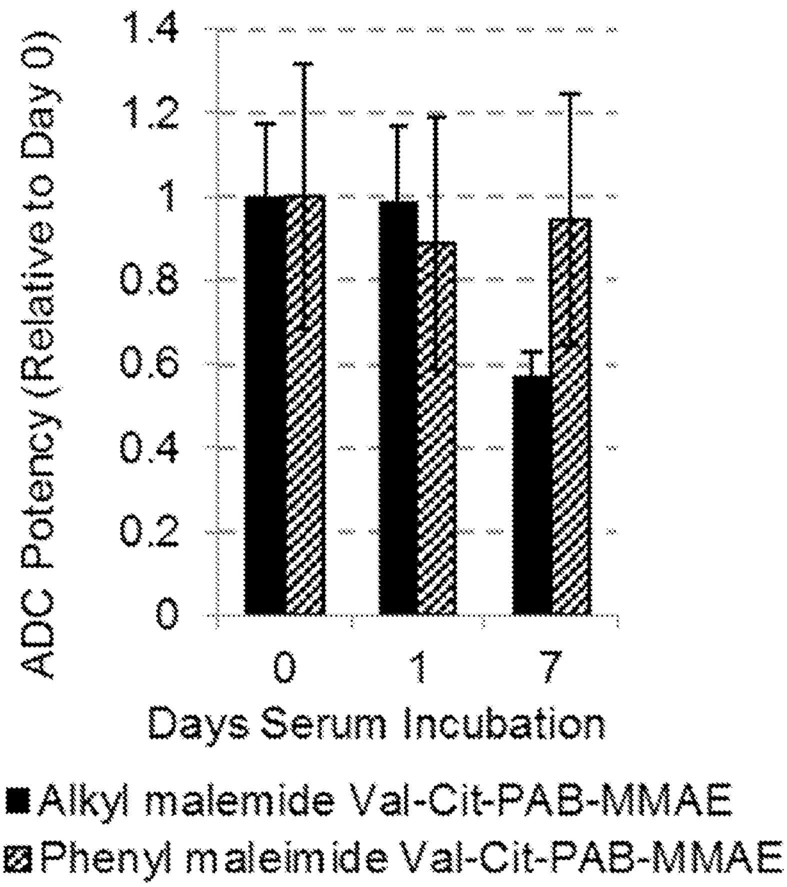

**Figure 31 Alternative Format for Stabilization of Thiol-linked ADCs**

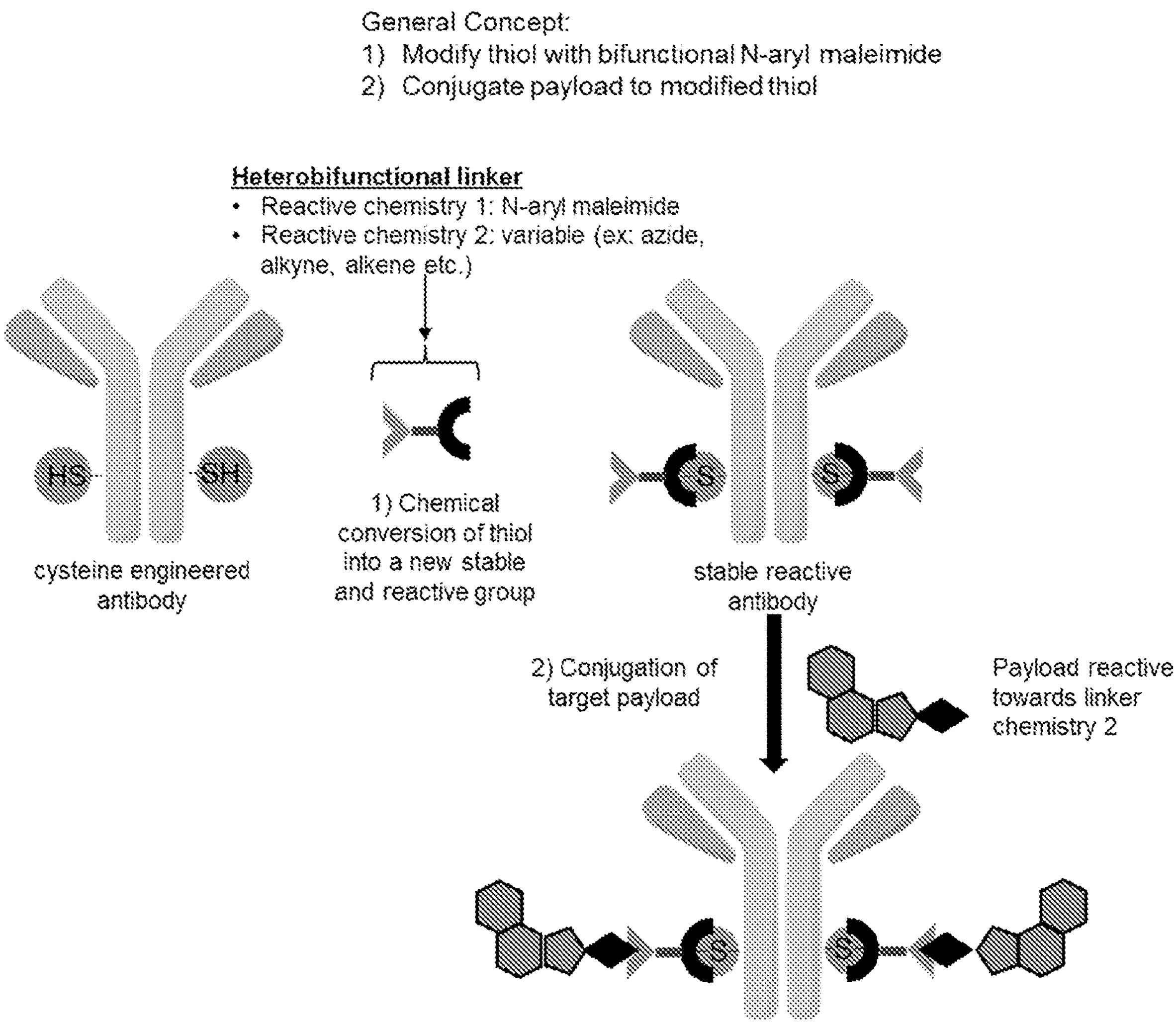

**Figure 32 Mass Spectrometry Analysis of N-phenyl Maleimide-PEG-BCN-mAb Conjugate**

**Unreated mAb**

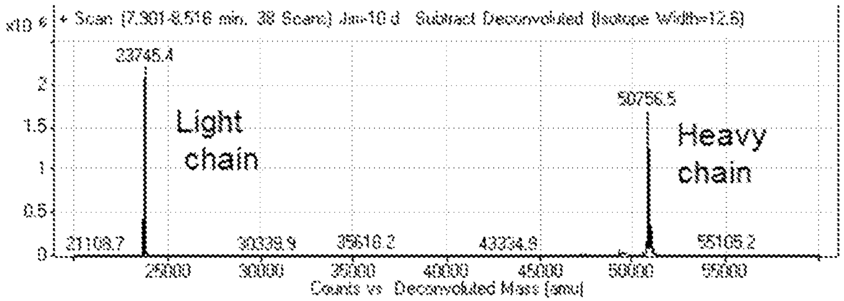


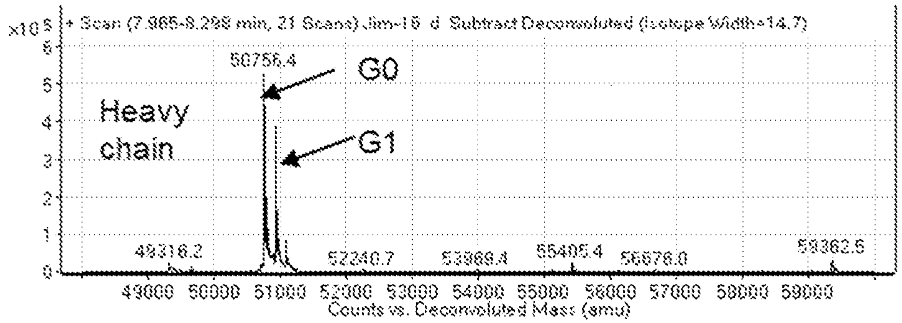


**Conjugated mAb**

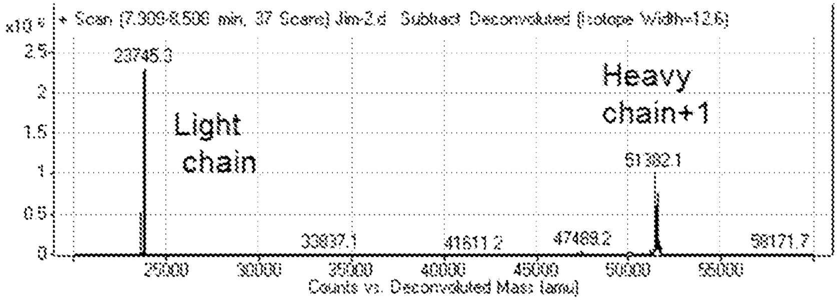


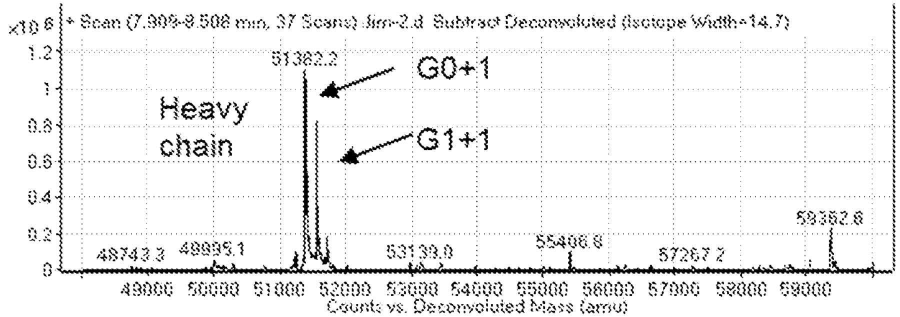

**Figure 33 Mass Spectrometry Analysis of N-Fluorophenyl Maleimide-PEG-BCN-mAb Conjugate**

**Unreated mAb**

**Conjugated mAb**

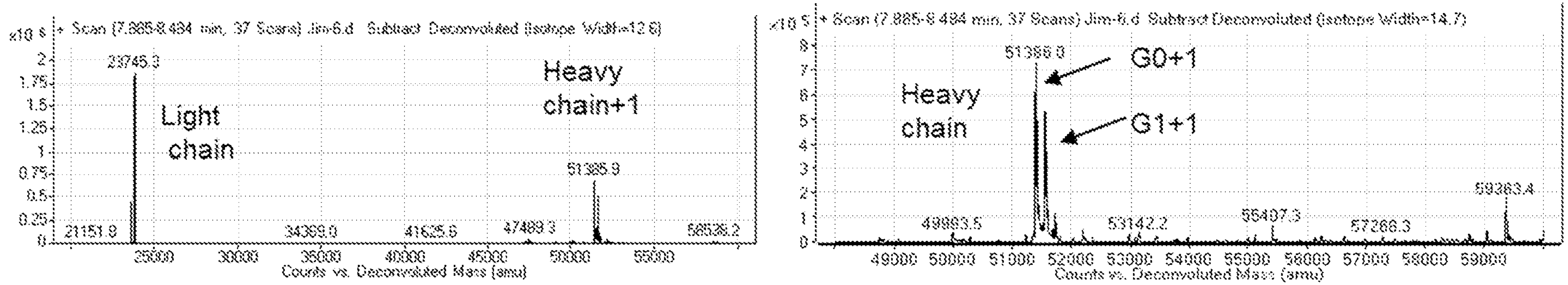

**Figure 34 Mass Spectrometry Analysis of N-Alkyl Maleimide-PEG-BCN-mAb Conjugate**

**Unreated mAb**

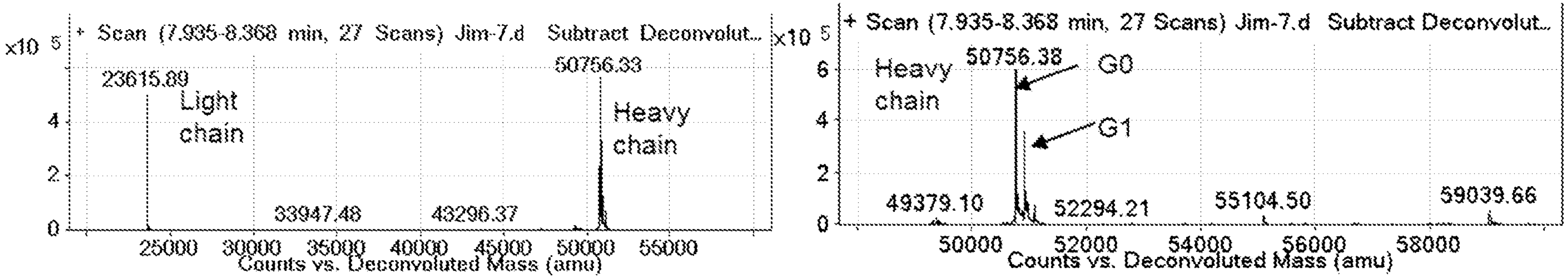


**Conjugated mAb**

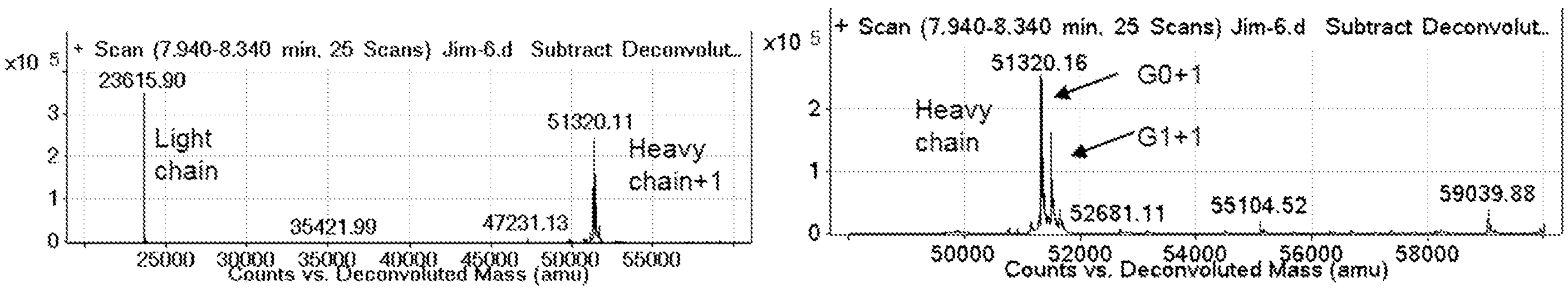

**Figure 35 Conjugation Efficiency of x-Maleimide-PEG-BCNs to T289C mAb**

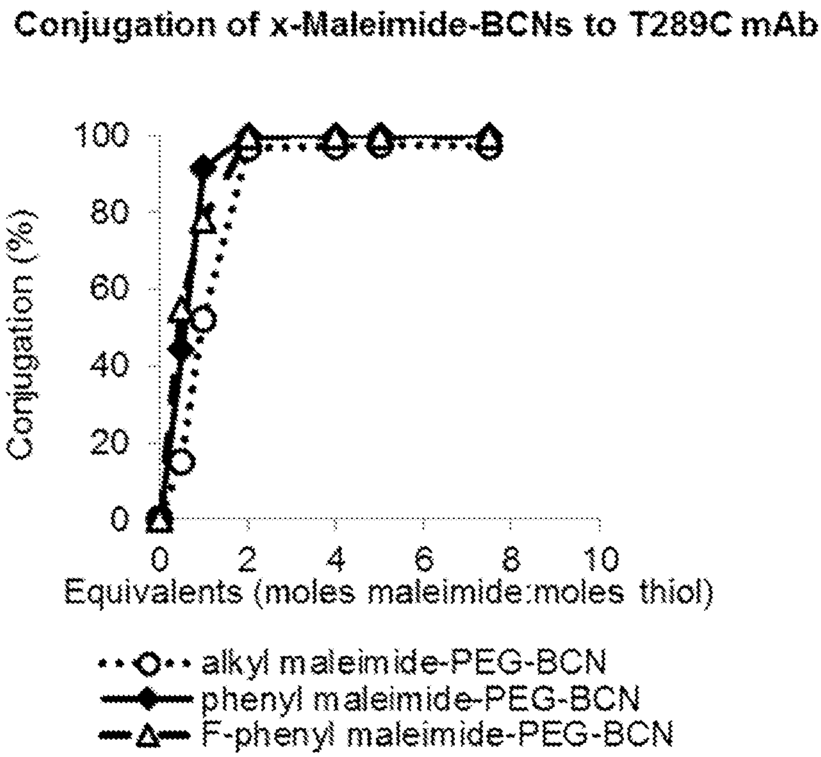

**Figure 36 Thiosuccinimide hydrolysis kinetics for x-maleimide-PEG-BCN conjugates**

**A**

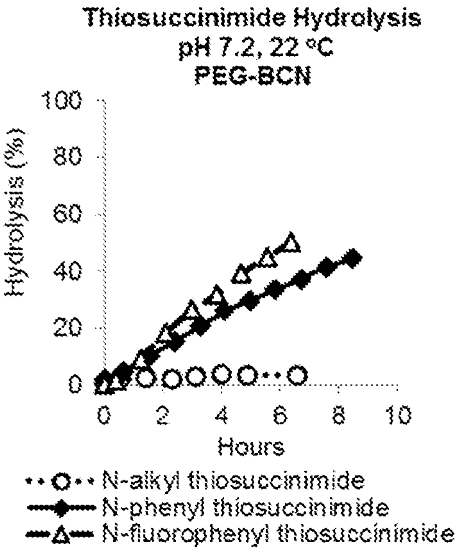


**B**

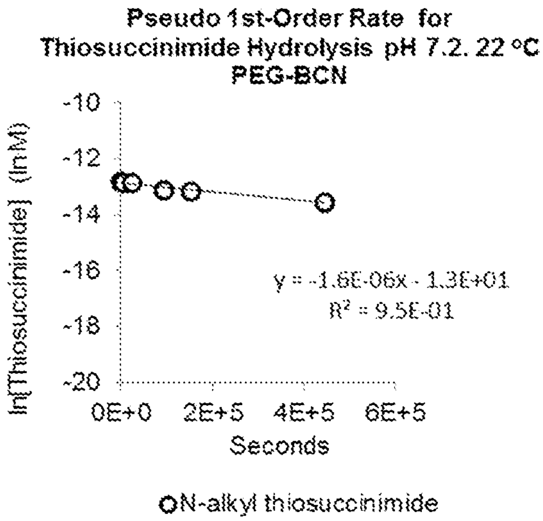


**C**

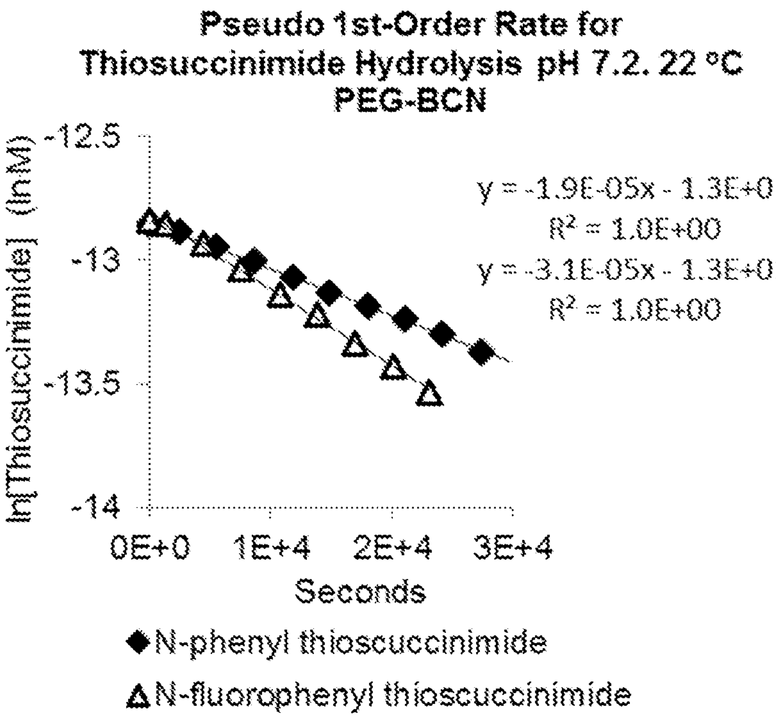

**Figure 37 Analysis of mAb-BCN-Ac4GlcNAz Conjugates**

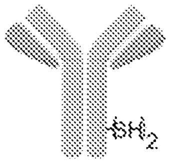

mAb T289C

Reduced LCMS

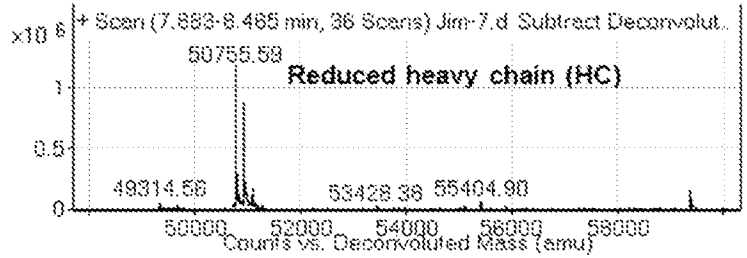


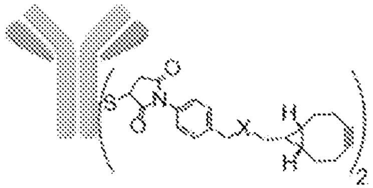

BCN-functionalized mAb

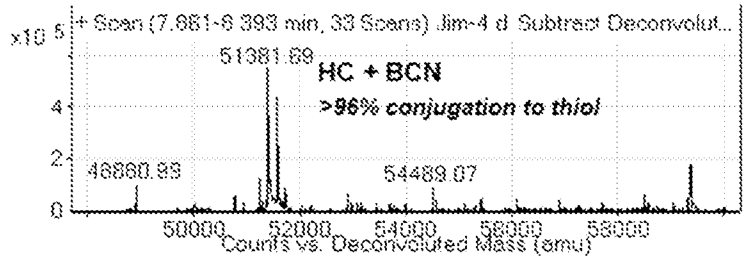


Mass added: 626.1

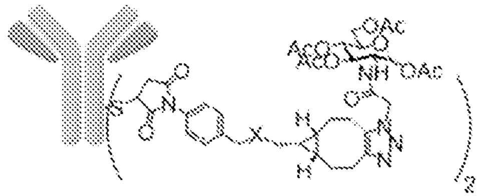

mAb-BCN-AC4GlcNAz conjugate

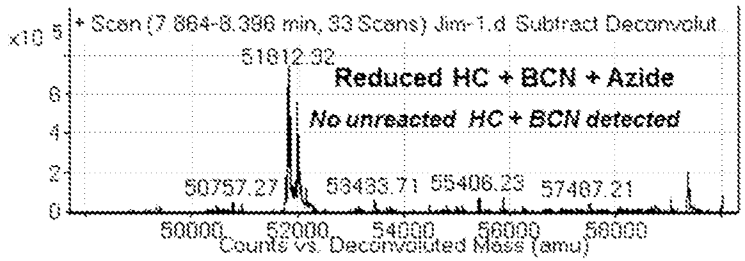


Mass added: 430.6

**Figure 38 Reactivity of mAb-BCN Conjugate After Storage**

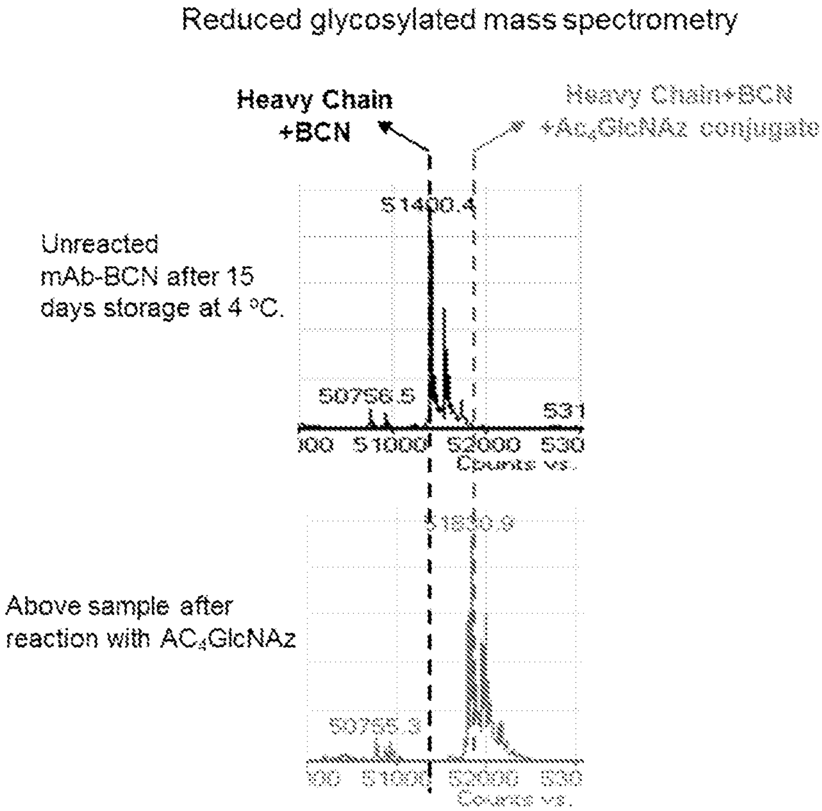

**Figure 39 Analysis of mAb-PBD Conjugates**

5T4-targeted T289C mAb – N-phenyl maleimide-PEG-BCN conjugate

mAb
+
crosslinker

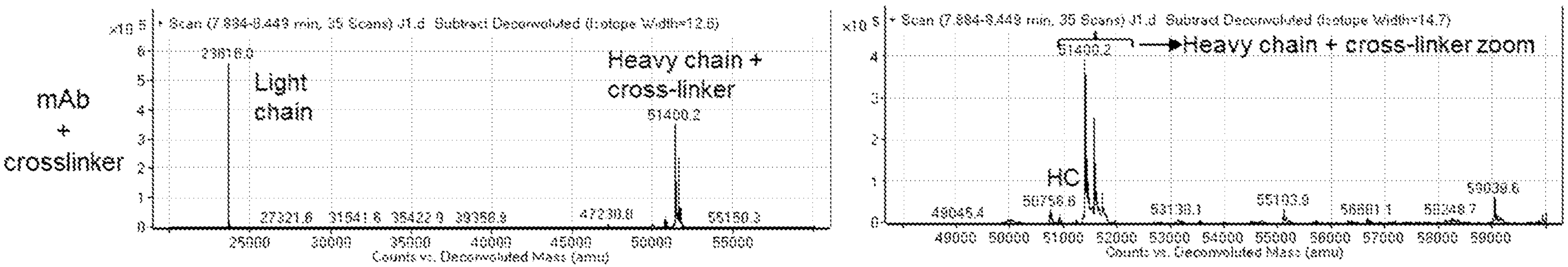


**5T4-targeted T289C mAb – N-phenyl maleimide-PEG-BCN-PBD conjugate**

mAb
+
crosslinker
+
PBD

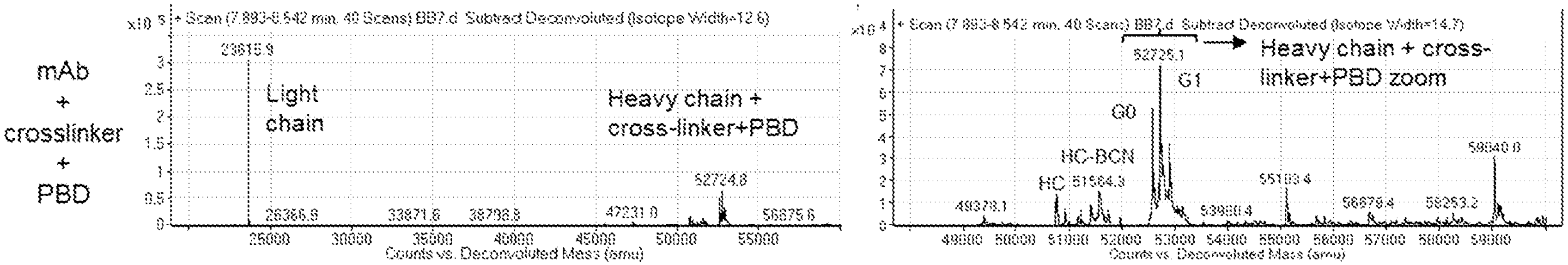

**Figure 40 In Vitro Activity of mAb-BCN-PBD ADCs Towards MDA-MB-361 Breast Cancer Cells**

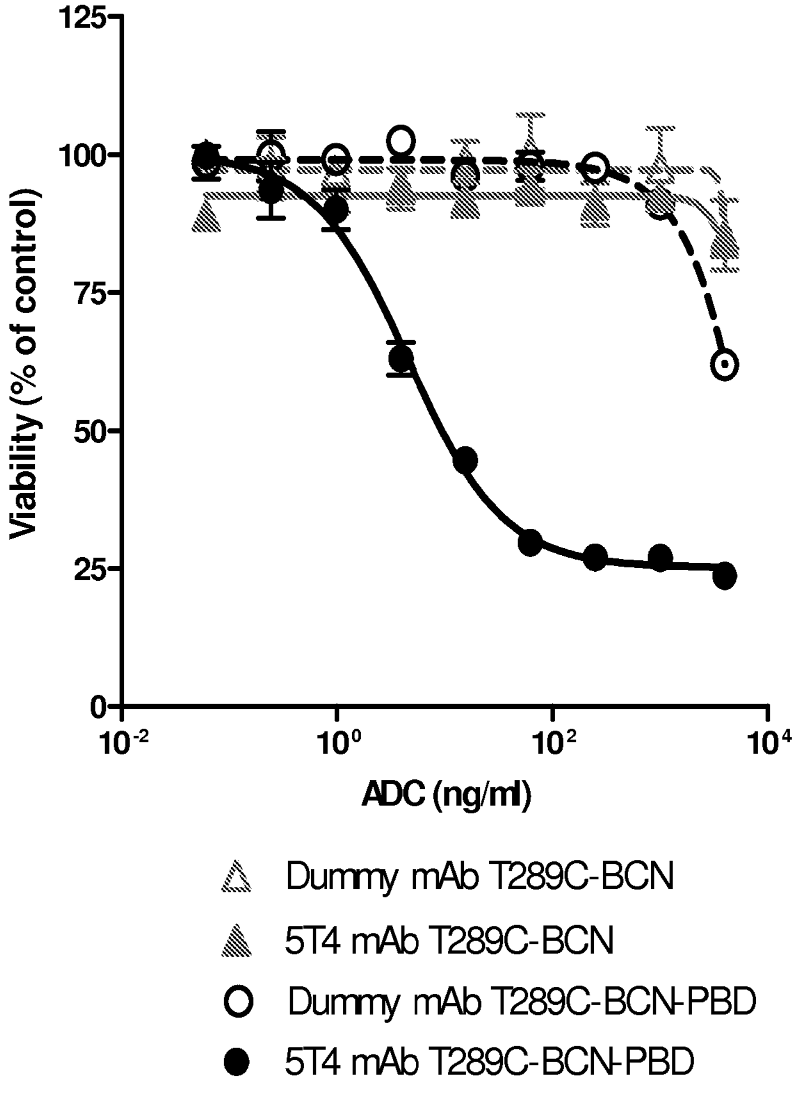

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013173337 A
- WO 2013121175 A
- WO 20131211175 A
- WO 9627011 A
- WO 2007024715 A
- WO 2009018386 A
- WO 2009080251 A
- WO 2013006544 A
- WO 2013070565 A
- WO 2013096291 A
- WO 9520401 A
- US 5789208 A
- US 6335163 B
- WO 04009618 A
- US 20030082630 A
- US 20030157561 A
- US 200800139791 A
- WO 2005056764 A
- WO 2009058379 A
- WO 2011130324 A
- EP 1641818 A1
- WO 0220565 A
- WO 0248189 A
- US 5635483 A
- US 5780588 A
- US 5663149 A
- WO 2002088172 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 5712374 A
- US 5714586 A
- US 5739116 A
- US 5767285 A
- US 5770701 A
- US 5770710 A
- US 5773001 A
- US 5877296 A
- WO 2012019123 A
- US 7776814 B
- WO 2000012507 A
- WO 2007039752 A
- WO 2005110423 A
- WO 2005085251 A
- WO 2005040170 A
- US 7612062 B
- WO 1993021232 A
- US 5830912 A
- WO 9820734 A


### Non-patent literature cited in the description


- **LIBURDY et al.** *J. of Immunological methods,* 1979, vol. 28, 233-242
- **ALLEY et al.** *Bioconjugate Chem.,* 2008, vol. 19, 759-765
- **MD. ROWSHON ALAM et al.** *Bioconjugate Chem,* 2011, vol. 22, 1673-1681
- *Protein Eng. Des. Sel.,* 2005, vol. 18, 435-444
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Mol. Biol.,* 2003, vol. 332, 489-503
- *PNAS,* 2003
- *Biol.,* 2007, 369
- **WOYKE et al.** *Antimicrob. Agents and Chemother.,* 2001, vol. 45, 3580-3584

- **LIU et al.** *Proc. Natl. Acad. Sci. USA,* 1996, vol. 93, 8618-8623
- **CHARI et al.** *Cancer Research,* 1992, vol. 52, 127-131
- **HINMAN et al.** *Cancer Research,* 1993, vol. 53, 3336-3342
- **LODE et al.** *Cancer Research,* 1998, vol. 58, 2925-2928
- **SASSE et al.** *J. Antibiot.,* 2000, vol. 53, 879-885
- **STEINMETZ et al.** *Chem. Int. Ed.,* 2004, vol. 43, 4888-4892
- **KHALIL et al.** *ChemBioChem.,* 2006, vol. 7, 678-683
- **KAUR et al.** *Biochem. J.,* 2006, vol. 396, 235-242
- **DOMLING et al.** *Mol. Diversity,* 2005, vol. 9, 141-147
- **JUO, PEI-SHOW.** Concise Dictionary of Biomedicine and Molecular Biology. CRC Press, 2002
- The Dictionary of Cell and Molecular Biology. Academic Press, 1999
- Oxford Dictionary Of Biochemistry And Molecular Biology. Oxford University Press, 2000
- Remington's Pharmaceutical Sciences. Mack Publishing Company, 1991
- **LI, J. ; XU, Q. ; CORTES, D. et al.** Reaction of cysteines substituted in the amphipathic N-terminal tail of a bacterial potassium channel with hydrophilic and hydrophobic maleimides. *PNAS,* 2002, vol. 99 (18), 11605-11610
- **MOSSER, G.** N-Substituted maleimide inactivation of the response of taste cell stimulation. *J. Neurobiol.,* 1976, vol. 7 (5), 457-468
- **OLEKSANDR, K. ; LERICHE, G. et al.** Selective Irreversible chemical tagging of cysteine with 3-arylpropiolonitriles. *Bioconjug. Chem.,* 2014, vol. 25, 202-206
- **KHAN, M.N.** Kinetics and mechanism of the alkaline hydrolysis of maleimide. *J. Pharm. Sci.,* 1984, vol. 73 (12), 1767-1171
- **DORONINA SO ; TOKI BE ; TORGOV MY ; MENDELSOHN BA ; CERVENY CG ; CHACE DF ; DEBLANC RL ; GEARING RP ; BOVEE TD ; SIEGALL CB.** Development of potent monoclonal antibody auristatin conjugates for cancer therapy. *Nat Biotechnol.,* 2003, vol. 21, 778-784
- **DUBOWCHIK GM ; FIRESTONE RA ; PADILLA L ; WILLNER D ; HOFSTEAD SJ ; MOSURE K ; KNIPE JO ; LASCH, SJ ; TRAIL PA.** Cathepsin B-labile dipeptide linkers for lysosomal release of doxorubicin from internalizing immunoconjugates: model studies of enzymatic drug release and antigen-specific in vitro anticancer activity. *Bioconjug Chem.,* 2002, vol. 13, 855-869